(19) Europäisches Patentamt
European Patent Office
Office européen des brevets

(11) EP 1 265 914 B1

(12) **EUROPEAN PATENT SPECIFICATION**

(45) Date of publication and mention of the grant of the patent:
**26.12.2007 Bulletin 2007/52**

(21) Application number: **01920759.6**

(22) Date of filing: **22.03.2001**

(51) Int Cl.:
***C07K 14/00*** *(2006.01)*

(86) International application number:
**PCT/US2001/009600**

(87) International publication number:
**WO 2001/070775 (27.09.2001 Gazette 2001/39)**

# (54) WNT-1 RELATED POLYPEPTIDES, AND NUCLEIC ACIDS ENCODING THE SAME

WNT-1 VERWANDTE POLYPEPTIDE UND DAFÜR KODIERENDE NUKLEINSÄUREN

NOUVEAUX POLYPEPTIDES ET ACIDES NUCLEIQUES CODANT CES POLYPEPTIDES

(84) Designated Contracting States:
**AT BE CH CY DE DK ES FI FR GB GR IE IT LI LU MC NL PT SE TR**

(30) Priority: **22.03.2000 US 191258 P**

(43) Date of publication of application:
**18.12.2002 Bulletin 2002/51**

(73) Proprietors:
- **Curagen Corporation**
  **New Haven, CT 06511 (US)**
- **GENENTECH, INC.**
  **South San Francisco**
  **California 94080 (US)**

(72) Inventors:
- **RASTELLI, Luca, K.**
  **Guilford, CT 06437 (US)**
- **PENNICA, Diane**
  **Burlingame , CA94010 (US)**

(74) Representative: **MacLean, Martin Robert et al**
**Mathys & Squire**
**120 Holborn**
**London EC1N 2SQ (GB)**

(56) References cited:
- **DATABASE [Online] 3 March 2000 (2000-03-03) MARRA ET AL: "The WashU-HHMI mouse EST project" retrieved from EMBL, accession no. AA966965 XP002177851 cited in the application**
- **DIANE PENNICA ET AL: "WISP genes are members of the connective tissue growth factor family that are up-regulated in Wnt-1 transformed cells and aberrantly expressed in human colon tumors" PROCEEDINGS OF THE NATIONAL ACADEMY OF SCIENCES OF USA, NATIONAL ACADEMY OF SCIENCE. WASHINGTON, US, vol. 95, no. 25, December 1998 (1998-12), pages 14717-14722, XP002105893 ISSN: 0027-8424 cited in the application**
- **DATABASE [Online] 29 September 2000 (2000-09-29) KAWAKAMI ET AL.: "NEDO human cDNA sequencing project" retrieved from EMBL, accession no. AK027111 XP002177852**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Wnt family members are cysteine-rich, glycosylated signaling proteins that mediate diverse developmental processes such as the control of cell proliferation, adhesion, cell polarity, and the establishment of cell fates. Components of the Wnt signaling pathway have been linked to tumorigenesis in familial and sporadic colon carcinomas, breast cancer, and melanoma. Experiments suggest that the adenomatous polyposis coli (APC) tumor suppressor gene also plays an important role in Wnt signaling by regulating beta-catenin levels. APC is phosphorylated by GSK-3b, binds to beta-catenin and facilitates its degradation. Mutations in either APC or beta-catenin have been associated with colon carcinomas and melanomas, suggesting these mutations contribute to the development of these types of cancer, implicating the Wnt pathway in tumorigenesis.

Although much has been learned about the Wnt signaling pathway over the past several years, only a few of the transcriptionally activated downstream components activated by Wnt have been characterized. Those that have been described cannot account for all of the diverse functions attributed to Wnt signaling.

EMBL-EBI ID:AKO27111 lists the nucleic acid sequence of a homo sapiens cDNA (clone HSI07327), deposited by Kawakami, T. et al. (29/9/00).

EP 1 354950 (& WO 02/053737) and JP 2000-402288 describe an NF-KB activating gene cloned from a cDNA library prepared from human lung fibroblasts, and uses thereof for identifying an NF-KB activation inhibitor or promoter.

EBI Database Abstract AAB56802 (& WO 00/55174) describes a human prostate cancer associated protein sequence.

The invention is based in part upon the discovery of novel nucleic acid sequences encoding novel polypeptides. Nucleic acids encoding the polypeptides disclosed in the invention will hereinafter be collectively designated as "WUP" (Wnt1 *UP* regulated) nucleic acid or polypeptide sequences.

The invention is defined by reference to the claims of the present specification.

Although methods and materials similar or equivalent to those described herein can be used in the practice or testing of the present invention, suitable methods and materials are described below. In addition, the materials, methods, and examples are illustrative only and not intended to be limiting.

## BRIEF DESCRIPTION OF THE DRAWINGS

**FIG 1** illustrates a protein domain analysis.

## DETAILED DESCRIPTION

To identify additional downstream genes in the Wnt signaling pathway that are relevant to the transformed cell phenotype, the inventors looked at gene expression in Wnt-1 expressing C57MG mouse mammary epithelial cells compared to the gene expression pattern found in normal C57MG and in Wnt-4 expressing C57MG cells. Wnt-4 is not able to induce tumors and autocrine cellular transformation as Wnt-1 does. The inventors have indentified genes and polypeptides that are up-regulated in Wnt-1 expressing C57MG cell (WUP), and their human orthologs.

Genes that are upregulated in Wnt-1 expressing cells represent attractive targets for treating diseases such as cancer. One such gene, *WUP*, is described in the instant invention. A protein likely involved in mitochondrial or endoplasmic reticulum protein transport and processing, *WUP* is upregulated in cells having high metabolic demands, such as cancer cells that undergo rapid proliferation.

### *Definitions*

Unless defined otherwise, all technical and scientific terms have the same meaning as is commonly understood by one of skill in the art to which this invention belongs. The definitions below are presented for clarity.

The recommendations of (Demerec et al., 1966) where these are relevant to genetics are adapted herein. To distinguish between genes (and related nucleic acids) and the proteins that they encode, the abbreviations for genes are indicated by *italicized* (or underlined) text while abbreviations for the proteins start with a capital letter and are not italicized. Thus, *WUP* or WUP refers to the nucleotide sequence that encodes WUP.

"Isolated," when referred to a molecule, refers to a molecule that has been identified and separated and/or recovered from a component of its natural environment. Contaminant components of its natural environment are materials that interfere with diagnostic or therapeutic use.

"Container" is used broadly to mean any receptacle for holding material or reagent. Containers may be fabricated of glass, plastic, ceramic, metal, or any other material that can hold reagents. Acceptable materials will not react adversely with the contents.

1. *Nucleic acid-related definitions*

(a) *control sequences*

Control sequences are DNA sequences that enable the expression of an operably-linked coding sequence in a particular host organism. Prokaryotic control sequences include promoters, operator sequences, and ribosome binding sites. Eukaryotic cells utilize promoters, polyadenylation signals, and enhancers.

(b) *operably-linked*

Nucleic acid is operably-linked when it is placed into a functional relationship with another nucleic acid sequence. For example, a promoter or enhancer is operably-linked to a coding sequence if it affects the transcription of the sequence, or a ribosome-binding site is operably-linked to a coding sequence if positioned to facilitate translation. Generally, "operably-linked" means that the DNA sequences being linked are contiguous, and, in the case of a secretory leader, contiguous and in reading phase. However, enhancers do not have to be contiguous. Linking is accomplished by conventional recombinant DNA methods.

(c) *isolated nucleic acids*

An isolated nucleic acid molecule is purified from the setting in which it is found in nature and is separated from at least one contaminant nucleic acid molecule. Isolated *WUP* molecules are distinguished from the specific *WUP* molecule, as it exists in cells. However, an isolated *WUP* molecule includes *WUP* molecules contained in cells that ordinarily express the WUP where, for example, the nucleic acid molecule is in a chromosomal location different from that of natural cells.

2. *Protein-related definitions*

(a) *purified polypeptide*

When the molecule is a purified polypeptide, the polypeptide will be purified (1) to obtain at least 15 residues of N-terminal or internal amino acid sequence using a sequenator, or (2) to homogeneity by SDS-PAGE under non-reducing or reducing conditions using Coomassie blue or silver stain. Isolated polypeptides include those expressed heterologously in genetically-engineered cells or expressed *in vitro,* since at least one component of the WUP natural environment will not be present. Ordinarily, isolated polypeptides are prepared by at least one purification step.

(b) *active polypeptide*

An active WUP or WUP fragment retains a biological and/or an immunological activity of native or naturally-occurring WUP. Immunological activity refers to the ability to induce the production of an antibody against an antigenic epitope possessed by a native WUP; biological activity refers to a function, either inhibitory or stimulatory, caused by a native WUP that excludes immunological activity. A biological activity of WUP includes, for example, its upregulation in Wntl-expressing cells.

(c) *Abs*

Antibody may be single anti-WUP monoclonal Abs (including agonist, antagonist, and neutralizing Abs), anti-WUP antibody compositions with polyepitopic specificity, single chain anti-WUP Abs, and fragments of anti-WUP Abs. A "monoclonal antibody" refers to an antibody obtained from a population of substantially homogeneous Abs, *i.e.*, the individual Abs comprising the population are identical except for naturally-occurring mutations that may be present in minor amounts

(d) *epitope tags*

An epitope tagged polypeptide refers to a chimeric polypeptide fused to a "tag polypeptide". Such tags provide epitopes against which Abs can be made or are available, but do not interfere with polypeptide activity. To reduce anti-tag antibody reactivity with endogenous epitopes, the tag polypeptide is preferably unique. Suitable tag polypeptides generally have at least six amino acid residues and usually between about 8 and 50 amino acid residues, preferably between 8 and 20 amino acid residues). Examples of epitope tag sequences include HA from *Influenza* A virus and FLAG.

The novel *WUP* of the invention include the nucleic acids whose sequences are provided in Tables 5 and 7. The invention also includes a mutant or variant *WUP* having the sequence identity as defined in the claims, *any* of whose bases may be changed from the corresponding base shown in Tables 5 and 7 while still encoding a protein that maintains the activities and physiological functions of WUP fragment. The invention further includes nucleic acids whose sequences are complementary to those just described, The invention additionally includes nucleic acids having the sequence identity as defined in the claims or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as anti-sense binding nucleic acids in therapeutic applications in a subject.

The invention includes polypeptides comprising an amino acid sequence having at least 80%, preferably at least 90% preferably at least 98% sequence identity to SEQ ID NOS: 6 or 8 wherein said polypeptide is an active WUP polypeptide, as well as nucleotides encoding any of these polypeptides, and compliments of any of these nucleotides.

The novel WUP of the invention include the protein fragment which consists of the amino acid sequence provided in Tables 6 and 8 (SEQ ID NO:39). The invention also include a WUP mutant or variant protein having the sequence identity as defined in the claims, any of whose residues may be changed from the corresponding residue shown in SEQ ID NOs: 6 or 8 while still encoding a protein that maintains its native activities and physiological functions. The invention further encompasses Abs and antibody fragments, such as $F_{ab}$ or $(F_{ab})_2$, that bind immunospecifically to any of the WUP of the invention.

The human ortholog of the mouse sequence is shown in Table 5; the start and stop codons are indicated in boldface and by underlining.

**Table 5** *hWUP1* nucleotide sequence (SEQ ID NO:5)

| | | | | | | |
|---|---|---|---|---|---|---|
| ggctttgtag | ctgctccgca | gcccagcccg | ggcgcgctcg | cagagtccta | ggcggtgcgc | 60 |
| ggcntcctgc | ctcctccctc | ctcggcggtc | gcggcccgcg | cctccgcggt | gcctgccttc | 120 |
| gctctcaggt | tgaggagctc | aagcttggga | aaatggtgtg | cattccttgt | atcgtcattc | 180 |
| cagttctgct | ctggatctac | aaaaaattcc | tggagccata | tatataccct | ctggtttccc | 240 |
| ccttcgttag | tcgtatatgg | cctaagaaag | caatacaaga | atccaatgat | acaaacaaag | 300 |
| gcaaagtaaa | ctttaagggt | gcagacatga | atggattacc | aacaaaagga | ccaacagaaa | 360 |
| tctgtgataa | aaagaaagac | taaagaaatt | ttcctaaagg | accccatcat | ttaaaaaatg | 420 |
| gacctgataa | tatgaagcat | cttccttgta | attgtctctg | accttttat | ctgagaccgg | 480 |
| aattcaggat | aggagtctag | atatttacct | gatactaatc | aggaaatata | tgatatccgt | 540 |
| atttaaaatg | tagttagtta | tatttaatga | cctcattcct | aagttccttt | ttcgttaatg | 600 |
| tagctttcat | ttctgttatt | gctgtttgaa | taatatgatt | aaatagaagg | tttgtgccag | 660 |
| tagacattat | gttactaaat | cagcacttta | aaatctttgg | ttctctaatt | catatgaatt | 720 |
| tgctgtttgc | tctaatttct | ttgggctctt | ctaatttgag | tggagtacaa | ttttgttgtg | 780 |
| aaacagtcca | gtgaaactgt | gcagggaaat | gaaggtagaa | ttttgggagg | taataatgat | 840 |
| gtgaaacata | aagatttaat | aattactgtc | caacacagtg | gagcagcttg | tccacaaata | 900 |
| tagtaattac | tatttattgc | tctaaggaag | attaaaaaaa | gatagggaaa | agggggaaac | 960 |
| ttctttgaaa | aatgaaacat | ctgttacatt | aatgtctaat | tataaaattt | taatccttac | 1020 |
| tgcatttctt | ctgttcctac | aaatgtatta | aacattcagt | ttaactggta | aaaaaaaaaa | 1080 |
| aaaaaaaccc | ggggggggg | | | | | 1099 |

The nucleotide sequence SEQ ID NO:5, comprises in part a sequence that was thought to encode a peptide from nucleotides from 670 to 791; however, the inventors have determined that in fact, the proper peptide, based on homology, is encoded by nucleotides 3 to 380, giving the proper translation start as MVCI (SEQ ID NO:39; Table6).

**Table 6** hWUP1 polypeptide sequence (SEQ ID NO:39)

| | | | | | | | | | | | | | | | |
|---|---|---|---|---|---|---|---|---|---|---|---|---|---|---|---|
| Met | Val | Cys | Ile | Pro | Cys | Ile | Val | Ile | Pro | Val | Leu | Leu | Trp | Ile | Tyr |
| 1 | | | | 5 | | | | | 10 | | | | | 15 | |
| Lys | Lys | Phe | Leu | Glu | Pro | Tyr | Ile | Tyr | Pro | Leu | Val | Ser | Pro | Phe | Val |
| | | | 20 | | | | | 25 | | | | | 30 | | |
| Ser | Arg | Ile | Trp | Pro | Lys | Lys | Ala | Ile | Gln | Glu | Ser | Asn | Asp | Thr | Asn |
| | | 35 | | | | | 40 | | | | | 45 | | | |
| Lys | Gly | Lys | Val | Asn | Phe | Lys | Gly | Ala | Asp | Met | Asn | Gly | Leu | Pro | Thr |
| | 50 | | | | | 55 | | | | | 60 | | | | |
| Lys | Gly | Pro | Thr | Glu | Ile | Cys | Asp | Lys | Lys | Lys | Asp | | | | |
| 65 | | | | | 70 | | | | | 75 | | | | | |

A very similar human sequence was also identified (SEQ ID NO:7).

**Table 7** *hWUP2* nucleotide sequence (SEQ ID NO:7)

| | | | | | | |
|---|---|---|---|---|---|---|
| gtgagtgtgc | ccgggctagc | ggcctgggtt | gggctttgta | gctgctccgc | ggcccagccc | 60 |
| gggcgcgctc | gcagagtcct | aggcggtgcg | cggcctcctg | cctcctccct | cctcggcggt | 120 |
| cgcggcccgc | cggcctccgc | ggtgcctgcc | ttcgctctca | ggttgaggag | ctcaagcttg | 180 |
| ggaaaatggt | gtgcattcct | tgtatcgtca | ttccagttct | gctctggatc | tacaaaaaat | 240 |
| tcctggagcc | atatatatac | cctctggttt | ccccttcgt | tagtcgtata | tggcctaaga | 300 |
| aagcaataca | agaatccaat | gatacaaaca | aaggcaaagt | aaactttaag | ggtgcagaca | 360 |
| tgaatggatt | accaacaaaa | ggaccaacag | aaatctgtga | taaaaagaaa | gactaaagaa | 420 |
| attttcctaa | aggaccccat | catttaaaaa | atggacctga | taatatgaag | catcttcctt | 480 |
| gtaattgtct | ctgacctttt | tatctgagac | cggaattcag | gataggagtc | tagatattta | 540 |
| cctgatacta | atcaggaaat | atatgatatc | cgtatttaaa | atgtagttag | ttatatttaa | 600 |
| tgacctcatt | cctaagttcc | tttttcgtta | atgtagcttt | catttctgtt | attgctgttt | 660 |
| gaataatatg | attaaataga | aggtttgtgc | cagtagacat | tatgttacta | aatcagcact | 720 |
| ttaaaatctt | tggttctcta | attcatatga | atttgctgtt | tgctctaatt | tctttgggct | 780 |
| cttctaattt | gagtggagta | caatttttgtt | gtgaaacagt | ccagtgaaac | tgtgcaggga | 840 |
| aatgaaggta | gaattttggg | aggtaataat | gatgtgaaac | ataaagattt | aataattact | 900 |
| gtccaacaca | gtggagcagc | ttgtccacaa | atatagtaat | tactatttat | tgctctaagg | 960 |
| aagattaaaa | aaagataggg | aaaaggggga | aacttctttg | aaaaatgaaa | catctgttac | 1020 |
| attaatgtct | aattataaaa | ttttaatcct | tactgcattt | cttctgttcc | tacaaatgta | 1080 |
| ttaaacattc | agtttaaaaa | aaaaaaaaaa | aaa | | | 1113 |

A polypeptide encoded by SEQ ID NO:7 is presented in Table 8 (SEQ ID NO:39).

**Table 8.** hWUP2 polypeptide sequence (SEQ ID NO:39)

| | | | | | | | | | | | | | | | |
|---|---|---|---|---|---|---|---|---|---|---|---|---|---|---|---|
| Met | Val | Cys | Ile | Pro | Cys | Ile | Val | Ile | Pro | Val | Leu | Leu | Trp | Ile | Tyr |
| 1 | | | | 5 | | | | | 10 | | | | | 15 | |
| Lys | Lys | Phe | Leu | Glu | Pro | Tyr | Ile | Tyr | Pro | Leu | Val | Ser | Pro | Phe | Val |
| | | | 20 | | | | | 25 | | | | | 30 | | |
| Ser | Arg | Ile | Trp | Pro | Lys | Lys | Ala | Ile | Gln | Glu | Ser | Asn | Asp | Thr | Asn |
| | | 35 | | | | | 40 | | | | | 45 | | | |
| Lys | Gly | Lys | Val | Asn | Phe | Lys | Gly | Ala | Asp | Met | Asn | Gly | Leu | Pro | Thr |
| | 50 | | | | | 55 | | | | | 60 | | | | |
| Lys | Gly | Pro | Thr | Glu | Ile | Cys | Asp | Lys | Lys | Lys | Asp | | | | |
| 65 | | | | | 70 | | | | | 75 | | | | | |

Table 10 shows the novel proteins aligned together with other putative peptides encoded by extension of the rat EST extension (SEQ ID NO:9), a rabbit EST (GenBank C86606; SEQ ID NO:10), a fish EST (GenBank AU036392, SEQ ID NO:11) and a putative *Drosophila* protein (GenBank 097172; SEQ ID NO:12). In Table 10, SEQ ID NO:2 is referred to as "cgrry0c0261_11202-243_EXT_REV", SEQ ID NO:4 is "ss.Cura16.3p.contig.full.991216_REVCOMP", SEQ ID NO:6 is "V34238patented__rev", and SEQ ID NO:8 is "87769892".

**Table 10**

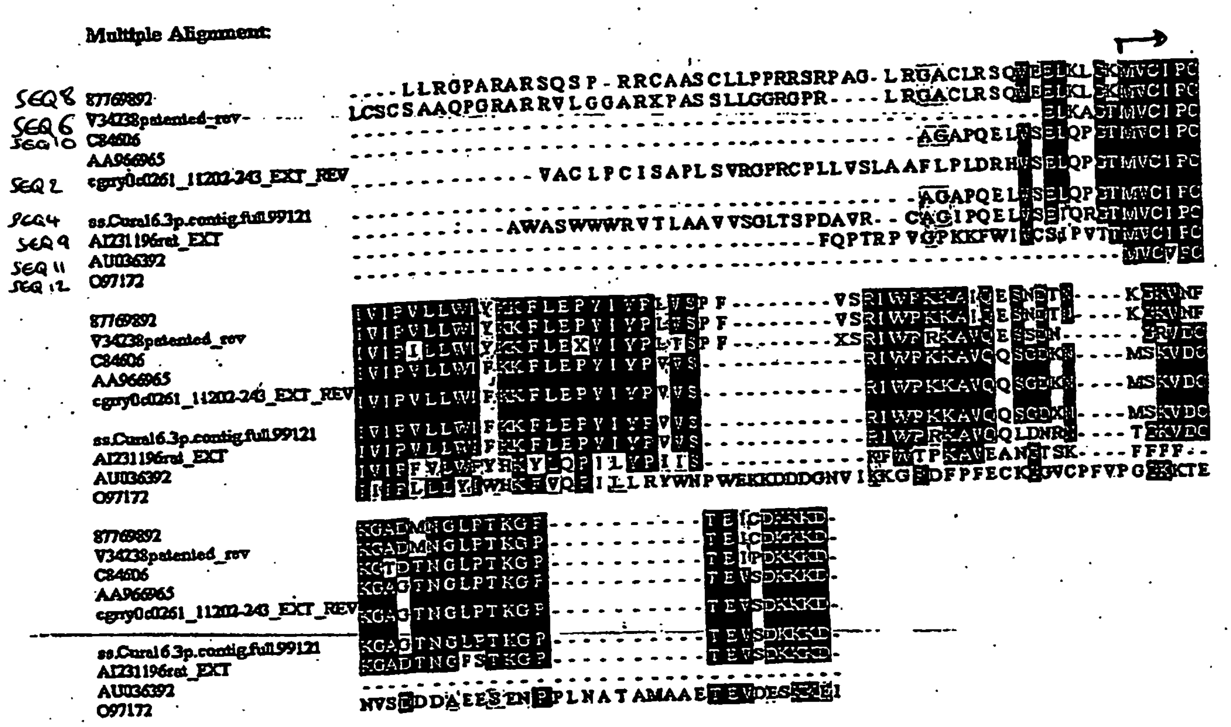


This aligment demonstrates that SEQ ID NO:9 is a highly conserved protein and demonstrates that the Met in MVCI (residues 1-4 of SEQ ID NO:9) indicates a Kozak Met that is the translation start site. Further analysis indicates that this protein is homologous to signal peptidase I Serine proteins. In *E. coli,* these proteins are responsible for cleaving N-terminal leader sequences from secreted or periplasmic proteins. Prodom shows very good homology to Protein ATP Synthase Hydrogen Ion Transport of the mitochondrion membrane.

The homology to transmembrane protein fits well with the hydrophobic profile. Blocks analysis, shown in Figure 1, also reveals homology to serin proteases.

PSORT (Nakai and Horton,1999) predicts that all of the orthologs localize to the endoplasmic reticulum membrane, but the homology with bacterial and mitochodrion proteins indicates that WUP likely localizes to the membrane of the mitochondrion. Because of its homology with the *E. coli* signal peptidase I serine proteins, WUP is likely involved in mitochondrial import and processing of mitochondrial proteins.

The nucleic acids and proteins of the invention are useful for the manufacture of medicaments for the treatment of cancers, including colon cancer, breast cancer, and melanoma. However, no medical use is part of this invention as claimed. For example, a cDNA encoding WUP may be useful in gene therapy, and WUP protein may be useful when administered to a subject in need thereof. The novel nucleic acid encoding WUP, and the WUP protein of the invention, may further be useful in in vitro diagnostic applications, wherein the presence or amount of the nucleic acid or the protein are to be assessed. These materials are further useful in the generation of Abs that bind immunospecifically to the novel substances of the invention for use in therapeutic or diagnostic methods.

WUP *polynucleotides*

One aspect of the on pertains to isolated nucleic acid molecules that encode WUP as defined in the pending claims. Nucleic acid fragments thereof may be useful as hybridization probes to identify WUP-encoding nucleic acids (*e.g., WUP* mRNAs) and for use as polymerase chain reaction (PCR) primers for the amplification and/or mutation of *WUP* molecules. A "nucleic acid molecule" includes DNA molecules (e.g., cDNA or genomic DNA), RNA molecules (e.g., mRNA), analogs of the DNA or RNA generated using nucleotide analogs, derivatives, and homologs, wherein said molecule have the sequence identity as defined in the claims. The nucleic acid molecule may be single-stranded or double-stranded, but preferably comprises double-stranded DNA.

The full- or partial length native sequence *WUP* may be used to "pull out" similar (homologous) sequences (Ausubel et al., 1987; Sambrook, 1989), such as: (1) full-length or fragments of WUP cDNA from a cDNA library from any species (*e.g.* human, murine, feline, canine, bacterial, viral, retroviral, yeast), (2) from cells or tissues, (3) variants within a species, and (4) homologues and variants from other species. To find related sequences that may encode related genes, the probe may be designed to encode unique sequences or degenerate sequences. Sequences may also be genomic sequences including promoters, enhancer elements and introns of native sequence *WUP*.

For example, *WUP* coding region in another species may be isolated using such probes. A probe of about 40 bases is designed, based on *WUP*, and made. To detect hybridizations, probes are labeled using, for example, radionuclides such as $^{32}P$ or $^{35}S$, or enzymatic labels such as alkaline phosphatase coupled to the probe via avidin-biotin systems. Labeled probes are used to detect nucleic acids having a complementary sequence to that of *WUP* in libraries of cDNA, genomic DNA or mRNA of a desired species.

Such probes can be used as a part of a diagnostic test kit for identifying cells or tissues which mis-express a WUP, such as by measuring a level of a WUP in a sample of cells from a subject *e.g.*, detecting *WUP* mRNA levels or determining whether a genomic WUP has been mutated or deleted

2. *isolated nucleic acid*

An isolated nucleic acid molecule is separated from other nucleic acid molecules that are present in the natural source of the nucleic acid. Preferably, an isolated nucleic acid is free of sequences that naturally flank the nucleic acid (*i.e.*, sequences located at the 5'- and 3'-termini of the nucleic acid) in the genomic DNA of the organism from which the nucleic acid is derived. For example, in various embodiments, isolated *WUP* molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g.*, brain, heart, liver, spleen, *etc*.). Moreover, an isolated nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e.g.*, a nucleic acid molecule having the nucleotide sequence of SEQ ID NOS: 6 or 8, or a complement of this aforementioned nucleotide sequence, can be isolated using standard molecular biology techniques and the provided sequence information. Using all or a portion of the nucleic acid sequence of SEQ ID NOS: 6 or 8 as a hybridization probe, *WUP* molecules can be isolated using standard hybridization and cloning techniques (Ausubel et al., 1987; Sambrook. 1989).

PCR amplification techniques can be used to amplify *WUP* using cDNA, mRNA or alternatively, genomic DNA, as a template and appropriate oligonucleotide primers. Such nucleic acids can be cloned into an appropriate vector and characterized by DNA sequence analysis. Furthermore, oligonucleotides corresponding to *WUP* sequences can be prepared by standard synthetic techniques, *e.g.,* an automated DNA synthesizer.

*3. oligonucleotide*

An oligonucleotide comprises a series of linked nucleotide residues, which oligonucleotide has a sufficient number of nucleotide bases to be used in a PCR reaction or other application. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. An oligonucleotide comprising a nucleic acid molecule less than 100 nt in length may further comprise at least 6 contiguous nucleotides of SEQ ID NOS: 5 or 7, or a complement thereof. Oligonucleotides may be chemically synthesized and may also be used as probes.

*4. complementary nucleic acid sequences; binding*

In another embodiment, an isolated nucleic acid molecule of the invention comprises a nucleic acid molecule that is a complement of the nucleotide sequence shown in SEQ ID NOS: 5 or 7. A portion of this nucleotide sequence (*e.g.*, a fragment can be used as a probe or primer or a fragment encoding a biologically-active portion of a WUP). A nucleic acid molecule that is complementary to the nucleotide sequence shown in SEQ ID NOS: 5 or 7, is one that is sufficiently complementary to the nucleotide sequence shown in SEQ ID NOS: 5 or 7, that it can hydrogen bond with little or no mismatches to the nucleotide sequence shown in SEQ ID NOS: 5 or 7, thereby forming a stable duplex.

"Complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof Binding includes ionic, non-ionic, van der Waals, hydrophobic interactions, and the like. A physical interaction can be either direct or indirect Indirect interactions may be through or due to the effects of another polypeptide or compound. Direct binding refers to interactions that do not take place through, or due to, the effect of another polypeptide or compound, but instead are without other substantial chemical intermediates.

Nucleic acid fragments are at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full-length sequence. Fragments may be derived from any contiguous portion of a nucleic acid or amino acid sequence of choice.

5. *derivatives*, *and analogs*

Derivatives are nucleic acid sequences or amino acid sequences formed from the native compounds either directly or by modification or partial substitution. Analogs are nucleic acid sequences or amino acid sequences that have a structure similar to, but not identical to, the native compound but differ from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid, as described below. Derivatives or analogs of the nucleic acids or proteins include, but are not limited to, molecules comprising regions that are substantially homologous to nucleic acids or proteins in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions (Ausubel et al., 1987).

6. *homology*

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of WUP. Isoforms can be expressed in different tissues of the same organism as a result of, for example; alternative splicing of RNA. Alternatively, different genes can encode isoforms. In the invention, homologous nucleotide sequences include nucleotide sequences encoding for a WUP of species other than humans, including, but not limited to: vertebrates, and thus can include, *e.g.*, frog, mouse, rat, rabbit, dog, cat, cow, horse, and other organisms insofar as there fall under the claims. Homologous nucleotide sequences also include, but are not limited to, naturally occurring allelic variations and mutations of the nucleotide sequences set forth herein. A homologous nucleotide sequence does not, however, include the exact nucleotide sequence encoding human WUP. Homologous nucleic acid sequences include those nucleic acid sequences that encode conservative amino acid substitutions (see below) in SEQ ID NOS:6 or 8, as well as a polypeptide possessing WUP biological activity. Various biological activities of the WUP are described below. insofar as there fall under the claims.

7. *open reading frames*

The open reading frame (ORF) of a WUP gene encodes WUP. An ORF is a nucleotide sequence that has a start codon (ATG) and terminates with one of the three "stop" codons (TAA, TAG, or TGA). In this invention, however, an ORF may be any part of a coding sequence that may or may not comprise a start codon and a stop codon. To achieve a unique sequence, preferable WUP ORFs encode at least 50 amino acids.

*WUP polypeptides*

1. *mature*

A WUP can encode a mature WUP. A "mature" form of a polypeptide or disclosed in the present invention is the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an open leading frame described herein. The product "mature** form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an open leading fame, or the proteolytic cleavage of a signal peptide or leader sequence. Thus a mature form arizing form a precursor polypeptide or protein that has residues 1 to N, where residue 1 is the N-terminal methionine, would have residues 2 through N remaining after removal of the N terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event Such additional processes include, by way of non-limiting example, glycosylation, myristoylation or phosphorylation. In general, a mature polypeptide or protein may result from the operation of only one of these processes, or a combination of any of them.

2. *active*

An active WUP polypeptide retains a biological and/or an immunological activity similar, but not necessarily identical, to an activity of a naturally-occuring (wild-type) WUP polypeptide of the invention, including mature forms. A particular biological assay, with or without dose dependency, can be used to determine WUP activity. Immunological activity refers to the ability to induce the production of an antibody against an antigenic epitope possessed by a native WUP; biological activity refers to a function, either inhibitory or stimulatory, caused by a native WUP that excludes immunological activity.

WUP *nucleic acid variant and hybridization*

1. *variant polynucleotides, genes and recombinant genes* The invention further encompasses nucleic acid molecules having the sequence identity as defined in the claims that differ from the nucleotide sequences shown in SEQ ID NOS: 5 or 7 due to degeneracy of the genetic code and thus encode the same WUP as that encoded by the nucleotide sequences shown in. SEQ ID NO NOS: 5 or 7. An isolated nucleic acid molecule of the invention has a nucleotide sequence encoding a protein having an amino acid sequence shown in SEQ D NOS: 6 or 8.

In addition to the *WUP* sequences shown in SEQ ID NOS: 5 or 7, DNA sequence polymorphisms that change the amino acid sequences of the WUP may exist within a population. For example, allelic variation among individuals will exhibit genetic polymorphism in *WUP.* The terms "gene" and "recombinant gene" refer to nucleic acid molecules comprising an open reading frame (ORF) encoding WUP, preferably a vertebrate WUP. Such natural allelic variations can typically result in 1-5% variance in *WUP.* Any and all such nucleotide variations and resulting amino acid polymorphisms in the WUP, which are the result of natural allelic variation and that do not alter the functional activity of the WUP are within the scope of the invention.

Moreover, *WUP* from other species that have a nucleotide sequence that differs from the sequence of SEQ ID NOS: 5 or 7, are contemplated as defined in the claims. Nucleic acid molecules corresponding to natural allelic variants and homologues of the *WUP* cDNAs can be isolated based on their homology to the *WUP* of SEQ ID NOS: 5 or 7 using cDNA-derived probes to hybridize to homologous WUP sequences under stringent conditions.

"WUP variant polynucleotide" or "WUP variant nucleic acid sequence" means a nucleic acid molecule which encodes an active WUP polypeptide as defined in the claims, and has (1) at least 80% nucleic acid sequence identity with SEQ ID NO: 7 or at least 90% identity with SEQ ID NO:5, (2) a full-length native WUP lacking the signal peptide. Ordinarily, a WUP variant polynucleotide will have at least 80% nucleic acid sequence identity to SEQ ID NO:7, or at least 90% nucleic acid sequence identity with SEQ ID NO:5 and yet more preferably at least about 98 % nucleic acid sequence identity with the nucleic acid sequence of SEQ ID NO:5 or 7. A WUP variant polynucleotide may encode full-length native WUP lacking the signal peptide. Variants do not encompass the native nucleotide sequence.

"Percent (%) nucleic acid sequence identity" with respect to WUP-encoding nucleic acid sequences identified herein is defined as the percentage of nucleotides in a candidate sequence that are identical with the nucleotides in the *WUP* sequence of interest, after aligning the sequences and introducing gaps, if necessary, to achieve the maximum percent sequence identity. Alignment for purposes of determining % nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

When nucleotide sequences are aligned, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) can be calculated as follows:

$$\%\text{nucleic acid sequence identity} = W/Z \cdot 100$$

where

W is the number of nucleotides cored as identical matches by the sequence alignment program's or algorithm's alignment of C and D
and
Z is the total number of nucleotides in D.

When the length of nucleic acid sequence C is not equal to the length of nucleic acid sequence D, the % nucleic acid sequence identity of C to D will not equal the % nucleic acid sequence identity of D to C.

2. *Stringency*

Homologs (*i.e*., nucleic acids encoding WUP derived from species other than human) or other related sequences (*e.g.*, paralogs) can be obtained by low, moderate or high stringency hybridization with all or a portion of the particular human sequence as a probe using methods well known in the art for nucleic acid hybridization and cloning.

The specificity of single stranded DNA to hybridize complementary fragments is determined by the "stringency" of the reaction conditions. Hybridization stringency increases as the propensity to form DNA duplexes decreases. In nucleic acid hybridization reactions, the stringency can be chosen to either favor specific hybridizations (high stringency), which can be used to identify, for example, full-length clones from a library. Less-specific hybridizations (low stringency) can be used to identify related, but not exact, DNA molecules (homologous, but not identical) or segments.

DNA duplexes are stabilized by: (1) the number of complementary base pairs, (2) the type of base pairs, (3) salt concentration (ionic strength) of the reaction mixture, (4) the temperature of the reaction, and (5) the presence of certain organic solvents, such as formamide which decreases DNA duplex stability. In general, the longer the probe, the higher the temperature required for proper annealing. A common approach is to vary the temperature: higher relative temperatures result in more stringent reaction conditions. (Ausubel et al., 1987) provide an excellent explanation of stringency of hybridization reactions.

To hybridize under "stringent conditions" describes hybridization protocols in which nucleotide sequences at least 60% homologous to each other remain hybridized. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium.

(a) *high stringency*

"Stringent hybridization conditions" conditions enable a probe, primer or oligonucleotide to hybridize only to its target sequence. Stringent conditions are sequence-dependent and will differ. Stringent conditions comprise: (1) low ionic strength and high temperature washes (*e.g.* 15 mM sodium chloride, 1.5 mM sodium citrate, 0.1 % sodium dodecyl sulfate at 50˚C); (2) a denaturing agent during hybridization (*e.g.* 50% (v/v) formamide, 0.1% bovine serum albumin, 0.1% Ficoll, 0.1% polyvinylpyrrolidone, 50mM sodium phosphate buffer (pH 6.5; 750 mM sodium chloride, 75 mM sodium citrate at 42˚C); or (3) 50% formamide. Washes typically also comprise 5X SSC (0.75 M NaCl, 75 mM sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42˚C, with washes at 42˚C in 0.2 x SSC (sodium chloride/sodium citrate) and 50% formamide at 55˚C, followed by a high-stringency wash consisting of 0.1 x SSC containing EDTA at 55˚C. Preferably, the conditions are such that sequences at least about 65%, 70%, 75%, 85%, 90%, 95%, 98%, or 99% homologous to each other typically remain hybridized to each other. These conditions are presented as examples and are not meant to be limiting.

(b) *moderate stringency*

"Moderately stringent conditions" use washing solutions and hybridization conditions that are less stringent (Sambrook, 1989), such that a polynucleotide will hybridize to the entire derivatives or analogs of SEQ ID NOS: 5 or 7. One example comprises hybridization in 6X SSC, 5X Denhardt's solution, 0.5% SDS and 100 mg/ml denatured salmon sperm DNA. at 55˚C, followed by one or more washes in 1X SSC, 0.1% SDS at 37˚C The temperature, ionic strength, *etc*., can be adjusted to accommodate experimental factors such as probe length. Other moderate stringency conditions are described in (Ausubel et al., 1987; Kriegler, 1990).

(c) *low stringency*

"Low stringent conditions" use washing solutions and hybridization conditions that are less stringent than those for moderate stringency (Sambrook, 1989), such that a polynucleotide will hybridize to the entire, derivatives or analogs of SEQ ID NOS: 5 or 7. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40˚C, followed by one or mare washes in 2X SSC, 25 mM Tris-HCl (pH 7.4), 5 mM EDTA, and 0.1% SDS at 50˚C. Other conditions of low stringency, such as those for cross-species hybridizations are described in (Ausubel et al., 1987; Kriegler, 1990; Shilo and Weinberg. 1981).

3. *Conservative mutations*

In addition to nauvauy-occurring allelic variants of *WUP*, changes can be introduced by mutation into SEQ ID NOS: 5 or 7 that incur alterations in the acid sequences of the encoded WUP that do not alter WUP function. For example, nucleotide substitutions leading to amino acid substitutions at "non-essential" amino acid residues can be made in the sequence of SEQ ID NOS: 6 or 8. A "non-essential" amino acid residue is a residue that can be altered from the wild-type sequences of the WUP without altering their biological activity, whereas an "essential' amino add residue is required for such biological activity. For example, amino acid residues that are conserved among the WUP of the invention are predicted to be particularly non-amenable to alteration. Amino acids for which conservative substitutions can be made are well known in the art

Useful conservative substitutions are shown in Table A, "Preferred substitutions." Conservative substitutions whereby an amino acid of one class is replaced with another amino acid of the same type fall within the scope of the subject invention so long as the substitution does not materially alter the biological activity of the compound. If such substitutions result in a change in biological activity, then more substantial changes, indicated in Table B as exemplary are introduced and the products screened for WUP polypeptide biological activity.

Table A Preferred substitutions

| Original residue | Exemplary substitutions | Preferred substitutions |
|---|---|---|
| Ala (A) | Val, Leu, TIe | Val |
| Arg (R) | Lys, Gin, Asn | Lys |
| Asn (N) | Gin, His, Lys, Arg | Gin |

(continued)

| Original residue | Exemplary substitutions | Preferred substitutions |
|---|---|---|
| Asp (D) | Glu | Glu |
| Cys (C) | Ser | Ser |
| Gin (Q) | Asn | Asn |
| Glu (E) | Asp | Asp |
| Gly (G) | Pro, Ala | Ala |
| His (H) | Asn, Gln, Lys, Arg | Arg |
| Ile (I) | Leu, Val, Met, Ala, Phe, Norleucine | Leu |
| Leu (L) | Norleucine, Be, Val, Met, Ala, Phe | Ile |
| Lys (K) | Arg, Gln, Asn | Arg |
| Met (M) | Leu, Phe, Ile | Leu |
| Phe (F) | Leu, Val, Ile, Ala, Tyr | Leu |
| Pro (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) Val (V) | Ile, Leu, Met, Phe, Ala, Ile, Leu Norleucine Ala | Leu |

Non-conservative substitutions that effect (1) the structure of the polypeptide backbone, such as a β-sheet or α-helical conformation, (2) the charge or (3) hydrophobicity, or (4) the bulk of the side chain of the target site can modify WUP polypeptide function or immunological identity. Residues are divided into groups based on common side-chain properties as denoted in Table B. Non-conservative substitutions entail exchanging a member of one of these classes for another class. Substitutions may be introduced into conservative substitution sites or more preferably into non-conserved sites.

**Table B** Amino acid classes

| Class | Amino acids |
|---|---|
| hydrophobic | Norleucine, Met, Ala, Val, Leu, Ile |
| neutral hydrophilic | Cys, Ser, Tbr |
| acidic | Asp, Glu |
| basic | Asn; Gln, His, Lys, Arg |
| disrupt chain conformation | Gly, Pro |
| aromatic | Trp, Tyr, Phe |

The variant polypeptides can be made using methods known in the art such as oligonucleotide-mediated (site-directed) mutagenesis, alanine scanning, and PCR mutagenesis. Site-directed mutagenesis (Carter, 1986; Zoller and Smith, 1987), cassette mutagenesis, restriction selection mutagenesis (Wells et al., 1985) or other known techniques can be performed on the cloned DNA to produce the WUP variant DNA (Ausubel et al., 1987; Sambrook, 1989).

In one embodiment, the isolated nucleic acid molecule comprises a nucleotide sequence encoding a protein, wherein the protein comprises an ammo acid sequence at least 80%, 90%, and most preferably about 95% homologous to SEQ ID NOS:6 or 8.

4. *Anti-sence nucleic acids*

Using antisense and sense WUP oligonucleotides can prevent WUP polypeptide expression. These oligonucleotides bind to target nucleic acid sequences, forming duplexes that block transcription or translation of the target sequence by enhancing degradation of the duplexes, terminating prematurely transcription or translation, or by other means.

Antisense or sense oligonucleotides are singe-stranded nucleic acids, either RNA or DNA, which can bind target *WUP* mRNA (sense) or *WUP* DNA (antisense) sequences Anti-sense nucleic acids can be designed according to Watson and Crick or Hoogsteen base pairing rules. The anti-sense nucleic acid molecule can be complementary to the entire coding region of *WUP* mRNA, but more preferably, to only a portion of the coding or noncoding region of *WUP* mRNA. For example, the anti-sense oligonucleotide can be complementary to the region surrounding the translation start site of *WUP* mRNA. Antisense or sense oligonucleotides may comprise a fragment of the WUP DNA coding region of at least about 14 nucleotides, preferably from about 14 to 30 nucleotides. In general, antisense RNA or DNA molecules can comprise at least 5, 10, 15, 20, 25, 30, 35, 40, 45, 50, 55, 60, 65, 70, 75, 80, 85, 90, 95, 100 bases in length or more. Among others, (Stein and Cohen, 1988; van der Krol et al., 1988b) describe methods to derive antisense or a sense oligonucleotides from a given cDNA sequence.

Examples of modified nucleotides that can be used to generate the anti-sense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-dian-tinopuiine. Alternatively, the anti-sense nucleic acid can be produced biologically using an expression vector into which a nucleic acid has been sub-cloned in an anti-sense orientation such that the transcribed RNA will be complementary to a target nucleic acid of interest.

To introduce antisense or sense oligonucleotides into target cells (cells containing the target nucleic acid sequence), any gene transfer method may be used Examples of gene transfer methods include (1) biological, such as gene transfer vectors like Epstein-Barr virus or conjugating the exogenous DNA to a ligand-binding molecule, (2) physical, such as electroporation and injection, and (3) chemical, such as $CaPO_4$ precipitation and oligonucleotide-lipd complexes.

An antisense or sense oligonucleotide is inserted into a suitable gene transfer retroviral vector. A cell containing the target nucleic acid sequence is contacted with the recombinant retroviral vector, either *in vivo* or *ex vivo*. Examples of suitable retroviral vectors include those derived from the murine retrovirus M-MuLV, N2 (a retrovirus derived from M-MuLV), or the double copy vectors designated DCT5A, DCT5B and DCT5C (WO 90/13641, 1990). To achieve sufficient nucleic acid molecule transcription, vector constructs in which the transcription of the anti-sense nucleic acid molecule is controlled by a strong pol II or pol III promoter are preferred.

To specify target cells in a mixed population of cells cell surface receptors that are specific to the target cells can be exploited. Antisense and sense oligonucleotides are conjugated to a ligand-binding molecule, as described in (WO 91/04753, 1991). Ligands are chosen for receptors that are specific to the target cells. Examples of suitable ligand-binding molecules include cell surface receptors, growth factors, cytokines, or other ligands that bind to cell surface receptors or molecules. Preferably, conjugation of the ligand-binding molecule does not substantially interfere with the ability of the receptors or molecule to bind the ligand-binding molecule conjugate, or block entry of the sense or antisense oligonucleotide or its conjugated version into the cell.

Liposomes efficiently transfer sense or an anti sense oligonucleotide to cells (WO 90/16448, 1990). The sense or antisense oligonucleotide-lipid complex is preferably dissociated within the cell by an endogenous lipase.

The anti-sense nucleic acid molecule of the invention may be an $\alpha$-anomeric nucleic add molecule. An $\alpha$-anomeric nucleic acid molecule forms specific double-stranded hybrids with complementary RNA in which, contrary to the usual $\alpha$-units, the strands run parallel to each other (Gautier et al., 1987). The anti-sense nucleic acid molecule can also comprise a 2'-o-methylribonucleotide (Inoue et al,.1987a) or a chimeric RNA-DNA analogue (Inoue et al.,1987b).

An anti-sense nucleic acid of the invention may be a ribozyme. Ribozymes are catalytic RNA molecules with ribonuclease activity that are capable of cleaving a single-stranded nucleic acid, such as an mRNA, to which they have a complementary region. Thus, ribozymes, such as hammerhead ribozymes (Haseloff and Gerlach, 1988) can be used to catalytically cleave *WUP* mRNA transcripts and thus inhibit translation. A ribozyme specific for a WUP-encoding nucleic acid can be designed based on the nucleotide ,sequence of a WUP cDNA (*i.e.* SEQ ID NOS: 5 or 7). For example, a derivative of a *Tetrahymena* L-19- IVS RNA can be constructed in which the nucleotide sequence of the active site is complementary to the nucleotide sequence to be cleaved in a WUP-encoding mRNA (Cech et al., U.S Patent No.

5,116,1992; Cech et al, U.S. Patent No. 4,987,071, 1991). WUP mRNA can also be used to select a catalytic RNA having a specific ribonuclease activity from a pool of RNA molecules (Bartel and Szostak, 1993).

Alternatively, *WUP* expression can be inhibited by targeting nucleotide sequences complementary to the regulatory region of the *WUP* (*e.g.*, the *WUP* promoter and/of enhancers) to form triple helical structure that prevent transcription of the *WUP* in target cells (Helene, 1991; Helene et al., 1992; Maher, 1992).

Modifications of antisense and sense oligonucleotides can augment their effectiveness. Modified sugar-phosphodiester bonds or other sugar linkages (WO 91/0.6629, 1991); increase *in vivo* stability by conferring resistance to endogenous nucleases without disrupting binding specificity to target sequences. Other modifications can increase the affinities of the oligonucleotides for their targets, soch as covalently linked organic moieties (WO 90/10448, 1990) or poly-(L)-lysine. Other attachments modify binding specificities of the oligonucleotides for their targets, including metal complexes or intercalating (*e.g.* ellipticine) and alkylating agents.

For example, the deoxyribose phosphate backbone of the nucleic acids can be modified to generate peptide nucleic acids (Hyrup and Nielsen, 1996). "Peptide nucleic acids" or "PNAs" refer to nucleic acid mimics (*e.g.*, DNA mimics) in that the deoxyribose phosphate backbone is replaced by a pseudopeptide backbone and only the four natural nucleobases are retained The neutral backbone of PNAs allows for specific hybridization to DNA and RNA under conditions of low ionic strength. The synthesis of PNA oligomers can be performed using standard solid phase peptide synthesis protocols (Hyrup and Nielsen, 1996; Perry-O'Reefe et al.,. 1996).

PNAs of WUP can be used in therapeutic and diagnostic applications. For example, PNAs can be used as anti-sense or antigene agents for sequence-specific modulation of gene expression by inducing transcription or translation arrest or inhibiting replication. WUP PNAs may also be used in the analysis of single base pair mutations (*e.g.*, PNA directed PCR clamping; as artificial restriction enzymes when used in combination with other enzymes, *e.g.*, $S_1$ nucleases (Hyrup and Nielsen, 1996); or as probes or primers for DNA sequence and hybridization (Hyrup and Nielsen, 1996; Perry-O'Keefe et al., 1996).

PNAs of WUP can be modified to enhance their stability or cellular uptake. Lipophilic or other helper groups may be attached to PNAs, PNA-DNA dimmers formed, or the use of liposomes or other drug delivery techniques. For example, PNA-DNA chimeras can be generated that may combine the advantageous properties of PNA and DNA. Such chimeras allow DNA recognition enzymes (*e.g.*, RNase H and DNA polymerases) to interact with the DNA portion while the PNA portion provides high binding affinity and specificity. PNA-DNA chimeras can be linked using linkers of appropriate lengths selected in terms of base stacking, number of bonds between the nucleobases, and orientation (Hyrup and Nielsen, 1996). The synthesis of PNA-DNA chimeras can be performed (Finn et al., 1996; Hyrup and Nielsen, 1996). For example, a DNA chain can be synthesized on a solid support using standard phosphoramidite coupling chemistry, and modified nucleoside analogs, *e.g.*, 5'-(4-methoxytrityl)amino-5'-deoxy-thymidine phosphoramidite, can be used between the PNA and the 5' end of DNA (Finn et al., 1996; Hyrup and Nielsen, 1996). PNA monomers are then coupled in a stepwise manner to produce a chimeric molecule with a 5' PNA segment and a 3' DNA segment (Finn et al., 1996). Alternatively, chimeric molecules can be synthesized with a 5' DNA segment and a 3' PNA segment (Petersen et al., 1976).

The oligonucleotide may include other appended groups such as peptides (*e.g.*, for targeting host cell receptors *in vivo),* or agents facilitating transport across the cell membrane (Lemaitre et al., 1987; Letsinger et al., 1989) or PCT Publication No. WO88/09810) or the blood-brain barrier (*e.g.*, PCT Publication No. WO 89/10134). In addition, oligonucleotides can be modified with hybridization-triggered cleavage agents (van der Krol et al., 1988a) or intercalating agents (Zon, 1988). The oligonucleotide may be conjugated to another molecule, *e.g.,* a peptide, a hybridization triggered cross-linking agent, a transport agent, a hybridization- polypeptides as defined in the pending claims triggered cleavage agent, and the like.

*WUP polypeptides*

One aspect of the invention pertains to isolated WUP, polypeptides as defined in the pending claims. These polypeptides are suitable for use as immunogens to raise anti-WUP Abs. In one embodiment, native WUP can be isolated from cells or tissue sources by an appropriate purification scheme using standard protein purification techniques. In another embodiment, WUP are produced by recombinant DNA techniques. Alternative to recombinant expression, a WUP or polypeptide can be synthesized chemically using standard peptide synthesis techniques.

1. *Polypeptides*

A WUP polypeptide includes the amino acid sequence of WUP whose sequences are provided in SEQ ID NOS: 6 or 8. The invention also includes a mutant or variant protein having the sequence identity defined in the claims any of whose residues may be changed from the corresponding residues shown in SEQ ID NOS: 6 or 8, while still encoding a protein that maintains its WUP activities and physiological functions, wherein said polypeptide is an active WUP polypeptide.

2. *Variant WUP polypeptides*

In general, a WUP variant is a polypeptide that preserves WUP-like function and includes any variant in which residues at a particular position in the sequence have been substituted by other amino acids, and further includes the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

"WUP polypeptide variant" means an active WUP polypeptide having at least: (1) 80% amino acid sequence identity with SEQ ID NOs: 6 or 8 or (2) a WUP polypeptide sequence consisting of an amino acid sequence of SEQ ID NO : 39. For example, WUP polypeptide variants include WUP polypeptides wherein one or more amino acid residues are added or deleted at the N- or C- terminus of the full-length native amino acid sequence. A WUP polypeptide variant will have at least about 80% amino acid sequence identity, preferably at least about 81% amino acid sequence identity, more preferably at least about 82%, 83%, 84%, 85%, 86%, 87%, 88%, 89%, 90%, 91˚b, 92%. 93%, 94%, 95%, 96%, 97%, 98% amino acid sequence identity and most preferably at least about 99% amino acid sequence identity to the sequence of SEQ ID NOs:6 or 8, wherein said polypeptide is an active WUP polypeptide. A WUP polypeptide variant may have a sequence lacking the signal peptide. Ordinarily, WUP variant polypeptides are at least 100, 150, 200, or 300 amino acids in length, or more.

"Percent (%) amino acid sequence identity" is defined as the percentage of amino acid residues that are identical with amino acid residues in the disclosed WUP polypeptide sequence in a candidate sequence when the two sequences are aligned To determine % amino acid identity, sequences are aligned and if necessary, gaps are introduced to achieve the maximum % sequence identity; conservative substitutions are not considered as part of the sequence identity. Amino acid sequence alignment procedures to determine percent identity are well known to those of skill in the art. Often publicly available computer software such as BLAST, BLAST2, AUGN2 or Megalign (DNASTAR) software is used to align peptide sequences. Those skilled in the art can determine appropriate parameters for measuring alignment, including any algorithms needed to achieve maximal alignment over the full length of the sequences being compared.

When amino acid sequences are aligned, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) can be calculated as:

$$\%\text{amino acid sequence identity} = X/Y \cdot 100$$

where

X is the number of amino acid residues scored as identical matches as by the sequence alignment program's or algorithm's alignment of A and B
and
Y is the total number of amino acid residues in B.

If the length of ammo acid sequence A is not equal to the length of amino acid sequence B, the % amino acid sequence identity of A to B will not equal the % amino acid sequence identity of B to. A.

3. *Isolated/purified polypeptides*

An "isolated" or "purified" polypeptide, protein or biologically active fragment is separated and/or recovered from a component of its natural environment. Contaminant components include materials that would typically interface with diagnostic or therapeutic uses for the polypeptide, and may include enzymes, hormones, and other proteinaceous or non-proteinaceous materials. Preferably, the polypeptide is purified to a sufficient degree to obtain at least 15 residues of N-terminal or internal amino acid sequence. To be substantially isolated, preparations having less than 30% by dry weight of non-WUP contaminating material (contaminants), more preferably less than 20%, 10% and most preferably less than 5% contaminants. An isolated, recombinantly-produced WUP or biologically active portion is preferably substantially free of culture medium, *i.e.*, culture medium represents less than 20%, more preferably less than about 10%, and most preferably less than about 5% of the volume of the WUP preparation. Examples of contaminants include cell debris, culture media, and substances used and produced during *in vitro* synthesis of WUP.

4. *Biologically active*

Biologically active portions of WUP include peptides comprising amino acid sequences sufficiently homologous to or derived from the amino acid sequences of the WUP (SEQ ID NOS: 6 or 8) that include fewer ammo acids than the full-length WUP, and exhibit at least one activity of a WUP. Biologically active portions comprise a domain or motif with at least one activity of native WUP. A biologically active portion of a WUP can be a polypeptide that is, for example, 10, 25,50,100 or more amino acid residues in length. Other biologically active portions, in which other regions of the protein are deleted, can be prepared by recombinant techniques and evaluated for one or more of the functional activities of a native WUP.

Biologically active portions of WUP may have an amino acid sequence shown in SEQ ID NOS: 6 or 8, or substantially homologous to SEQ ID NOS: 6 or 8, and retain the functional activity of the protein of SEQ ID NOS: 6 or 8, yet differs in amino acid sequence due to natural allelic variation or mutagenesis.

5. *Determining homology between two or more sequences*

"WUP variant" means an active WUP having the sequence identity defined in the claims and at least: (1) 80% amino acid sequence identity with SEQ ID NOs 6 or 8 (2) a WUP sequence lacking the signal peptide. For example, WUP variants include WUP wherein one or more amino acid residues are added or deleted at the N- or C- terminus of the full-length native amino acid sequence. A WUP variant will have at least 80% amino acid sequence identity, preferably at least about 81% amino acid sequence identity, more preferably at least about 82%, 83%, 84%, 85%, 86%, 87%, 88%, 89%, 90%, 91%, 92%, 93%, 94%, 95%, 96%, 97%, 98% amino acid sequence identity and most preferably at least about 99% amino acid sequence identity with SEQ ID NOs: 6 or 8. A WUP variant may have a sequence lacking the signal peptide. Ordinarily, WUP variant polypeptides are at least about 80, 90, 100, 150, 200, or 300 amino acids in length, or more.

"Percent (%) amino acid sequence identity" is defined as the percentage of amino acid residues that are identical with amino acid residues in the disclosed WUP sequence in a candidate sequence when the two sequences are aligned. To determine % amino acid identity, sequences are aligned and if necessary, gaps are introduced to achieve the maximum % sequence identity, conservative substitutions are not considered as part of the sequence identity. Amino acid sequence alignment procedures to determine percent identity are well known to those of skill in the art. Often publicly available computer software such as BLAST, BLAST2, ALIGN2 or Megalign (DNASTAR) software is used to align peptide sequences. Those skilled in the art can determine appropriate parameters for measuring alignment, including any algorithms needed to achieve maximal alignment over the full length of the sequences being compared.

When amino acid sequences are aligned, the % amino acid sequence identity of a given amino acid sequence A to, with, or against a given amino add sequence B (which can alternatively be phrased as a given amino acid sequence A that has or comprises a certain % amino acid sequence identity to, with, or against a given amino, acid sequence B) can be calculated as:

$$\%\text{amino acid sequence identity} = X/Y \cdot 100$$

where

X is the number of amino acid residues scored as identical matches by the sequence alignment program's or algorithm's alignment of A and B
and
Y is the total number of amino acid residues in B.

If the length of amino acid sequence A is not equal to the length of amino acid sequence B, the % amino acid sequence identity of A to B will not equal the % amino acid sequence identity of B to A.

6. *Chimeric and fusion proteins*

Fusion polypeptides are useful in expression studies, cell-localization, bioassays, and WUP purification. A WUP "chimeric protein" or "fusion protein" comprises WUP fused to a non-WUP polypeptide. A non-WUP polypeptide is not substantially homologous to WUP (SEQ ID NOS:6 or 8). A WUP fusion protein may include an amino acid sequence having at least 80 % sequence identity to the sequence of SEQ ID NOs: 6 or 8, including any number of the biologically active portions. WUP may be fused to the C-terminus of the GST (glutathione S-transferase) sequences. Such fusion proteins facilitate the purification of recombinant WUP. In certain host cells, (*e.g.* mammalian), heterologous signal sequences fusions may ameliorate WUP expression and/or secretion. Additional exemplary fusions are presented in Table C.

Other fusion partners can adapt WUP therapeutically. Fusions with members of the immunoglobulin (Ig) protein family are useful in therapies that inhibit WUP ligand or substrate interactions, consequently suppressing WUP-mediated signal transduction *in vivo*. WUP-Ig fusion polypeptides can also be used as immunogens to produce anti-WUP Abs in a subject, to purify WUP ligands, and to screen for molecules that inhibit interactions of WUP with other molecules.

Fusion proteins can be easily created using recombinant methods. A nucleic acid encoding WUP can be fused in-frame with a non-WUP encoding nucleic acid, to the WUP $NH_2$- or COO- -terminus, or internally. Fusion genes may also be synthesized by conventional techniques, including automated DNA synthesizers. PCR amplification using anchor primers that give rise to complementary overhangs between two consecutive gene fragments that can subsequently be annealed and reamplified to generate a chimeric gene sequence (Ausubel et al., 1987) is also useful. Many vectors are commercially available that facilitate sub-cloning WUP in-frame to a fusion moiety.

Table C Useful non-WUP fusion polypeptides

| Reporter | *in vitro* | *in vivo* | Notes | Reference |
|---|---|---|---|---|
| Human growth hormone (hGH) | Radioimmuno-assay | none | Expensive, insensitive, narrow linear range. | (Selden et al., 1986) |
| β-glucuronidase (GUS) | Colorimetric, fluorescent, or chemiluminescent | colorimetric (histo-chemical staining with X-gluc) | sensitive, broad linear range, non-iostopic. | (Gallagher, 1992) |
| Green fluorescent protein (GFP) and related molecules (RFP, BFP, WUP, *etc*.) | Fluorescent | fluorescent | can be used in live cells; resists photobleaching | (Chalfie et al., 1994) |
| Luciferase (firefly) | bioluminsecent | Bioluminescent | protein is unstable, difficult to reproduce, signal is brief | (de Wet et al., 1987) |
| Chloramphenicoal acetyltransferase (CAT) | Chromatography, differential extraction, fluorescent, or immunoassay | none | Expensive radioactive substrates, time-consuming, insensitive, narrow linear range | (Gorman et al., 1982) |
| β-galacto-sidase | colorimetric, fluorescence, chemiluminscence | colorimetric (histochemical staining with X- gal), bioluminescent in live cells | sensitive, broad linear range; some cells have high endogenous activity | (Alam and Cook, 1990) |
| Secrete alkaline phosphatase (SEAP) | colorimetric, bioluminescent, chemiluminescent | none | Chemiluminscence assay is sensitive and broad linear range; some cells have endogenouse alkaline phosphatase activity | (Berger et al., 1988) |

*Therapeutic applications of WUP*

1. *Agonists and antagonists*

"Antagonist" includes any molecule that partially or fully blocks, inhibits, or neutralizes a biological activity of endogenous WUP. Similarly, "agonist" includes any molecule that mimics a biological activity of endogenous WUP. Molecules that can act as agonists or antagonists include Abs or antibody fragments, fragments or variants of endogenous WUP, peptides, antisense oligonucleotides, small organic molecules, *etc.*

2. *Identifying antagonists and agonists*

To assay for antagonists, WUP is added to, or expressed in, a cell along with the compound to be screened for a particular activity. If the compound inhibits the activity of interest in the presence of the WUP, that compound is an antagonist to the WUP; if WUP activity is enhanced, the compound is an agonist.

(a) *Specific examples of potential antagonists and agonist*

Any molecule that alters WUP cellular effects is a candidate antagonist or agonist Screening techniques well known to those skilled in the art can identify these molecules. Examples of antagonists and agonists include: (1) small organic and inorganic compounds, (2) small peptides, (3) Abs and derivatives, (4) polypeptides closely related to WUP, (5) antisense DNA and RNA, (6) ribozymes, (7) triple DNA helices and (8) nucleic acid aptamers.

Small molecules that bind to the WUP active site or other relevant part of the polypeptide and inhibit the biological activity of the WUP are antagonists. Examples of small molecule antagonists include small peptides, peptide-like molecules, preferably soluble, and synthetic non-peptidyl organic or inorganic compounds. These same molecules, if they enhance WUP activity, are examples of agonists.

Almost any antibody that affects WUP's function is a candidate antagonist, and occasionally, agonist. Examples of antibody antagonists include polyclonal, monoclonal, single-chain, anti-idiotypic, chimeric Abs, or humanized versions of such Abs or fragments. Abs may be from any species in which an immune response can be raised. Humanized Abs are also contemplated.

Alternatively, a potential antagonist or agonist may be a closely related protein, for example, a mutated form of the WUP that recognizes a WUP-interacting protein but imparts no effect, thereby competitively inhibiting WUP action. Alternatively, a mutated WUP may be constitutively activated and may act as an agonist.

Antisense RNA or DNA constructs can be effective antagonists. Antisense RNA or DNA molecules block function by inhibiting translation by hybridizing to targeted mRNA. Antisense technology can be used to control gene expression through triple-helix formation or antisense DNA or RNA, both of which depend on polynucleotide binding to DNA or RNA. For example, the 5' coding portion of the WUP sequence is used to design an antisense RNA oligonucleotide of from about 10 to 40 base pairs in length. A DNA oligonucleotide is designed to be complementary to a region of the gene involved in transcription (triple helix) (Beal and Dervan, 1991; Cooney et al., 1988; Lee et al., 1979), thereby preventing transcription and the production of the WUP. The antisense RNA oligonucleotide hybridizes to the mRNA *in vivo* and blocks translation of the mRNA molecule into the WUP (antisense) (Cohen, 1989; Okano et al., 1991). These oligonucleotides can also be delivered to cells such that the antisense RNA or DNA may be expressed *in vivo* to inhibit production of the WUP. When antisense DNA is used, oligodeoxyribonucleotides derived from the translation-initiation site, *e.g.*, between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques (WO 97/33551, 1997; Rossi, 1994).

To inhibit transcription, triple-helix nucleic acids that are single-stranded and comprise deoxynucleotides are useful antagonists. These oligonucleotides are designed such that triple-helix formation via Hoogsteen base-pairing rules is promoted, generally requiring stretches of purines or pyrimidines (WO 97/33551, 1997).

Aptamers are short oligonucleotide sequences that can be used to recognize and specifically bind almost any molecule. The systematic evolution of ligands by exponential enrichment (SELEX) process (Ausubel et al., 1987; Ellington and Szostak, 1990; Tuerk and Gold, 1990) is powerful and can be used to find such aptamers. Aptamers have many diagnostic and clinical uses; almost any use in which an antibody has been used clinically or diagnostically, aptamers too may be used. In addition, are cheaper to make once they have been identified, and can be easily applied in a variety of formats, including administration in pharmaceutical compositions, in bioassays, and diagnostic tests (Jayasena, 1999).

*Anti-WUP Abs*

The invention encompasses Abs and antibody fragments, such as $F_{ab}$ or $(F_{ab})_2$, that bind immunospecifically to any WUP epitopes, as defined in the claims.

"Antibody" (Ab) comprises single Abs directed against WUP (anti-WUP Ab; including agonist, antagonist, and neutralizing Abs), anti-WUP Ab compositions with poly-epitope specificity, single chain anti-WUP Abs, and fragments of anti-WUP Abs. A "monoclonal antibody" is obtained from a population of substantially homogeneous Abs, i.e., the individual Abs comprising the population are identical except for possible naturally-occuning mutations that may be present in minor amounts. Exemplary Abs include polyclonal (pAb), monoclonal (mAb), humanized, bi-specific (bsAb), and heteroconjugate Abs.

*1. Polyclonal Abs (pAbs)*

Polyclonal Abs can be raised in a mammalian host, for example, by one or more injections of an immunogen and, if desired, an adjuvant Typically, the immunogen and/or adjuvant are injected in the mammal by multiple subcutaneous or intraperitoneal injections. The immunogen may include WUP or a fusion protein. Examples of adjuvants include Freund's complete and monophosphoryl Lipid A synthetic-trehalose dicorynomycolate (MPL-TDM). To improve the immune response, an immunogen may be conjugated to a protein that is immunogenic in the host, such as keyhole limpet hemocyanin (KLH), serum albumin, bovine thyroglobulin, and soybean trypsin inhibitor. Protocols for antibody production are described by (Ausubel et al., 1987; Harlow and Lane, 1988). Alternatively, pAbs may be made in chickens, producing IgY molecules (Schade et al., 1996).

*2. Monoclonal Abs (mAbs)*

Anti-WUP mAbs may be prepared using hybridoma methods (Milstein and Cuello, 1983). Hybridoma methods comprise at least four steps: (1) immunizing a host, or lymphocytes from a host; (2) harvesting the mAb secreting (or potentially secreting) lymphocytes, (3) fusing the lymphocytes to immortalized cells, and (4) selecting those cells that secrete the desired (anti-WUP) mAb.

A mouse, rat, guinea pig, hamster, or other appropriate host is immunized to elicit lymphocytes that produce or are capable of producing Abs that will specifically bind to the immunogen. Alternatively, the lymphocytes may be immunized *in vitro.* If human cells are desired, peripheral blood lymphocytes (PBLs) are generally used; however, spleen cells or lymphocytes from other mammalian sources are preferred. The immunogen typically includes WUP or a fusion protein.

The lymphocytes are then fused with an immortalized cell line to form hybridoma cells, facilitated by a fusing agent such as polyethylene glycol (Goding, 1996). Rodent, bovine, or human myeloma cells immortalized by transformation may be used, or rat or mouse myeloma cell lines. Because pure populations of hybridoma cells and not unfused immortalized cells are preferred, the cells after fusion are grown in a suitable medium that contains one or more substances that inhibit the growth or survival of unfused, immortalized cells. A common technique uses parental cells that lack the enzyme hypoxanthine guanine phosphoribosyl transferase (HGPRT or HPRT). In this case, hypoxanthine, aminopterin and thymidine are added to the medium (HAT medium) to prevent the growth of HGPRT-deficient cells while permitting hybridomas to grow.

Preferred immortalized cells fuse efficiently; can be isolated from mixed populations by selecting in a medium such as HAT; and support stable and high-level expression of antibody after fusion. Preferred immortalized cell lines are murine myeloma lines, available from the American Type Culture Collection (Manassas, VA). Human myeloma and mouse-human heteromyeloma cell lines also have been described for the production of human mAbs (Kozbor et al., 1984; Schook, 1987).

Because hybridoma cells secrete antibody extracellularly, the culture media can be assayed for the presence of mAbs directed against WUP (anti-WUP mAbs). Immunoprecipitation or *in vitro* binding assays, such as radio immunoassay (RIA) or enzyme-linked immunoabsorbent assay (ELISA), measure the binding specificity of mAbs (Harlow and Lane, 1988; Harlow and Lane, 1999), including Scatchard analysis (Munson and Rodbard, 1980).

Anti-WUP mAb secreting hybridoma cells may be isolated as single clones by limiting dilution procedures and sub-cultured (Goding, 1996). Suitable culture media include Dulbecco's Modified Eagle's Medium, RPMI-1640, or if desired, a protein-free or -reduced or serum-free medium (*e.g.*, Ultra DOMA PF or HL-1; Biowhittaker; Walkersville, MD). The hybridoma cells may also be grown *in vivo* as ascites.

The mAbs may be isolated or purified from the culture medium or ascites fluid by conventional Ig purification procedures such as protein A-Sepharose, hydroxylapatite chromatography, gel electrophoresis, dialysis, ammonium sulfate precipitation or affinity chromatography (Harlow and Lane, 1988; Harlow and Lane, 1999).

The mAbs may also be made by recombinant methods (U.S. Patent No. 4166452, 1979). DNA encoding anti-WUP mAbs can be readily isolated and sequenced using conventional procedures, *e.g.*, using oligonucleotide probes that specifically bind to murine heavy and light antibody chain genes, to probe preferably DNA isolated from anti-WUP-secreting mAb hybridoma cell lines. Once isolated, the isolated DNA fragments are sub-cloned into expression vectors that are then transfected into host cells such as simian COS-7 cells, Chinese hamster ovary (CHO) cells, or myeloma cells that do not otherwise produce Ig protein, to express mAbs. The isolated DNA fragments can be modified, for example, by substituting the coding sequence for human heavy and light chain constant domains in place of the homologous murine sequences (U.S. Patent No. 4816567, 1989; Morrison et al., 1987), or by fusing the Ig coding sequence to all or part of the coding sequence for a non-Ig polypeptide. Such a non-Ig polypeptide can be substituted for the constant domains of an antibody, or can be substituted for the variable domains of one antigen-combining site to create a chimeric bivalent antibody.

3. *Monovalent Abs*

The Abs may be monovalent Abs that consequently do not cross-link with each other. For example, one method involves recombinant expression of Ig light chain and modified heavy chain. Heavy chain truncations generally at any point in the $F_c$ region will prevent heavy chain cross-linking. Alternatively, the relevant cysteine residues are substituted with another amino acid residue or are deleted, preventing crosslinking. *In vitro* methods are also suitable for preparing monovalent Abs. Abs can be digested to produce fragments, such as $F_{ab}$ fragments (Harlow and Lane, 1988; Harlow and Lane, 1999).

4. *Humanized and human Abs*

Anti-WUP Abs may further comprise humanized or human Abs. Humanized forms of non-human Abs are chimeric Igs, Ig chains or fragments (such as $F_v$, $F_{ab}$, $F_{ab}$', $F_{(ab')2}$ or other antigen-binding subsequences of Abs) that contain minimal sequence derived from non-human Ig.

Generally, a humanized antibody has one or more amino acid residues introduced from a non-human source. These non-human amino acid residues are often referred to as "import" residues, which are typically taken from an "import" variable domain. Humanization is accomplished by substituting rodent CDRs or CDR sequences for the corresponding sequences of a human antibody (Jones et al., 1986; Riechmann et al., 1988; Verhoeyen et al., 1988). Such "humanized" Abs are chimeric Abs (U.S. Patent No. 4816567, 1989), wherein substantially less than an intact human variable domain has been substituted by the corresponding sequence from a non-human species. In practice, humanized Abs are typically human Abs in which some CDR residues and possibly some FR residues are substituted by residues from analogous sites in rodent Abs. Humanized Abs include human Igs (recipient antibody) in which residues from a complementary determining region (CDR) of the recipient are replaced by residues from a CDR of a non-human species (donor antibody) such as mouse, rat or rabbit, having the desired specificity, affinity and capacity. In some instances, corresponding non-human residues replace $F_v$ framework residues of the human Ig. Humanized Abs may comprise residues that are found neither in the recipient antibody nor in the imported CDR or framework sequences. In general, the humanized antibody comprises substantially all of at least one, and typically two, variable domains, in which most if not all of the CDR regions correspond to those of a non-human Ig and most if not all of the FR regions are those of a human Ig consensus sequence. The humanized antibody optimally also comprises at least a portion of an Ig constant region ($F_c$), typically that of a human Ig (Jones et al., 1986; Presta, 1992; Riechmann et al., 1988).

Human Abs can also be produced using various techniques, including phage display libraries (Hoogenboom et al., 1991; Marks et al., 1991) and the preparation of human mAbs (Boerner et al., 1991; Reisfeld and Sell, 1985). Similarly, introducing human Ig genes into transgenic animals in which the endogenous Ig genes have been partially or completely inactivated can be exploited to synthesize human Abs. Upon challenge, human antibody production is observed, which closely resembles that seen in humans in all respects, including gene rearrangement, assembly, and antibody repertoire (U.S. Patent No. 5545807, 1996; U.S. Patent No. 5545806, 1996; U.S. Patent No. 5569825, 1996; U.S. Patent No. 5633425, 1997; U.S. Patent No. 5661016, 1997; U.S. Patent No. 5625126, 1997; Fishwild et al., 1996; Lonberg and Huszar, 1995; Lonberg et al., 1994; Marks et al., 1992).

5. *Bi-specific mAbs*

Bi-specific Abs are monoclonal, preferably human or humanized, that have binding specificities for at least two different antigens. For example, a binding specificity is WUP; the other is for any antigen of choice, preferably a cell-surface protein or receptor or receptor subunit.

Traditionally, the recombinant production of bi-specific Abs is based on the co-expression of two Ig heavy-chain/light-chain pairs, where the two heavy chains have different specificities (Milstein and Cuello, 1983). Because of the random assortment of Ig heavy and light chains, the resulting hybridomas (quadromas) produce a potential mixture of ten different antibody molecules, of which only one has the desired bi-specific structure. The desired antibody can be purified using affinity chromatography or other techniques (WO 93/08829, 1993; Traunecker et al., 1991).

To manufacture a bi-specific antibody (Suresh et al., 1986), variable domains with the desired antibody-antigen combining sites are fused to Ig constant domain sequences. The fusion is preferably with an Ig heavy-chain constant domain, comprising at least part of the hinge, CH2, and CH3 regions. Preferably, the first heavy-chain constant region (CH1) containing the site necessary for light-chain binding is in at least one of the fusions. DNAs encoding the Ig heavy-chain fusions and, if desired, the Ig light chain, are inserted into separate expression vectors and are co-transfected into a suitable host organism.

The interface between a pair of antibody molecules can be engineered to maximize the percentage of heterodimers that are recovered from recombinant cell culture (WO 96/27011, 1996). The preferred interface comprises at least part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This mechanism increases the yield of the heterodimer over unwanted end products such as homodimers.

Bi-specific Abs can be prepared as full length Abs or antibody fragments (e.g. $F_{(ab')2}$ bi-specific Abs). One technique to generate bi-specific Abs exploits chemical linkage. Intact Abs can be proteolytically cleaved to generate $F_{(ab')2}$ fragments (Brennan et al., 1985). Fragments are reduced with a dithiol complexing agent, such as sodium arsenite, to stabilize vicinal dithiols and prevent intermolecular disulfide formation. The generated $F_{ab'}$ fragments are then converted to thionitrobenzoate (TNB) derivatives. One of the $F_{ab'}$-TNB derivatives is then reconverted to the $F_{ab'}$-thiol by reduction with mercaptoethylamine and is mixed with an equimolar amount of the other $F_{ab'}$-TNB derivative to form the bi-specific antibody. The produced bi-specific Abs can be used as agents for the selective immobilization of enzymes.

$F_{ab'}$ fragments may be directly recovered from *E. coli* and chemically coupled to form bi-specific Abs. For example, fully humanized bi-specific $F_{(ab')2}$ Abs can be produced (Shalaby et al., 1992). Each $F_{ab'}$ fragment is separately secreted from *E. coli* and directly coupled chemically *in vitro,* forming the bi-specific antibody.

Various techniques for making and isolating bi-specific antibody fragments directly from recombinant cell culture have also been described. For example, leucine zipper motifs can be exploited (Kostelny et al., 1992). Peptides from the *Fos* and *Jun* proteins are linked to the $F_{ab'}$ portions of two different Abs by gene fusion. The antibody homodimers are reduced at the hinge region to form monomers and then reoxidized to form antibody heterodimers. This method can also produce antibody homodimers. The "diabody" technology (Holliger et al., 1993) provides an alternative method to generate bi-specific antibody fragments. The fragments comprise a heavy-chain variable domain ($V_H$) connected to a light-chain variable domain ($V_L$) by a linker that is too short to allow pairing between the two domains on the same chain. The $V_H$ and $V_L$ domains of one fragment are forced to pair with the complementary $V_L$ and $V_H$ domains of another fragment, forming two antigen-binding sites. Another strategy for making bi-specific antibody fragments is the use of single-chain $F_v$ ($sF_v$) dimers (Gruber et al., 1994). Abs with more than two valencies are also contemplated, such as tri-specific Abs (Tutt et al., 1991).

Exemplary bi-specific Abs may bind to two different epitopes on a given WUP. Alternatively, cellular defense mechanisms can be restricted to a particular cell expressing the particular WUP: an anti-WUP arm may be combined with an arm that binds to a leukocyte triggering molecule, such as a T-cell receptor molecule (e.g. CD2, CD3, CD28, or B7), or to $F_c$ receptors for IgG ($F_c\gamma R$), such as $F_c\gamma RI$ (CD64), $F_c\gamma RII$ (CD32) and $F_c\gamma RIII$ (CD16). Bi-specific Abs may also be used to target cytotoxic agents to cells that express a particular WUP. These Abs possess a WUP-binding arm and an arm that binds a cytotoxic agent or a radionuclide chelator.

6. *Heteroconjugate Abs*

Heteroconjugate Abs, consisting of two covalently joined Abs, have been proposed to target immune system cells to unwanted cells (4,676,980, 1987) and for treatment of human immunodeficiency virus (HIV) infection (WO 91/00360, 1991; WO 92/20373, 1992). Abs prepared *in vitro* using synthetic protein chemistry methods, including those involving cross-linking agents, are contemplated. For example, immunotoxins may be constructed using a disulfide exchange reaction or by forming a thioether bond. Examples of suitable reagents include iminothiolate and methyl-4-mercaptobutyrimidate (4,676,980, 1987).

7. *Immunoconjugates*

Immunoconjugates may comprise an antibody conjugated to a cytotoxic agent such as a chemotherapeutic agent, toxin (*e.g.*, an enzymatically active toxin or fragment of bacterial, fungal, plant, or animal origin), or a radioactive isotope (*i.e.*, a radioconjugate).

Useful enzymatically-active toxins and fragments include Diphtheria A chain, non-binding active fragments of Diphtheria toxin, exotoxin A chain from *Pseudomonas aentginosa,* ricin A chain, abrin A chain, modeccin A chain, a-sarcin, *Aleurites fordii* proteins, Dianthin proteins, *Phytolaca americana* proteins, *Momordica charantia* inhibitor, curcin, crotin, *Sapaonaria officinalis* inhibitor, gelonin, mitogellin, restrictocin, phenomycin, enomycin, and the tricothecenes. A variety of radionuclides are available for the production of radioconjugated Abs, such as $^{212}Bi$, $^{131}I$, $^{131}In$, $^{90}Y$, and $^{186}Re$.

Conjugates of the antibody and cytotoxic agent are made using a variety of bi-functional protein-coupling agents, such as N-succinimidyl-3-(2-pyridyldithiol) propionate (SPDP), iminothiolane (IT), bi-functional derivatives of imidoesters (such as dimethyl adipimidate HCl), active esters (such as disuccinimidyl suberate), aldehydes (such as glutareldehyde), *bis*-azido compounds (such as bis (p-azidobenzoyl) hexanediamine), *bis*-diazonium derivatives (such as bis-(p-diazoniumbenzoyl)-ethylenediamine), diisocyanates (such as tolyene 2,6-diisocyanate), and *bis*-active fluorine compounds (such as 1,5-difluoro-2,4-dinitrobenzene). For example, a ricin immunotoxin can be prepared (Vitetta et al., 1987). $^{14}C$-labeled 1-isothiocyanatobenzyl-3-methyldiethylene triaminepentaacetic acid (MX-DTPA) is an exemplary chelating agent for conjugating radionuclide to antibody (WO 94/11026, 1994).

The antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a streptavidin "ligand" (*e.g.*, biotin) that is conjugated to a cytotoxic agent (*e.g.*, a radionuclide).

8. *Effector function engineering*

The antibody can be modified to enhance its effectiveness in treating a disease, such as cancer. For example, cysteine residue(s) may be introduced into the $F_c$ region, thereby allowing interchain disulfide bond formation in this region. Such homodimeric Abs may have improved internalization capability and/or increased complement-mediated cell killing and antibody-dependent cellular cytotoxicity (ADCC) (Caron et al., 1992; Shopes, 1992). Homodimeric Abs with enhanced anti-tumor activity can be prepared using hetero-bifunctional cross-linkers (Wolff et al., 1993). Alternatively, an antibody engineered with dual $F_c$ regions may have enhanced complement lysis (Stevenson et al., 1989).

9. *Immunoliposomes*

Liposomes containing the antibody may also be formulated (U.S. Patent No. 4485045, 1984; U.S. Patent No. 4544545, 1985; U.S. Patent No. 5013556, 1991; Eppstein et al., 1985; Hwang et al., 1980). Useful liposomes can be generated by a reverse-phase evaporation method with a lipid composition comprising phosphatidylcholine, cholesterol, and PEG-derivatized phosphatidylethanolamine (PEG- PE). Such preparations are extruded through filters of defined pore size to yield liposomes with a desired diameter. $F_{ab'}$ fragments of the antibody can be conjugated to the liposomes (Martin and Papahadjopoulos, 1982) via a disulfide-interchange reaction. A chemotherapeutic agent, such as Doxorubicin, may also be contained in the liposome (Gabizon et al., 1989). Other useful liposomes with different compositions are contemplated.

10. *Diagnostic applications of Abs directed against WUP*

Anti-WUP Abs can be used to localize and/or quantitate WUP (*e.g.*, for use in measuring levels of WUP within tissue samples or for use in diagnostic methods, *etc.).* Anti-WUP epitope Abs can be utilized as pharmacologically active compounds.

Anti-WUP Abs can be used to isolate WUP by standard techniques, such as immunoaffinity chromatography or immunoprecipitation. These approaches facilitate purifying endogenous WUP antigen-containing polypeptides from cells and tissues. These approaches, as well as others, can be used to detect WUP in a sample to evaluate the abundance and pattern of expression of the antigenic protein. Anti-WUP Abs can be used to monitor protein levels in tissues as part of a clinical testing procedure; for example, to determine the efficacy of a given treatment regimen. Coupling the antibody to a detectable substance (label) allows detection of Ab-antigen complexes. Classes of labels include fluorescent, luminescent, bioluminescent, and radioactive materials, enzymes and prosthetic groups. Useful labels include horseradish peroxidase, alkaline phosphatase, β-galactosidase, acetylcholinesterase, streptavidin/biotin, avidin/biotin, umbelliferone, fluorescein, fluorescein isothiocyanate, rhodamine, dichlorotriazinylamine fluorescein, dansyl chloride, phycoerythrin, luminol, luciferase, luciferin, aequorin, and $^{125}I$, $^{131}I$, $^{35}S$ or $^{3}H$.

11. *Antibody therapeutics*

Abs of the invention, including polyclonal, monoclonal, humanized and fully human Abs, can be used therapeutically. Such agents will generally be employed to treat or prevent a disease or pathology in a subject. An antibody preparation, preferably one having high antigen specificity and affinity generally mediates an effect by binding the target epitope(s). Generally, administration of such Abs may mediate one of two effects: (1) the antibody may prevent ligand binding, eliminating endogenous ligand binding and subsequent signal transduction, or (2) the antibody elicits a physiological result by binding an effector site on the target molecule, initiating signal transduction.

A therapeutically effective amount of an antibody relates generally to the amount needed to achieve a therapeutic objective, epitope binding affinity, administration rate, and depletion rate of the antibody from a subject. Common ranges for therapeutically effective doses may be, as a nonlimiting example, from about 0.1 mg/kg body weight to about 50 mg/kg body weight Dosing frequencies may range, for example, from twice daily to once a week.

12. *Pharmaceutical compositions of Abs*

Anti-WUP Abs, as well as other WUP interacting molecules (such as aptamers) identified in other assays, can be administered in pharmaceutical compositions to treat various disorders. Principles and considerations involved in preparing such compositions, as well as guidance in the choice of components can be found in (de Boer, 1994; Gennaro, 2000; Lee, 1990).

Abs that are internalized are preferred when whole Abs are used as inhibitors. Liposomes may also be used as a delivery vehicle for intracellular introduction. Where antibody fragments are used, the smallest inhibitory fragment that specifically binds to the epitope is preferred For example, peptide molecules can be designed that bind a preferred epitope based on the variable-region sequences of a useful antibody. Such peptides can be synthesized chemically and/or produced by recombinant DNA technology (Marasco et al., 1993). Formulations may also contain more than one active compound for a particular treatment, preferably those with activities that do not adversely affect each other. The composition may comprise an agent that enhances function, such as a cytotoxic agent, cytokine, chemotherapeutic agent, or growth-inhibitory agent.

The active ingredients can also be entrapped in microcapsules prepared by coacervation techniques or by interfacial polymerization; for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for *in vivo* administration are highly preferred to be sterile. This is readily accomplished by filtration through sterile filtration membranes or any of a number of techniques.

Sustained-release preparations may also be prepared, such as semi-permeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethylmethacrylate), or poly(vinylalcohol)), polylactides (Boswell and Scribner, U.S. Patent No. 3,773,919, 1973), copolymers of L-glutamic acid and $\gamma$ ethyl-L-glutamate, nondegradable ethylene-vinyl acetate, degradable lactic acid-glycolic acid copolymers such as injectable microspheres composed of lactic acid-glycolic acid copolymer, and poly-D-(-)-3-hydroxybutyric acid. While polymers such as ethylene-vinyl acetate and lactic acid-glycolic acid enable release of molecules for over 100 days, certain hydrogels release proteins for shorter time periods and may be preferred.

*WUP recombinant expression vectors and host cells*

Vectors are tools used to shuttle DNA between host cells or as a means to express a nucleotide sequence. Some vectors function only in prokaryotes, while others function in both prokaryotes and eukaryotes, enabling large-scale DNA preparation from prokaryotes for expression in eukaryotes. Inserting the DNA of interest, such as WUP nucleotide sequence or a fragment, is accomplished by ligation techniques and/or mating protocols well known to the skilled artisan. Such DNA is inserted such that its integration does not disrupt any necessary components of the vector. In the case of vectors that are used to express the inserted DNA protein, the introduced DNA is operably-linked to the vector elements that govern its transcription and translation.

Vectors can be divided into two general classes: Cloning vectors are replicating plasmid or phage with regions that are non-essential for propagation in an appropriate host cell, and into which foreign DNA can be inserted; the foreign DNA is replicated and propagated as if it were a component of the vector. An expression vector (such as a plasmid, yeast, or animal virus genome) is used to introduce foreign genetic material into a host cell or tissue in order to transcribe and translate the foreign DNA. In expression vectors, the introduced DNA is operably-linked to elements, such as promoters, that signal to the host cell to transcribe the inserted DNA. Some promoters are exceptionally useful, such as inducible promoters that control gene transcription in response to specific factors. Operably-linking *WUP* or anti-sense construct to an inducible promoter can control the expression of *WUP* or fragments, or anti-sense constructs. Examples of classic inducible promoters include those that are responsive to $\alpha$-interferon, heat-shock, heavy metal ions, and steroids such as glucocorticoids (Kaufman, 1990) and tetracycline. Other desirable inducible promoters include those that are not endogenous to the cells in which the construct is being introduced, but, however, is responsive in those cells when the induction agent is exogenously supplied.

Vectors have many difference manifestations. A "plasmid" is a circular double stranded DNA molecule into which additional DNA segments can be introduced. Viral vectors can accept additional DNA segments into the viral genome. Certain vectors are capable of autonomous replication in a host cell (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. In general, useful expression vectors are often plasmids. However, other forms of expression vectors, such as viral vectors (*e.g.*, replication defective retroviruses, adenoviruses and adeno-associated viruses) are contemplated

Recombinant expression vectors that comprise *WUP* (or fragments) regulate WUP transcription by exploiting one or more host cell-responsive (or that can be manipulated *in vitro)* regulatory sequences that is operably-linked to *WUP*. "Operably-linked" indicates that a nucleotide sequence of interest is linked to regulatory sequences such that expression of the nucleotide sequence is achieved.

Vectors can be introduced in a variety of organisms and/or cells (Table D). Alternatively, the vectors can be transcribed and translated *in vitro,* for example using T7 promoter regulatory sequences and T7 polymerase.

Table D Examples of hosts for cloning or expression

| Organisms | Examples | Sources and References* |
|---|---|---|
| Prokaryotes | | |
| Enterobacteriaceae | *E. coli*<br>K 12 strain MM294<br>X1776<br>W3110<br>K5 772 | <br>ATCC 31,446<br>ATCC 31,537<br>ATCC 27,325<br>ATCC 53,635 |
| | *Enterobacter* | |
| | *Erwinia* | |
| | *Klebsiella* | |
| | *Proteus* | |
| | *Salmonella (S. tyhpimurium)* | |
| | *Serratia (S. marcescans)* | |
| | *Shigella* | |
| | *Bacilli (B. subtilis* and *B. licheniformis)* | |
| | *Pseudomonas* (P. *aeruginosa)* | |
| | *Streptomyces* | |
| Eukaryotes | | |

(continued)

| Organisms | Examples | Sources and References* |
|---|---|---|
| Yeasts | *Saccharomyces cerevisiae* | |
| | *Schizosaccharomyces pombe* | |
| | *Kluyveromyces*<br>*K. lactis* MW98-8C, CBS683, CBS4574<br>*K. fragilis*<br>*K. bulgaricus*<br>*K. wickeramii*<br>*K. waltii*<br>*K. drosophilarum*<br>*K. thermotolerans*<br>*K. marxianus; yarrowia* | (Fleer et al., 1991)<br>(de Louvencourt et al., 1983) (de Louvencourt et al., 1983)<br>ATCC 12,424<br>ATCC 16,045<br>ATCC 24,178<br>ATCC 56,500<br>ATCC 36,906<br><br>(EPO 402226, 1990) |
| | *Pichia pastoris* | (Sreekrishna et al., 1988) |
| | *Candida* | |
| | *Trichoderma reesia* | |
| | *Neurospora crassa* | (Case et al., 1979) |
| | *Torulopsis* | |
| | *Rhodotorula* | |
| | *Schwanniomyces* (*S. occidentalis*) | |
| Filamentous Fungi | *Neurospora* | |
| | *Penicillium* | |
| | *Tolypocladium Aspergillus (A. nidulans* and *A. niger)* | (WO 91/00357, 1991) (Kelly and Hynes, 1985; Tilbum et al., 1983; Yelton et Tilbum et al., 1983; Yelton et al., 1984) |
| Invertebrate cells | *Drosophila S2* | |
| | *Spodoprera* Sf9 | |
| Vertebrate cells | Chinese Hamster Ovary (CHO) | |
| | simian COS COS-7 | ATCC CRL 1651 |
| | HEK 293 | |
| *Unreferenced cells are generally available from American Type Culture Collection (Manassas, VA). | | |

Vector choice is dictated by the organism or cells being used and the desired fate of the vector. Vectors may replicate once in the target cells, or may be "suicide" vectors. In general, vectors comprise signal sequences, origins of replication, marker genes, enhancer elements, promoters, and transcription termination sequences. The choice of these elements depends on the organisms in which the vector will be used and are easily determined Some of these elements may be conditional, such as an inducible or conditional promoter that is turned "on" when conditions are appropriate. Examples of inducible promoters include those that are tissue-specific, which relegate expression to certain cell types, steroid-responsive, or heat-shock reactive. Some bacterial repression systems, such as the lac operon, have been exploited in mammalian cells and transgenic animals (Fieck et al., 1992; Wyborski et al., 1996; Wyborski and Short, 1991). Vectors often use a selectable marker to facilitate identifying those cells that have incorporated the vector. Many selectable markers are well known in the art for the use with prokaryotes, usually antibiotic-resistance genes or the use of autotrophy and auxotrophy mutants.

Using antisense and sense WUP oligonucleotides can prevent WUP polypeptide expression. These oligonucleotides bind to target nucleic acid sequences, forming duplexes that block transcription or translation of the target sequence by enhancing degradation of the duplexes, terminating prematurely transcription or translation, or by other means.

Antisense or sense oligonucleotides are singe-stranded nucleic acids, either RNA or DNA, which can bind target WUP mRNA (sense) or WUP DNA (antisense) sequences. Antisense or sense oligonucleotides may comprise a fragment of the WUP DNA coding region of at least about 14 nucleotides, preferably from about 14 to 30 nucleotides. In general, antisense RNA or DNA molecules can comprise at least 5,10,15, 20, 25, 30, 35, 40, 45, 50, 55, 60, 65, 70, 75, 80, 85, 90, 95, 100 bases in length or more. Among others, (Stein and Cohen, 1988; van der Krol et al., 1988b) describe methods to derive antisense or a sense oligonucleotides from a given cDNA sequence.

Modifications of antisense and sense oligonucleotides can augment their effectiveness. Modified sugar-phosphodiester bonds or other sugar linkages (WO 91/06629, 1991), increase in *vivo* stability by conferring resistance to endogenous nucleases without disrupting binding specificity to target sequences. Other modifications can increase the affinities of the oligonucleotides for their targets, such as covalently linked organic moieties (WO 90/10448, 1990) or poly-(L)-lysine. Other attachments modify binding specificities of the oligonucleotides for their targets, including metal complexes or intercalating (*e.g.* ellipticine) and alkylating agents.

To introduce antisense or sense oligonucleotides into target cells (cells containing the target nucleic acid sequence), any gene transfer method may be used and are well known to those of skill in the art. Examples of gene transfer methods include 1) biological, such as gene transfer vectors like Epstein-Barr virus or conjugating the exogenous DNA to a ligand-binding molecule (WO 91/04753, 1991), 2) physical, such as electroporation, and 3) chemical, such as $CaPO_4$ precipitation and oligonucleotide-lipid complexes (WO 90/10448, 1990).

The terms "host cell" and "recombinant host cell" are used interchangeably. Such terms refer not only to a particular subject cell but also to the progeny or potential progeny of such a cell. Because certain modifications may occur in succeeding generations due to either mutation or environmental influences, such progeny may not, in fact, be identical to the parent cell, but are still included within the scope of the term.

Methods of eukaryotic cell transfection and prokaryotic cell transformation are well known in the art. The choice of host cell will dictate the preferred technique for introducing the nucleic acid of interest. Table E, which is not meant to be limiting, summarizes many of the known techniques in the art. Introduction of nucleic acids into an organism may also be done with *ex vivo* techniques that use an *in vitro* method of transfection, as well as established genetic techniques, if any, for that particular organism.

Table E Methods to introduce nucleic acid into cells

| Cells | Methods | References | Notes |
|---|---|---|---|
| Prokaryotes (bacteria) | Calcium chloride | (Cohen et al., 1972; Hanahan, 1983; Mandel and Higa, 1970) | |
| | Electroporation | (Shigekawa and Dower, 1988) | |
| Eukaryotes<br>Mammalian cells | Calcium phosphate transfection | *N*-(2-Hydroxyethyl) piperazine-*N'*-(2-ethanesulfonic acid (HEPES) buffered saline solution (Chen and Okayama, 1988; Graham and van der Eb, 1973; Wigler et al., 1978)<br>BES (*N,N*-bis(2-hydroxyethyl)-2-aminoethanesulfonic acid) buffered solution (Ishiura et al., 1982) | Cells may be "shocked" with glycerol or dimethylsulfoxide (DMSO) to increase transfection efficiency (Ausubel et al., 1987). |

(continued)

| Cells | Methods | References | Notes |
|---|---|---|---|
| | Diethylaminoethyl (DEAE)-Dextran transfection | (Fujita et al., 1986; Lopata et al., 1984; Selden et al., 1986) | Most useful for transient, but not stable, transfections. Chloroquine can be used to increase efficiency. |
| | Electroporation | (Neumann et al., 1982; Potter, 1988; Potter et al., 1984; Wong and Neumann, 1982) | Especially useful for hard-to-transfect lymphocytes. |
| | Cationic lipid reagent transfection | (Elroy-Stein and Moss, 1990; Felgner et al., 1987; Rose et al, 1991; Whitt et Rose al., 1991; Whitt et al., 1990) | Applicable to both *in vivo* and *in vitro* transfection. |
| | Retroviral | Production exemplified by (Cepko et al., 1984; Miller and Buttimore, 1986; Pear et al., 1993)<br>Infection *in vitro* and *in vivo*: (Austin and Cepko, 1990; Bodine et al., 1991; Fekete and Cepko, 1993; Lemischka et al., 1986; Turner et al., 1990; Williams et al., 1984) | Lengthy process, many packaging lines available at ATCC. Applicable to both *in vivo* and *in vitro* transfection. |
| | Polybrene | (Chaney et al., 1986; Kawai and Nishizawa, 1984) | |
| | Microinjection | (Capecchi, 1980) | Can be used to establish cell lines carrying integrated copies of WUP DNA sequences. |
| | Protoplast fusion | (Rassoulzadegan et al., 1982; Sandri-Goldin et al., 1981; Schaffner, 1980) | |
| Insect cells *(in vitro)* | Baculovirus systems | (Luckow, 1991; Miller, 1988; O'Reilly et al., 1992) | Useful for *in vitro* production of Production of eukaryoticeukaryotic modifications. |
| Yeast | Electroporation | (Becker and Guarente, 1991) | |
| | Lithium acetate Lithium acetate | (Gietz et al., 1998; Ito et al., 1983) | |
| | Spheroplast fusion | (Beggs, 1978; Hinnen et al., 1978) | Laborious, can produce aneuploids |

(continued)

| Cells | Methods | References | Notes |
|---|---|---|---|
| Plant cells (general reference: (Hansen and Wright, 1999)) | Agrobacterium transformation | (Bechtold and Pelletier, 1998; Escudero and Hohn, 1997; Hansen and Chilton, 1999; Touraev and al., 1997) | |
| | Biolistics (microprojectiles) | (Finer et al., 1999; Hansen and Chilton, 1999; Shillito, 1999) | |
| | Electroporation (protoplasts) | (Fromm et al., 1985; OuLee et al., 1986; Rhodes et al., 1988; Saunders et al., 1989) May be combined with liposomes (Trick and al., 1997) | |
| | Polyethylene glycol (PEG) treatment | (Shillito, 1999) | |
| | Liposomes | May be combined with electroporation (Trick and al., 1997) | |
| | *in planta* microinjection | (Leduc and al., 1996; Zhou and al., 1983) | |
| | Seed imbibition | (Trick and al., 1997) | |
| | Laser beam | (Hoffman, 1996) | |
| | Silicon carbide whiskers | (Thompson and al., 1995) | |

Vectors often use a selectable marker to facilitate identifying those cells that have incorporated the vector. Many selectable markers are well known in the art for the use with prokaryotes, usually antibiotic-resistance genes or the use of autotrophy and auxotrophy mutants. Table F lists often-used selectable markers for mammalian cell transfection.

Table F Useful selectable markers for eukaryote cell transfection

| Selectable Marker | Selection | Action | Reference |
|---|---|---|---|
| Adenosine deaminase (ADA) | Media includes 9-β-D-xylofuranosyl adenine (Xyl-A) | Conversion of Xyl-A to Xyl-ATP, which incorporates into nucleic acids, killing cells. ADA detoxifies | (Kaufman et al., 1986) al., 1986) |
| Dihydrofolate reductase (DHFR) | Methotrexate (MTX) and dialyzed serum (purine-free media) | MTX competitive inhibitor of DHFR In absence of exogenous purines, cells require DHFR, a necessary enzyme in purine biosynthesis. | (Simonsen and Levinson, 1983) |
| Aminoglycoside phosphotransferase ("APH", "neo", "G418") | G418 | G418, an aminoglycoside detoxified by APH, detoxified by APH, interferes with ribosomal function and consequently, translation. | (Southern and Berg, 1982) |

(continued)

| Selectable Marker | Selection | Action | Reference |
|---|---|---|---|
| Hygromycin-B-phosphotransferase (HPH) | hygromycin-B | Hygromycin-B, an aminocyclitol detoxified by HPH, disrupts protein translocation and promotes mistranslation. | (palmer et al., 1987) al., 1987) |
| Thymidine kinase Reverse selection (TK) | Forward selection (TK+): Media (HAT) incorporates aminopterin<br><br>(TK-): Media incorporates 5-bromodeoxyuridine (BrdU). | Forward: Aminopterin forces cells to synthesze dTTP from thymidine, a pathway requiring a pathway requiring TK.<br>Reverse: TK phosphorylates BrdU, which incorporates into nucleic acids, killing cells. | (Littlefield, 1964) |

A host cell of the invention, such as a prokaryotic or eukaryotic host cell in culture can be used to produce WUP. Accordingly, the invention provides methods for producing WUP using the host cells of the invention. In one embodiment, the method comprises culturing the host cell of the invention (into which a recombinant expression vector encoding WUP has been introduced) in a suitable medium, such that WUP is produced. In another embodiment, the method further comprises isolating WUP from the medium or the host cell.

*Transgenic WUP animals*

Transgenic animals are useful for studying the function and/or activity of WUP and for identifying and/or evaluating modulators of *WUP* activity. "Transgenic animals" are non-human animals, preferably mammals, more preferably a rodents such as rats or mice, in which one or more of the cells include a transgene. Other transgenic animals include primates, sheep, dogs, cows, goats, chickens, amphibians, *etc*. A "transgene" is exogenous DNA that is integrated into the genome of a cell from which a transgenic animal develops, and that remains in the genome of the mature animal. Transgenes preferably direct the expression of an encoded gene product in one or more cell types or tissues of the transgenic animal with the purpose of preventing expression of a naturally encoded gene product in one or more cell types or tissues (a "knockout" transgenic animal), or serving as a marker or indicator of an integration, chromosomal location, or region of recombination (*e.g. cre*/*loxP* mice). A "homologous recombinant animal" is a non-human animal, such as a rodent, in which endogenous *WUP* has been altered by an exogenous DNA molecule that recombines homologously with endogenous *WUP* in a (*e.g.* embryonic) cell prior to development the animaL Host cells with exogenous *WUP* can be used to produce non-human transgenic animals, such as fertilized oocytes or embryonic stem cells into which *WUP*-coding sequences have been introduced. Such host cells can then be used to create non-human transgenic animals or homologous recombinant animals.

1. *Approaches to transgenic animal production*

A transgenic animal can be created by introducing *WUP* into the male pronuclei of a fertilized oocyte (*e.g.*, by microinjection, retroviral infection) and allowing the oocyte to develop in a pseudopregnant female foster animal (pffa). The *WUP* cDNA sequences (SEQ ID NO: 5) can be introduced as a transgene into the genome of a non-human animal Alternatively, a homologue of *WUP*, such as the naturally-occuring variant of *WUP* (SEQ ID NO:7), can be used as a transgene. Intronic sequences and polyadenylation signals can also be included in the transgene to increase transgene expression. Tissue-specific regulatory sequences can be operably-linked to the *WUP* transgene to direct expression of *WUP* to particular cells. Methods for generating transgenic animals via embryo manipulation and microinjection, particularly animals such as mice, have become conventional in the art, *e.g.* (Evans et al., U.S. Patent No. 4,870,009,1989; Hogan, 0879693843, 1994; Leder and Stewart, U.S. Patent No. 4,736,866, 1988; Wagner and Hoppe, US Patent No. 4,873,191, 1989). Other non-mice transgenic animals may be made by similar methods. A transgenic founder animal, which can be used to breed additional transgenic animal, can be identified based upon the presence of the transgene in its genome and/or expression of the transgene mRNA in tissues or cells of the animals, Transgenic (*e.g. WUP*) animals, can be bred to other transgenic animals carrying other transgenes.

2. *Vectors for trangenic animal production*

To create a homologous recombinant animal, a vector containing at least a portion of WUP into which a deletion addition or substitution has been introduced to thereby alter, *e.g.*, functionally disrupt, *WUP.* In one approach, a knockout vector functionally disrupts the endogenous *WUP* gene upon homologous recombination, and thus a non-functional WUP protein, if any, is expressed.

Alternatively, the vector can be designed such that, upon homologous recombination, the endogenous *WUP* is mutated or otherwise altered but still encodes function protein (*e.g.*, the upstream regulatory region can be altered to thereby alter the expression of endogenous WUP). In this type of homologous recombination vector, the altered portion of the *WUP* is flanked at its 5'- and 3'-termini by additional nucleic acid of the *WUP* to allow for homologous recombination to occur between the exogenous *WUP* carried by the vector and an endogenous *WUP* in an embryonic stem cell. The additional flanking *WUP* nucleic acid is sufficient to engender homologous recombination with endogenous WKP. Typically, several kilobases of flanking DNA (both at the 5'- and 3'-termini) are included in the vector (Thomas and Capecchi, 1987). The vector is then introduced into an embryonic stem cell line (e.g., by electroporation), and cells in which the introduced WUP has homologously-recombihed with the endogenous WUP are selected (Li et al., 1992).

3. *Introduction of WUP transgene cells during development*

Selected cells are then injected into a blastocyst of an animal *(e.g.,* a mouse) to form aggregation chimeras (Bradley, 1987). A chimeric embryo can then be implanted into a suitable pffa and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described (Berns et al., WO 93/04169,1993; Bradley,1991; Kucherlapati et al., WO 91/01140,1991; Le Mouellic and Brullet, WO 90/11354, 1990).

Alternatively, transgenic animals that contain selected systems that allow for regulated expression of the transgene can be produced. An example of such a system is the *cre*/*loxP* recombinase system of bacteriophage P1 (Lakso et al., 1992). Another recombinase system is the FLP recombinase system of *Saccharomyces cerevisiae* (O'Gorman et al., 1991). If a *cre*/*loxP* recombinase system is used to regulate expression of the transgene, animals containing transgenes encoding both the Cre recombinase and a selected protein are required. Such animals can be produced as "double" transgenic animals, by mating an animal containing a transgene encoding a selected protein to another containing a transgene encoding a recombinase.

Clones of transgenic animals can also be produced (Wilmut et al., 1997). In brief, a cell from a transgenic animal can be isolated and induced to exit the growth cycle and enter $G_0$ phase. The quiescent cell can then be fused to an enucleated oocyte from an animal of the same species from which the quiescent cell is isolated The reconstructed oocyte is then cultured to develop to a morula or blastocyte and then transferred to a pffa. The offspring borne of this female foster animal will be a clone of the "parent" transgenic animal.

*Pharmaceutical compositions*

The *WUP* nucleic acid molecules, WUP polypeptides, and anti-WUP Abs (active compounds) of the invention, and derivatives, analogs and homologs thereof, can be incorporated into pharmaceutical compositions. Such compositions typically comprise the nucleic acid molecule, protein, or antibody and a pharmaceutically acceptable carrier. A pharmaceutically acceptable carrier" includes any and all solvents, dispersion media, coatings, antibacterial and antifungal agents, isotonic and absorption delaying agents, and the like, compatible with pharmaceutical administration (Gennaro, 2000). Preferred examples of such carriers or diluents include, but are not limited to, water, saline, finger's solutions, dextrose solution, and 5% human serum albumin. Liposomes and non-aqueous vehicles such as fixed oils may also be used Except when a conventional media or agent is incompatible with an active compound, use of these compositions is contemplated. Supplementary active compounds can also be incorporated into the compositions.

1. *General considerations*

A pharmaceutical composition is formulated to be compatible with its intended route of administration, including intravenous, intradermal, subcutaneous, oral (*e.g.*, inhalation), transdermal (*i.e.*, topical), transmucosal, and rectal administration. Solutions or suspensions used for parenteral, intradermal, or subcutaneous application can include: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

2. *Injectable formulations*

Pharmaceutical compositions suitable for injection include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL™ (BASF, Parsippany, NJ.) or phosphate buffered saline (PBS). In all cases, the composition must be sterile and should be fluid, for administration using a syringe. Such compositions should be stable during manufacture and storage and must be preserved against contamination from microorganisms such as bacteria and fungi. The carrier can be a solvent or dispersion medium containing, for example, water, ethanol, polyol (such as glycerol, propylene glycol, and liquid polyethylene glycol), and suitable mixtures. Proper fluidity can be maintained, for example, by using a coating such as lecithin, by maintaining the required particle size in the case of dispersion and by using surfactants. Various antibacterial and antifungal agents, for example, parabens, chlorobutanol, phenol, ascorbic acid, and thimerosal, can contain microorganism contamination. Isotonic agents, for example, sugars, polyalcohols such as manitol, sorbitol, and sodium chloride can be included in the composition. Compositions that can delay absorption include agents such as aluminum monostearate and gelatin.

Sterile injectable solutions can be prepared by incorporating the active compound *(e.g.,* a WUP or anti-WUP antibody) in the required amount in an appropriate solvent with one or a combination of ingredients as required, followed by sterilization. Generally, dispersions are prepared by incorporating the active compound into a sterile vehicle that contains a basic dispersion medium, and the other required ingredients as discussed Sterile powders for the preparation of sterile injectable solutions, methods of preparation include vacuum drying and freeze-drying that yield a powder containing the active ingredient and any desired ingredient from a sterile solutions.

3. *Oral compositions*

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally. Pharmaceutically compatible binding agents, and/or adjuvant materials can be included. Tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, PRIMOGEL, or corn starch; a lubricant such as magnesium stearate or STEROTES; a glidant such as colloidal silicon dioxide; a sweetening agent such as sucrose or saccharin; or a flavoring agent such as peppermint, methyl salicylate, or orange flavoring.

4. *Compositions for inhalation*

For administration by inhalation, the compounds are delivered as an aerosol spray from a nebulizer or a pressurized container that contains a suitable propellant, *e.g.*, a gas such as carbon dioxide.

5. *Systemic administration*

Systemic administration can also be transmucosal or transdermal. For transmucosal or transdermal administration, penetrants that can permeate the target barrier(s) are selected Transmucosal penetrants include, detergents, bile salts, and fusidic acid derivatives. Nasal sprays or suppositories can be used for transmucosal administration. For transdermal administration, the active compounds are formulated into ointments, salves, gels, or creams.

The compounds can also be prepared in the form of suppositories (*e.g.*, with bases such as cocoa butter and other glycerides) or retention enemas for rectal delivery.

6. *Carriers*

The active compounds may be prepared with carriers that protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid Such materials can be obtained commercially from ALZA Corporation (Mountain View, CA) and NOVA Pharmaceuticals, Inc. (Lake Elsinore, CA), or prepared by one of skill in the art. Liposomal suspensions can also be used as pharmaceutically acceptable carriers. These can be prepared according to methods known to those skilled in the art, such as in (Eppstein et al., US Patent No. 4,522,811, 1985).

7. *Unit dosage*

Oral formulations or parenteral compositions in unit dosage form can be created to facilitate administration and dosage uniformity. Unit dosage form refers to physically discrete units suited as single dosages for the subject to be treated, containing a therapeutically effective quantity of active compound in association with the required pharmaceutical carrier. The specification for the unit dosage forms of the invention are dictated by, and directly dependent on, the unique characteristics of the active compound and the particular desired therapeutic effect, and the inherent limitations of compounding the active compound

8. *Gene therapy compositions*

Nucleic acid molecules can be inserted into vectors for use as gene therapy vectors. Gene therapy vectors can be suitable for delivery to a subject by, for example, intravenous injection, local administration (Nabel and Nabel, US Patent No. 5,328,470, 1994), or by stereotactic injection (Chen et al., 1994). The pharmaceutical preparation of a gene therapy vector can include an acceptable diluent, or can comprise a slow release matrix in which the gene delivery vehicle is imbedded Alternatively, where the complete gene delivery vector can be produced intact from recombinant cells, *e.g.*, retroviral vectors, the pharmaceutical preparation can include one or more cells that produce the gene delivery system.

9. *Dosage*

Pharmaceutical compositions and methods may further comprise other therapeutically active compounds as noted herein which are usually applied in the treatment of the above mentioned pathological conditions.

In the use for treatment or prevention of conditions which require WUP modulation an appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0. 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be suitable for administration on a regimen of 1 to 4 times per day, preferably once or twice per day.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

10. *Kits for pharmaceutical compositions*

The pharmaceutical compositions can be included in a kit, container, pack, or dispenser together with instructions for administration. When supplied as a kit, the different components of the composition may be packaged in separate containers and admixed immediately before use. Such packaging of the components separately may permit long-term storage without losing the active components' functions.

Kits may also include reagents in separate containers that facilitate the execution of a specific test, such as diagnostic tests or tissue typing. For example, *WUP* DNA templates and suitable primers may be supplied for internal controls.

(a) *Containers or vessels*

The reagents included in the kits can be supplied in containers of any sort such that the life of the different components are preserved, and are not adsorbed or altered by the materials of the container. For example, sealed glass ampules may contain lyophilized luciferase or buffer that have been packaged under a neutral, non-reacting gas, such as nitrogen. Ampoules may consist of any suitable material, such as glass, organic polymers, such as polycarbonate, polystyrene, etc., ceramic, metal or any other material typically employed to hold reagents. Other examples of suitable containers include simple bottles that may be fabricated from similar substances as ampules, and envelopes, that may consist of foil-lined interiors, such as aluminum or an alloy. Other containers include test tubes, vials, flasks, bottles, syringes, or the like. Containers may have a sterile access port, such as a bottle having a stopper that can be pierced by a hypodermic injection needle. Other containers may have two compartments that are separated by a readily removable membrane that upon removal permits the components to mix. Removable membranes may be glass, plastic, rubber, etc.

(b) *Instructional materials*

Kits may also be supplied with instructional materials. Instructions may be printed on paper or other substrate, and/or may be supplied as an electronic-readable medium, such as a floppy disc, CD-ROM, DVD-ROM, Zip disc, videotape, audio tape, *etc.* Detailed instructions may not be physically associated with the kit; instead, a user may be directed to an internet web site specified by the manufacturer or distributor of the kit, or supplied as electronic mail.

*Screening and detection methods*

Isolated nucleic acid molecules can be used to express WUP (*e.g.*, via a recombinant expression vector in a host cell in gene therapy applications), to detect WUP mRNA (*e.g.,* in a biological sample) or a genetic lesion in a WUP, and to modulate WUP activity, as described below. In addition, WUP polypeptides can be used to screen drugs or compounds that modulate the WUP activity or expression as well as for the manufacture of a medicament to treat disorders characterized by insufficient or excessive production of WUP or production of WUP forms that have decreased or aberrant activity compared to WUP wild-type protein, or modulate biological function that involve WUP. In addition, anti-WUP Abs can be used to detect and isolate WUP and modulate WUP activity.

1. *Screening assays*

Screening assay may identify modalities, *i.e.,* candidate or test compounds or agents (*e.g.,* peptides, peptidomimetics, small molecules or other drugs), foods, combinations thereof, *etc.,* that effect WUP, a stimulatory or inhibitory effect, inlcuding translation, transcription, activity or copies of the gene in cells. Testing for compounds that increase or decrease WUP activity are desirable. A compound may modulate WUP activity by affecting: (1) the number of copies of the gene in the cell (amplifiers and deamplifiers); (2) increasing or decreasing transcription of the *WUP* (transcription up-regulators and down-regulators); (3) by increasing or decreasing the translation of *WUP* mRNA into protein (translation up-regulators and down-regulators); or (4) by increasing or decreasing the activity of WUP itself (agonists and antagonists).

(a) *effects of compounds*

To identify compounds that affect WUP at the DNA, RNA and protein levels, cells or organisms are contacted\ with a candidate compound and the corresponding change in WUP DNA, RNA or protein is assessed (Ausubel et al., 1987). For DNA amplifiers and deamplifiers, the amount of *WUP* DNA is measured, for those compounds that are transcription up-regulators and down-regulators the amount of *WUP* mRNA is determined; for translational up- and down-regulators, the amount of WUP polypeptides is measured. Compounds that are agonists or antagonists may be identified by contacting cells or organisms with the compound.

In one embodiment, many assays for screening candidate or test compounds that bind to or modulate the activity of WUP or polypeptide or biologically active portion are available. Test compounds can be obtained using any of the numerous approaches in combinatorial library methods, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptides, while the other four approaches encompass peptide, non-peptide oligomer or small molecule libraries of compounds (Lam, 1997).

(b) *small molecules*

A "small molecule" refers to a composition that has a molecular weight of less than about 5 kD and more preferably less than about 4 kD, and most preferable less than 0.6 kD. Small molecules can be, nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention. Examples of methods for the synthesis of molecular libraries can be found in: (Carell et al., 1994a; Carell et al., 1994b; Cho et al., 1993; DeWitt et al., 1993; Gallop et al., 1994; Zuckermann et al., 1994).

Libraries of compounds may be presented in solution (Houghten et al., 1992) or on beads (Lam et al., 1991), on chips (Fodor et al., 1993), bacteria, spores (Ladner et al., US Patent No. 5,223,409, 1993), plasmids (Cull et al., 1992) or on phage (Cwirla et al., 1990; Devlin et al., 1990; Felici et al., 1991; Ladner et al., US Patent No. 5,223,409, 1993; Scott and Smith, 1990). A cell-free assay comprises contacting WUP or biologically-active fragment with a known compound that binds WUP to form an assay mixture, contacting the assay mixture with a test compound, and determining the ability of the test compound to interact with WUP, where determining the ability of the test compound to interact with WUP comprises determining the ability of the WUP to preferentially bind to or modulate the activity of a WUP target molecule.

(c) *cell-free assays*

The cell-free assays of the invention may be used with both soluble or a membrane-bound forms of WUP. In the case of cell-free assays comprising the membrane-bound form, a solubilizing agent to maintain WUP in solution. Examples of such solubilizing agents include non-ionic detergents such as n-octylglucoside, n-dodecylglucoside, n-dodecylmaltoside, octanoyl-N-methylglucamide, decanoyl-N-methylglucamide, TRTTON® X-100 and others from the TRITON® series, THESIT®, Isotridecypoly(ethylene glycol ether)$_n$, N-dodecyl-N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

(d) *immobilization of target molecules to facilitate screening*

In more than one embodiment of the assay methods, immobilizing either WUP or its partner molecules can facilitate separation of complexed from uncomplexed forms of one or both of the proteins, as well as to accommodate high throughput assays. Binding of a test compound to WUP, or interaction of WUP with a target molecule in the presence and absence of a candidate compound, can be accomplished in any vessel suitable for containing the reactants, such as microtiter plates, test tubes, and micro-centrifuge tubes. A fusion protein can be provided that adds a domain that allows one or both of the proteins to be bound to a matrix. For example, GST-WUP fusion proteins or GST-target fusion proteins can be adsorbed onto glutathione sepharose beads (SIGMA Chemical, St. Louis, MO) or glutathione derivatized microtiter plates that are then combined with the test compound or the test compound and either the non-adsorbed target protein or WUP, and the mixture is incubated under conditions conducive to complex formation (*e.g.*, at physiological conditions for salt and pH). Following incubation, the beads or microtiter plate wells are washed to remove any unbound components, the matrix immobilized in the case of beads, complex determined either directly or indirectly, for example, as described Alternatively, the complexes can be dissociated from the matrix, and the level of WUP binding or activity determined using standard techniques.

Other techniques for immobilizing proteins on matrices can also be used in screening assays. Either WUP or its target molecule can be immobilized using biotin-avidin or biotin-streptavidin systems. Biotinylation can be accomplished using many reagents, such as biotin-NHS (N-hydroxy-succinimide; PIERCE Chemicals, Rockford, IL), and immobilized in wells of streptavidin-coated 96 well plates (PIERCE Chemical). Alternatively, Abs reactive with WUP or target molecules, but which do not interfere with binding of the WUP to its target molecule, can be derivatized to the wells of the plate, and unbound target or WUP trapped in the wells by antibody conjugation. Methods for detecting such complexes, in addition to those described for the GST-immobilized complexes, include immunodetection of complexes using Abs reactive with WUP or its target, as well as enzyme-linked assays that rely on detecting an enzymatic activity associated with the WUP or target molecule.

(e) *screens to identify modulators*

Modulators of WUP expression can be identified in a method where a cell is contacted with a candidate compound and the expression of WUP mRNA or protein in the cell is determined. The expression level of *WUP* mRNA or protein in the presence of the candidate compound is compared to WUP mRNA or protein levels in the absence of the candidate compound. The candidate compound can then be identified as a modulator of WUP mRNA or protein expression based upon this comparison. For example, when expression of WUP mRNA or protein is greater (*i.e.*, statistically significant) in the presence of the candidate compound than in its absence, the candidate compound is identified as a stimulator of WUP mRNA or protein expression. Alternatively, when expression of WUP mRNA or protein is less (statistically significant) in the presence of the candidate compound than in its absence, the candidate compound is identified as an inhibitor of WUP mRNA or protein expression. The level of WUP mRNA or protein expression in the cells can be determined by methods described for detecting WUP mRNA or protein.

(i) *hybrid assays*

WUP can be used as "bait" in two-hybrid or three hybrid assays (Bartel et al., 1993; Brent et al., WO94/10300, 1994; Iwabuchi et al., 1993; Madura et al., 1993; Saifer et al., US Patent No. 5,283,317, 1994; Zervos et al., 1993) to identify other proteins that bind or interact with WUP and modulate WUP activity. Such WUP-bps are also likely to be involved in the propagation of signals by the WUP as, for example, upstream or downstream elements of a WUP pathway.

The two-hybrid system is based on the modular nature of most transcription factors, which consist of separable DNA-binding and activation domains. Briefly, the assay utilizes two different DNA constructs. In one construct, the gene that codes for WUP is fused to a gene encoding the DNA binding domain of a known transcription factor *(e.g.,* GAL4). The other construct, a DNA sequence from a library of DNA sequences that encodes an unidentified protein ("prey" or "sample") is fused to a gene that codes for the activation domain of the known transcription factor. If the "bait" and the "prey" proteins are able to interact *in vivo,* forming a WUP-dependent complex, the DNA-binding and activation domains of the transcription factor are brought into close proximity. This proximity allows transcription of a reporter gene (*e.g.*, LacZ) that is operably-linked to a transcriptional regulatory site responsive to the transcription factor. Expression of the reporter gene can be detected, and cell colonies containing the functional transcription factor can be isolated and used to obtain the cloned gene that encodes the WUP-interacting protein.

Novel agents may be identified by the aforementioned screening assays and used for treatments as described herein.

2. *Detection assays*

Portions or fragments of *WUP* cDNA sequences identified herein (and the complete *WUP* gene sequences) are useful in themselves. By way of non-limiting example, these sequences can be used to: (1) identify an individual from a minute biological sample (tissue typing); and (2) aid in forensic identification of a biological sample.

(a) *Tissue typing*

The WUP sequences of the invention can be used to identify individuals from minute biological samples. In this technique, an individual's genomic DNA is digested with one or more restriction enzymes and probed on a Southern blot to yield unique bands. The sequences of the invention are useful as additional DNA markers for "restriction fragment length polymorphisms" (RFLP; (Smulson et al., US Patent No. 5,272,057,1993)).

Furthermore, the *WUP* sequences can be used to determine the actual base-by-base DNA sequence of targeted portions of an individual's genome. WUP sequences can be used to prepare two PCR primers from the 5'- and 3'-termini of the sequences that can then be used to amplify an the corresponding sequences from an individual's genome and then sequence the amplified fragment.

Panels of corresponding DNA sequences from individuals can provide unique, individual identifications, as each individual will have a unique set of such DMA sequences due to allelic differences. The sequences of the invention can be used to obtain such identification sequences from individuals and from tissue. The WUP sequences of the invention uniquely represent portions of an individual's genome. Allelic variation occurs to some degree in the coding regions of these sequences, and to a greater degree in the noncoding regions. The allelic variation between individual humans occurs with a frequency of about once ever 500 bases. Much of the allelic variation is due to single nucleotide polymorphisms (SNPs), which include RFLPs.

Each of the sequences described herein can, to some degree, be used as a standard against which DNA from an individual can be compared for identification purposes. Because greater numbers of polymorphisms occur in noncoding regions, fewer sequences are necessary to differentiate individuals. Noncoding sequences can positively identify individuals with a panel of 10 to 1,000 primers that each yield a noncoding amplified sequence of 100 bases. If predicted coding sequences, such as those in SEQ ID NOS: 5 or 7 are used, a more appropriate number of primers for positive individual identification would be 500-2,000.

*Predictive medicine*

The invention also pertains to the field of predictive medicine in which diagnostic assays, prognostic assays, pharmacogenomics, and monitoring clinical trials are used for-prognostic (predictive) purposes to treat an individual prophylactically. Accordingly, one aspect of the invention relates to diagnostic assays for determining WUP and/or nucleic acid expression as well as WUP activity, in the context of a biological sample (*e.g*., blood, serum, cells, tissue) to determine whether an individual is afflicted with a disease or disorder, or is at risk of developing a disorder, associated with aberrant WUP expression or activity, including cancer. The invention also provides for prognostic (or predictive) assays for determining whether an individual is at risk of developing a disorder associated with WUP, nucleic acid expression or activity. For example, mutations in *WUP* can be assayed in a biological sample. Such assays can be used for prognostic or predictive purpose to prophylactically treat an individual prior to the onset of a disorder characterized by or associated with WUP, nucleic acid expression, or biological activity.

Another aspect of the invention provides methods for determining WUP activity, or nucleic acid expression, in an individual to select appropriate therapeutic or prophylactic agents for that individual (referred to herein as "pharmacogenomics"). Pharmacogenomics allows for the selection of modalities (*e.g.*, drugs, foods) for therapeutic or prophylactic treatment of an individual based on the individual's genotype (*e.g.*, the individual's genotype to determine the individual's ability to respond to a particular agent). Another aspect of the invention pertains to monitoring the influence of modalities (*e.g.*, drugs, foods) on the expression or activity of WUP in clinical trials.

1. *Diagnostic assays*

An exemplary method for detecting the presence or absence of WUP in a biological sample involves obtaining a biological sample from a subject and contacting the biological sample with a compound or an agent capable of detecting WUP or *WUP* nucleic acid (*e.g.*, mRNA, genomic DNA) such that the presence of WUP is confirmed in the sample. An agent for detecting *WUP* mRNA or genomic DNA is a labeled nucleic acid probe that can hybridize to *WUP* mRNA or genomic DNA. The nucleic acid probe can be, for example, a full-length *WUP* nucleic acid, such as the nucleic acid of SEQ ID NOS: 5 or 7, or a portion thereof, such as an oligonucleotide of at least 15, 30, 50, 100, 250 or 500 nucleotides in length and sufficient to specifically hybridize under stringent conditions to *WUP* mRNA or genomic DNA.

An agent for detecting WUP polypeptide is an antibody capable of binding to WUP as defined in the claims, preferably an antibody with a detectable label. Abs can be polyclonal, or more preferably, monoclonal An intact antibody, or a fragment (*e.g.*, $F_{ab}$ or $F(ab)_2$) can be used. A labeled probe or antibody is coupled (*i.e.*, physically linking) to a detectable substance, as well as indirect detection of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently-labeled streptavidin. The term "biological sample" includes tissues, cells and biological fluids isolated from a subject, as well as tissues, cells and fluids present within a subject The detection method can be used to detect. *WUP* mRNA, protein, or genomic DNA in a biological sample *in vitro* as well as *in vivo.* For example, *in vitro* techniques for detection of *WUP* mRNA include Northern hybridizations and *in situ* hybridizations. *In vitro* techniques for detection of *WUP* polypeptide include enzyme linked immunosorbent assays (ELISAs), Western blots, immunoprecipitations, and immunofluorescence. *In vitro* techniques for detection of *WUP* genomic DNA include Southern hybridizations and fluorescence in situ hybridization (*FISH*). Furthermore, *in vivo* techniques for detecting *WUP* include introducing into a subject a labeled anti-WUP antibody. For example, the antibody can be labeled with a radioactive marker whose presence and location in a subject can be detected by standard imaging techniques.

The biological sample from the subject may contain protein molecules, and/or mRNA molecules, and/or genomic DNA molecules. A preferred biological sample is blood.

The methods may further involve obtaining a biological sample from a subject to provide a control, contacting the sample with a compound or agent to detect WUP, mRNA, or genomic DNA, and comparing the presence of WUP, mRNA or genomic DNA in the control sample with the presence of WUP, mRNA or genomic DNA in the test sample.

The invention also encompasses kits, as defined in the claims, for detecting WUP in a biological sample. For example, the kit can comprise: a labeled compound or agent capable of detecting WUP or *WUP* mRNA in a sample; reagent and/or equipment for determining the amount of WUP in the sample; and reagent and/or equipment for comparing the amount of WUP in the sample with a standard The compound or agent can be packaged in a suitable container. The kit can further comprise instructions for using the kit to detect WUP or nucleic acid.

2. *Prognostic assays*

The diagnostic methods described herein can furthermore be utilized to identify subjects having or at risk of developing a disease or disorder associated with aberrant WUP expression or activity. For example, the assays described herein, can be used to identify a subject having or at risk of developing a disorder associated with WUP, nucleic acid expression or activity. Alternatively, the prognostic assays can be used to identify a subject having or at risk for developing a disease or disorder associated with aberrant WUP expression or activity, in which a test sample is obtained from a subject and WUP or nucleic acid (*e.g.*, mRNA, genomic DNA) is detected A test sample is a biological sample obtained from a subject. For example, a test sample can be a biological fluid (*e.g.*, serum), cell sample, or tissue,

Prognostic assays can be used to determine whether a subject can be administered a modality (*e.g.*, an agonist, antagonist, peptidomimetic, protein, peptide, nucleic acid, small molecule, food, *etc*.) to treat a disease or disorder associated with aberrant WUP expression or activity. Such methods can be used to determine whether a subject can be effectively treated with an agent for a disorder. Methods may determine whether a subject can be effectively treated with an agent for a disorder associated with aberrant WUP expression or activity in which a test sample is obtained and WUP or nucleic acid is detected (*e.g.*, where the presence of WUP or nucleic acid is diagnostic for a subject that can be administered the agent to treat a disorder associated with aberrant WUP expression or activity).

Methods can also be used to detect genetic lesions in a WUP to determine if a subject with the genetic lesion is at risk for a disorder. Methods include detecting, in a sample from the subject, the presence or absence of a genetic lesion characterized by at an alteration affecting the integrity of a gene encoding a WUP polypeptide, or the mis-expression of *WUP*. Such genetic lesions can be detected by ascertaining: (1) a deletion of one or more nucleotides from *WUP;* (2) an addition of one or more nucleotides to *WUP;* (3) a substitution of one or more nucleotides in *WUP*, (4) a chromosomal rearrangement of a WUP gene; (5) an alteration in the level of a *WUP* mRNA transcripts, (6) aberrant modification of a WUP, such as a change genomic DNA methylation, (7) the presence of a non-wild-type splicing pattern of a WUP mRNA transcript, (8) a non-wild-type level of *WUP,* (9) allelic loss of *WUP*, and/or (10) inappropriate post-translational modification of WUP polypeptide. There are a large number of known assay techniques that can be used to detect lesions in *WUP*. Any biological sample containing nucleated cells may be used.

Lesion detection may use a probe/primer in a polymerase chain reaction (PCR) (*e.g.*, (Mullis, US Patent No. 4,683,202, 1987; Mullis et al., US Patent No. 4,683,195, 1987), such as anchor PCR or rapid amplification of cDNA ends (RACE) PCR, or, alternatively, in a ligation chain reaction (LCR) (*e.g.*, (Landegren et al., 1988; Nakazawa et al., 1994), the latter is particularly useful for detecting point mutations in *WUP*-genes (Abravaya et al., 1995). This method may include collecting a sample from a patient, isolating nucleic acids from the sample, contacting the nucleic acids with one or more primers that specifically hybridize to *WUP* under conditions such that hybridization and amplification of the *WUP* (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli et al., 1990), transcriptional amplification system (Kwoh et al., 1989); Qβ Replicase (Lizardi et al., 1988), or any other nucleic acid amplification method, followed by the detection of the amplified molecules using techniques well known to those of skill in the art. These detection schemes are especially useful for the detection of nucleic acid molecules present in low abundance.

Mutations in *WUP* from a sample can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes can be used to score for the presence of specific mutations by development or loss of a ribozyme cleavage site.

Hybridizing a sample and control nucleic acids, *e.g.*, DNA or RNA, to high-density arrays containing hundreds or thousands of oligonucleotides probes, can identify genetic mutations in *WUP* (Cronin et al., 1996; Kozal et al., 1996). For example, genetic mutations in *WUP* can be identified in two-dimensional arrays containing light-generated DNA probes as described in Cronin, et al., supra. Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected Each mutation array is composed of parallel probe sets, one complementary to the wild-type gene and the other complementary to the mutant gene.

In yet another embodiment, any of a variety of sequencing reactions known in the art can be used to directly sequence the *WUP* and detect mutations by comparing the sequence of the sample WUP-with the corresponding wild-type (control) sequence. Examples of sequencing reactions include those based on classic techniques (Maxam and Gilbert, 1977; Sanger et al., 1977). Any of a variety of automated sequencing procedures can be used when performing diagnostic assays (Naeve et al., 1995) including sequencing by mass spectrometry (Cohen et al., 1996; Griffin and Griffin, 1993; Koster, WO94/16101, 1994).

Other methods for detecting mutations in the *WUP* include those in which protection from cleavage agents is used to detect mismatched bases in RNA/RNA or RNA/DNA heteroduplexes (Myers et al., 1985). In general, the technique of "mismatch cleavage" starts by providing heteroduplexes formed by hybridizing (labeled) RNA or DNA containing the wild-type *WUP* sequence with potentially mutant RNA or DNA obtained from a sample. The double-stranded duplexes are treated with an agent that cleaves single-stranded regions of the duplex such as those that arise from base pair mismatches between the control and sample strands. For instance, RNA/DNA duplexes can be treated with RNase and DNA/DNA hybrids treated with $S_1$ nuclease to enzymatically digest the mismatched regions. In other embodiments, either DNA/DNA or RNA/DNA duplexes can be treated with hydroxylamine or osmium tetroxide and with piperidine in order to digest mismatched regions. The digested material is then separated by size on denaturing polyacrylamide gels to determine the mutation site (Grompe et al., 1989; Saleeba and Cotton, 1993). The control DNA or RNA can be labeled for detection.

Mismatch cleavage reactions may employ one or more proteins that recognize mismatched base pairs in double-stranded DNA (DNA mismatch repair) in defined systems for detecting and mapping point mutations in *WUP* cDNAs obtained from samples of cells. For example, the mutY enzyme of *E. coli* cleaves A at G/A mismatches and the thymidine DNA glycosylase from HeLa cells cleaves T at G/T mismatches (Hsu et al., 1994). According to an exemplary embodiment, a probe based on a wild-type *WUP* sequence is hybridized to a cDNA or other DNA product from a test cell(s). The duplex is treated with a DNA mismatch repair enzyme, and the cleavage products, if any, can be detected from electrophoresis protocols or the like (Modrich et al., US Patent No. 5,459,039, 1995).

Electrophoretic mobility alterations can be used to identify mutations in *WUP*. For example, single strand conformation polymorphism (SSCP) may be used to detect differences in electrophoretic mobility between mutant and wild type nucleic acids (Cotton, 1993; Hayashi, 1992; Orita et al., 1989). Single-stranded DNA fragments of sample and control *WUP* nucleic acids are denatured and then renatured. The secondary structure of single-stranded nucleic acids varies according to sequence; the resulting alteration in electrophoretic mobility allows detection of even a single base change. The DNA fragments may be labeled or detected with labeled probes. The sensitivity of the assay may be enhanced by using RNA (rather than DNA), in which the secondary structure is more sensitive to a sequence changes. The subject method may use heteroduplex analysis to separate double stranded heteroduplex molecules on the basis of changes in electrophoretic mobility (Keen et al., 1991).

The migration of mutant or wild-type fragments can be assayed using denaturing gradient gel electrophoresis (DGGE; (Myers et al., 1985). In DGGE, DNA is modified to prevent complete denaturation, for example by adding a GC clamp of approximately 40 bp of high-melting GC-rich DNA by PCR. A temperature gradient may also be used in place of a denaturing gradient to identify differences in the mobility of control and sample DNA (Rossiter and Caskey, 1990).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found (Saiki et al., 1986; Saiki et al., 1989). Such allele-specific oligonucleotides are hybridized to PCR-amplified target DNA or a number of different mutations when the oligonucleotides are attached to the hybridizing membrane and hybridized with labeled target DNA.

Alternatively, allele specific amplification technology that depends on selective PCR amplification may be used Oligonucleotide primers for specific amplifications may carry the mutation of interest in the center of the molecule (so that amplification depends on differential hybridization (Gibbs et al., 1989)) or at the extreme 3'-terminus of one primer where, under appropriate conditions, mismatch can prevent, or reduce polymerase extension (Prosser, 1993). Novel restriction site in the region of the mutation may be introduced to create cleavage-based detection (Gasparini et al., 1992). Certain amplification may also be performed using Taq ligase for amplification (Barany, 1991). In such cases, ligation occurs only if there is a perfect match at the 3'-terminus of the 5' sequence, allowing detection of a known mutation by scoring for amplification.

The described methods may be performed, for example, by using pre-packaged kits comprising at least one probe (nucleic acid or antibody) that may be conveniently used, for example, in clinical settings to diagnose patients exhibiting symptoms or family history of a disease or illness involving WUP.

Furthermore, any cell type or tissue in which WUP is expressed may be utilized in the prognostic assays described herein.

3. *Pharmacogenomics*

Agents, or modulators that have a stimulatory or inhibitory effect on WUP activity or expression, as identified by a screening assay can be administered to individuals to treat, prophylactically or therapeutically, disorders. In conjunction with such treatment, the pharmacogenomics (*i.e.*, the study of the relationship between a subject's genotype and the subject's response to a foreign modality, such as a food, compound or drug) may be considered. Metabolic differences of therapeutics can lead to severe toxicity or therapeutic failure by altering the relation between dose and blood concentration of the pharmacologically active drug. Thus, the pharmacogenomics of the individual permits the selection of effective agents (*e.g.*, drugs) for prophylactic or therapeutic treatments based on a consideration of the individual's genotype. Pharmacogenomics can further be used to determine appropriate dosages and therapeutic regimens. Accordingly, the activity of WUP, expression of *WUP* nucleic acid, or *WUP* mutation(s) in an individual can be determined to guide the selection of appropriate agent(s) for therapeutic or prophylactic treatment.

Pharmacogenomics deals with clinically significant hereditary variations in the response to modalities due to altered modality disposition and abnormal action in affected persons (Eichelbaum and Evert, 1996; Linder et al., 1997). In general, two pharmacogenetic conditions can be differentiated: (1) genetic conditions transmitted as a single factor altering the interaction of a modality with the body (altered drug action) or (2) genetic conditions transmitted as single factors altering the way the body acts on a modality (altered drug metabolism). These pharmacogenetic conditions can occur either as rare defects or as nucleic acid polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.*, N-acetyltransferase 2 (NAT 2) and cytochrome P450 enzymes CYP2D6 and CYP2C 19) explains the phenomena of some patients who show exaggerated drug response and/or serious toxicity after taking the standard and safe dose of a drug. These polymorphisms are expressed in two phenotypes in the population, the extensive metabolizer (EM) and poor metabolizer (PM). The prevalence of PM is different among different populations. For example, the *CYP2D6* gene is highly polymorphic and several mutations have been identified in PM, which all lead to the absence of functional CYP2D6. Poor metabolizers due to mutant *CYP2D6* and *CYP2C19* frequently experience exaggerated drug responses and side effects when they receive standard doses. If a metabolite is the active therapeutic moiety, PM shows no therapeutic response, as demonstrated for the analgesic effect of codeine mediated by its CYP2D6-formed metabolite morphine. At the other extreme are the so-called ultra-rapid metabolizers who are unresponsive to standard doses. Recently, the molecular basis of ultra-rapid metabolism has been identified to be due to CYP2D6 gene amplification.

The activity of WUP, expression of *WUP* nucleic acid, or mutation content of *WUP* in an individual can be determined to select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a WUP modulator, such as a modulator identified by one of the described exemplary screening assays.

4. *Monitoring effects during clinical trials*

Monitoring the influence of agents (*e.g.*, drugs, compounds) on the expression or activity of WUP can be applied not only in basic drug screening, but also in clinical trials. For example, the effectiveness of an agent determined by a screening assay to increase *WUP* expression, protein levels, or up-regulate WUP activity can be monitored in clinical trails of subjects exhibiting decreased *WUP* expression, protein levels, or down-regulated WUP activity. Alternatively, the effectiveness of an agent determined to decrease *WUP* expression, protein levels, or down-regulate WUP activity, can be monitored in clinical trails of subjects exhibiting increased *WUP* expression, protein levels, or up-regulated WUP activity. In such clinical trials, the expression or activity of WUP and, preferably, other genes that have been implicated in, for example, cancer can be used as a "read out" or markers for a particular cell's responsiveness.

For example, genes, including *WUP,* that are modulated in cells by treatment with a modality (*e.g.*, food, compound, drug or small molecule) can be identified. To study the effect of agents on cancer, for example, in a clinical trial, cells can be isolated and RNA prepared and analyzed for the levels of expression of *WUP* and other genes implicated in the disorder. The gene expression pattern can be quantified by Northern blot analysis, nuclear run-on or RT-PCR experiments, or by measuring the amount of protein, or by measuring the activity level of WUP or other gene products. In this manner, the gene expression pattern itself can serve as a marker, indicative of the cellular physiological response to the agent. Accordingly, this response state may be determined before, and at various points during, treatment of the individual with the agent.

The effectiveness of treatment of a subject with an agent (*e.g.*, an agonist, antagonist, protein, peptide, peptidomimetic, nucleic acid, small molecule, food or other drug candidate identified by the screening assays described herein) may be monitored, by the steps of (1) obtaining a pre-administration sample from a subject; (2) detecting the level of expression of a WUP, mRNA, or genomic DNA in the preadministration sample; (3) obtaining one or more post-administration samples from the subject; (4) detecting the level of expression or activity of the WUP, mRNA, or genomic DNA in the post-administration samples; (5) comparing the level of expression or activity of the WUP, mRNA, or genomic DNA in the pre-administration sample with the WUP, mRNA, or genomic DNA in the post administration sample or samples; and (6) altering the administration of the agent to the subject accordingly. For example, increased administration of the agent may be desirable to increase the expression or activity of WUP to higher levels than detected, i.e., to increase the effectiveness of the agent. Alternatively, decreased administration of the agent may be desirable to decrease expression or activity of WUP to lower levels than detected, *i.e.*, to decrease the effectiveness of the agent.

5. *Uses for treatment*

Prophylactic and therapeutic uses may treat a subject at risk of (or susceptible to) a disorder or having a disorder associated with aberrant WUP expression or activity. Examples include disorders in which cell metabolic demands (and consequently, demands on mitochondria and endoplasmic reticulum) are high, such as during rapid cell growth. Examples of such disorders and diseases include cancers, such as melanoma, breast cancer or colon cancer.

6. *Disease and disorders*

Diseases and disorders that are characterized by increased WUP levels or biological activity may be treated with therapeutics that antagonize (*i.e.,* reduce or inhibit) activity. Antagonists may be administered in a therapeutic or prophylactic manner. Therapeutics that may be used include: (1) WUP peptides, or analogs, derivatives, fragments or homologs thereof; (2) Abs to a WUP peptide; (3) *WUP* nucleic acids; (4) administration of antisense nucleic acid and nucleic acids that are "dysfunctional" *(i.e..* due to a heterologous insertion within the coding sequences) that are used to eliminate endogenous function of by homologous recombination (Capecchi, 1989); or (5) modulators (*i.e.*, inhibitors, agonists and antagonists, including additional peptide mimetic or Abs specific to WUP) that alter the interaction between WUP and its binding partner.

Diseases and disorders that are characterized by decreased WUP levels or biological activity may be treated with therapeutics that increase (*i.e.*, are agonists to) activity. Therapeutics that upregulate activity may be administered therapeutically or prophylactically. Therapeutics that may be used include peptides, or analogs, derivatives, fragments or homologs thereof; or an agonist that increases bioavailability.

Increased or decreased levels can be readily detected by quantifying peptide and/or RNA, by obtaining a patient tissue sample (*e.g.*, from biopsy tissue) and assaying *in vitro* for RNA or peptide levels, structure and/or activity of the expressed peptides (or *WUP* mRNAs). Methods include, but are not limited to, immunoassays (*e.g.*, by Western blot analysis, immunoprecipitation followed by sodium dodecyl sulfate (SDS) polyacrylamide gel electrophoresis, immunocytochemistry, *etc.)* and/or hybridization assays to detect expression of mRNAs (*e.g.*, Northern assays, dot blots, in situ hybridization, and the like)

7. *Prophylactic methods*

Subjects at risk for a disease that is caused or contributed to by aberrant WUP expression or activity can be identified by, for example, any or a combination of diagnostic or prognostic assays. Administration of a prophylactic agent can occur prior to the manifestation of symptoms characteristic of the WUP aberrancy, such that a disease or disorder is prevented or, alternatively, delayed in its progression. Depending on the type of WUP aberrancy, for example, a WUP agonist or WUP antagonist may be used to treat the subject The appropriate agent can be determined based on screening assays.

8. *Therapeutic methods*

Another aspect of the invention pertains to methods for identifying modulators of WUP expression or activity for therapeutic purposes. An agent that modulates WUP activity can be a nucleic acid or a protein, a naturally occurring cognate ligand of WUP, a peptide, a WUP peptidomimetic, or other small molecule. The agent may stimulate WUP activity. Examples of such stimulatory agents include active WUP and a *WUP* nucleic acid molecule that has been introduced into the cell. In another embodiment, the agent inhibits WUP activity. Examples of inhibitory agents include antisense *WUP* nucleic acids and anti-WUP Abs. Modulatory methods can be performed *in vitro* (*e.g.*, by culturing the cell with the agent) or, alternatively, *in vivo* (*e.g.*, by administering the agent to a subject). As such, the invention provides methods of identifying agents suitable for treating an individual afflicted with a disease or disorder characterized by aberrant expression or activity of a WUP or nucleic acid molecule. In this regard an agent (*e.g.*, an agent identified by a screening assay), or combination of agents that modulates (*e.g.*, up-regulates or down-regulates) WUP expression or activity may be employed. Alternatively, a WUP or nucleic acid molecule may be employed as therapy to compensate for reduced or aberrant WUP expression or activity.

Stimulation of WUP activity is desirable in situations in which WUP is abnormally down-regulated and/or in which increased WUP activity is likely to have a beneficial effect.

9. *Determination of the biological effect of the therapeutic*

Suitable *in vitro* or *in vivo* assays can be performed to determine the effect of a specific therapeutic and whether its administration is indicated for treatment of the affected time.

In various specific embodiments, *in vitro* assays may be performed with representative cells of the type(s) involved in the patient's disorder, to determine if a given therapeutic exerts the desired effect upon the cell type(s). Modalities for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

10. *Prophylactic and therapeutic uses of the compositions of the invention*

WUP nucleic acids and proteins are useful in potential prophylactic and therapeutic applications implicated in a variety of disorders including, but not limited to cancer.

As an example, a cDNA encoding WUP may be useful in gene therapy, and the protein may be useful when administered to a subject in need thereof. By way of non-limiting example, the compositions of the invention will have efficacy-for treatment of patients suffering from cancer.

*WUP* nucleic acids, or fragments thereof, may also be useful in diagnostic applications, wherein the presence or amount of the nucleic acid or the protein is to be assessed. A further use could be as an anti-bacterial molecule (*i.e.*, some peptides have been found to possess anti-bacterial properties). These materials are further useful in the generation of Abs that immunospecifically bind to the novel substances of the invention for use in therapeutic or diagnostic methods.

## EXAMPLE

The following example's experimental details can be found in (Pennica et al., 1998).

Wnt proteins mediate diverse developmental processes such as the control of cell proliferation, adhesion, cell polarity, and the establishment of cell fates. Although Wnt-1 is not expressed in normal mammary gland, expression of Wnt-1 in transgenic mice causes mammary tumors.

A PCR-based cDNA subtraction strategy, suppression subtractive hybridization (SSH) (Diatchenko et al., 1996), using RNA isolated from C57MG mouse mammary epithelial cells and C57MG cells stably transformed by a Wnt-1 retrovirus. Overexpression of Wnt-I in this cell line is sufficient to induce a partially transformed phenotype, characterized by elongated and refractile cells that lose contact inhibition and form a multilayered array (Brown et al., 1986; Wong et al., 1994). Genes that are differentially expressed between these two cell lines likely contribute to the transformed phenotype.

1. *Methods*

***SSH***. SSH was performed by using the PCR-Select cDNA Subtraction Kit (CLONTECH). Tester double-stranded cDNA was synthesized from 2 μg of poly(A)$^+$ RNA isolated from the C57MG/Wnt-I cell line and driver cDNA from 2 μg of poly(A)$^+$ RNA from the parent C57MG cells. The subtracted cDNA library was subcloned into a pGEM-T vector for further analysis.

***Expression of Human WUP RNA.*** PCR amplification of first-strand cDNA was performed with human Multiple Tissue cDNA panels (CLONTECH) and 300 μM of each dNTP at 94°C for 1 sec, 62°C for 30 sec, 72°C for 1 min, for 22-32 cycles.

*Gene Amplification and RNA Expression Analysis.* Relative gene amplification and RNA expression of *WUP* and *c-myc* in the cell lines were determined by quantitative PCR. Gene-specific primers and fluorogenic probes were designed and used to amplify and quantitate the genes. The -method was used for calculation of the SE of RNA expression levels. The *WUP*-specific signal was normalized to that of the glyceraldehyde-3-phosphate dehydrogenase housekeeping gene. All TaqMan assay reagents were obtained from Perkin-Elmer Applied Biosystems.

2. *Results*

To identify Wnt-1-inducible genes, the technique of SSH using the mouse mammary epithelial cell line C57MG and C57MG cells that stably express Wnt-1 and Wnt-4 was used. Candidate differentially expressed cDNAs (1,384 total) were sequenced. Thirty-nine percent of the sequences matched known genes or homologues, 32% matched expressed sequence tags, and 29% had no match. To confirm that the transcript was differentially expressed, Semiquantitative reverse transcription-PCR analysis was performed by using mRNA from the C57MG and C57MG/Wnt-1 cells.

The SSH technique determined that WUP was upregulated in Wnt-1 expressing cells 2-3-fold than that expressed in wild-type or Wnt-4-expressings C57MG cells. Quantitative PCR analysis (TaqMan)confirmed the upregulation, giving 1.4 fold increase in Wnt-1 expressing cells as opposed to wild-type or Wnt-4 expressing cells.

REFERENCES

U.S. Patent No. 4166452. Apparatus for testing human responses to stimuli. 1979.
U.S. Patent No. 4485045. Synthetic phosphatidyl cholines useful in forming liposomes. 1984.
U.S. Patent No. 4544545. Liposomes containing modified cholesterol for organ targeting. 1985.

4,676,980. Target specific cross-linked heteroantibodies. 1987.
U.S. Patent No. 4816567. Recombinant immunoglobin preparations. 1989.
WO 90/10448. Covalent conjugates of lipid and oligonucleotide. 1990.
WO 90/13641. Stably transformed eucaryotic cells comprisng a foreign transcribable DNA under the control of a pol III promoter. 1990.
EPO 402226. Transformation vectors for yeast *Yarrowia.* 1990.
WO 91/00360. Bispecific reagents for AIDS therapy. 1991.
WO 91/04753. Conjugates of antisense oligonucleotides and therapeutic uses thereof. 1991.
U.S. Patent No. 5013556. Liposomes with enhanced circulation time. 1991.
WO 91/06629. Oligonucleotide analogs with novel linkages. 1991.
WO 92/20373. Heteroconjugate antibodies for treatment of HIV infection. 1992.
WO 93/08829. Compositions that mediate killing of HIV-infected cells. 1993.
WO 94/11026. Therapeutic application of chimeric and radiolabeled antibodies to human B lymphocyte restricted differentiation antigen for treatment of B cells. 1994.
WO 96/27011. A method for making heteromultimeric polypeptides. 1996.
U.S. Patent No. 5545807. Production of antibodies from transgenic animals. 1996.
U.S. Patent No. 5545806. Ransgenic <sic> non-human animals for producing heterologous antibodies. 1996.
U.S. Patent No. 5569825. Transgenic non-human animals capable of producing heterologous antibodies of various isotypes. 1996.
WO 97/33551. Compositions and methods for the diagnosis, prevention, and treatment of neoplastic cell growth and proliferation. 1997.
U.S. Patent No. 5633425. Transgenic non-human animals capable of producing heterologous antibodies. 1997.
U.S. Patent No. 5661016. Transgenic non-human animals capable of producing heterologous antibodies of various isotypes. 1997.
U.S. Patent No. 5625126. Transgenic non-human animals for producing heterologous antibodies. 1997.
Abravaya, K., J.J. Carrino, S. Muldoon, and H.H. Lee. 1995. Detection of point mutations with a modified ligase chain reaction (Gap- LCR). Nucleic Acids Res. 23:675-82.
Alam, J., and J.L. Cook. 1990. Reporter genes: Application to the study of mammalian gene transcription. Anal. Biochem. 188:245-254.
Austin, C.P., and C.L. Cepko. 1990. Cellular migration patterns in the developing mouse cerebral cortex. Development. 110:713-732.
Ausubel, F.M., R. Brent, R.E. Kingston, D.D. Moore, et al. 1987. Current protocols in molecular biology. John Wiley & Sons, New York.
Barany, F. 1991. Genetic disease detection and DNA amplification using cloned thermostable ligase. Proc Natl Acad Sci USA. 88:189-93.
Bartel, D.P., and J.W. Szostak. 1993. Isolation of new ribozymes from a large pool of random sequences [see comment]. Science. 261:1411-8.
Bartel, P., C.T. Chien, R. Sternglanz, and S. Fields. 1993. Elimination of false positives that arise in using the two-hybrid system. Biotechniques. 14:920-4.
Beal, P.A., and P.B. Dervan. 1991. Second structural motif for recognition of DNA by oligonucleotide- directed triple-helix formation. Science. 251:1360-3.
Bechtold, N., and G. Pelletier. 1998. In planta Agrobacterium-mediated transformation of adult Arabidopsis thaliana plants by vacuum infiltration. Methods Mol Biol. 82:259-66.
Becker, D.M., and L. Guarente. 1991. High-efficiency transformation of yeast by electroporation. Methods Enzymol. 194:182-187.
Beggs, J.D. 1978. Transformation of yeast by a replicating hybrid plasmid. Nature. 275 :104-109.
Berger, J., J. Hauber, R. Hauber, R. Geiger, et al. 1988. Secreted placental alkaline phosphatase: A powerful new qunatitative indicator of gene expression in eukaryotic cells. Gene. 66:1-10.
WO 93/04169. GENE TARGETING IN ANIMAL CELLS USING ISOGENIC DNA CONSTRUCTS. 1993.
Bodine, D.M., K.T. McDonagh, N.E. Seidel, and A.W. Nienhuis. 1991. Survival and retrovirus infection of murine hematopoietic stem cells in vitro: effects of 5-FU and method of infection. Exp. Hematol. 19:206-212.
Boerner, P., R. Lafond, W.Z. Lu, P. Brams, et al. 1991. Production of antigen-specific human monoclonal antibodies from in vitro-primed human splenocytes. J Immunol. 147:86-95.
U.S. Patent No. 3,773,919. Polylactide-drug mixtures. 1973.
Bradley. 1987. Teratocarcinomas and Embryonic Stem Cells: A Practical Approach. Oxford University Press, Inc., Oxford. 268 pp.
Bradley, A. 1991. Modifying the mammalian genome by gene targeting. Curr Opin Biotechnol. 2:823-9.
Brennan, M., P.F. Davison, and H. Paulus. 1985. Preparation of bispecific antibodies by chemical recombination of monoclonal immunoglobulin G1 fragments. Science. 229:81-3.
WO94/10300. INTERACTION TRAP SYSTEM FOR ISOLATING NOVEL PROTEINS. 1994.
Brown, A.M., R.S. Wildin, T.J. Prendergast, and H.E. Varmus. 1986. A retrovirus vector expressing the putative mammary oncogene int-1 causes partial transformation of a mammary epithelial cell line. Cell. 46:1001-9.
Capecchi, M.R. 1980. High efficiency transformation by direct microinjection of DNA into cultured mammalian cells. Cell. 22:479.
Capecchi, M.R. 1989. Altering the genome by homologous recombination. Science. 244:1288-92.
Carell, T., E.A. Wintner, and J. Rebek Jr. 1994a. A novel procedure for the synthesis of libraries containing small organic molecules. Angewandte Chemie International Edition. 33:2059-2061.
Carell, T., E.A. Wintner, and J. Rebek Jr. 1994b. A solution phase screening procedure for the isolation of active compounds from a molecular library. Angewandte Chemie International Edition. 33:2061-2064.
Caron, P.C., W. Laird, M.S. Co, N.M. Avdalovic, et al. 1992. Engineered humanized dimeric forms of IgG are more effective antibodies. J Exp Med. 176:1191-5.
Carter, P. 1986. Site-directed mutagenesis. Biochem J. 237:1-7.
Case, M.E., M. Schweizer, S.R. Kushner, and N.H. Giles. 1979. Efficient transformation of Neurospora crassa by utilizing hybrid plasmid DNA. Proc Natl Acad Sci USA. 76:5259-63.
U.S. Patent No. 5,116,742. RNA ribozyme restriction endoribonucleases and methods. 1992.
U.S. Patent No. 4,987,071. RNA ribozyme polymerases, dephosphorylases, restriction endoribonucleases and methods. 1991.
Cepko, C.L., B.E. Roberts, and R.E. Mulligan. 1984. Construction and applications of a highly transmissible murine retrovirus shuttle vector. Cell. 37:1053-1062.
Chalfie, M., Y. tu, G. Euskirchen, W.W. Ward, et al. 1994. Green fluorescent protein as a marker for gene expression. Science. 263:802-805.
Chaney, W.G., D.R. Howard, J.W. Pollard, S. Sallustio, et al. 1986. High-frequency transfection of CHO cells using Polybrene. Somatic Cell Mol. Genet. 12:237.
Chen, C., and H. Okayama. 1988. Calcium phosphate-mediated gene transfer: A highly efficient system for stably transforming cells with plasmid DNA. BioTechniques. 6:632-638.
Chen, S.H., H.D. Shine, J.C. Goodman, R.G. Grossman, et al. 1994. Gene therapy for brain tumors: regression of experimental gliomas by adenovirus-mediated gene transfer in vivo. Proc Natl Acad Sci U S A. 91:3054-7.
Cho, C.Y., E.J. Moran, S.R. Cherry, J.C. Stephans, et al. 1993. An unnatural biopolymer. Science. 261:1303-5.
Cohen, A.S., D.L. Smisek, and B.H. Wang. 1996. Emerging technologies for sequencing antisense oligonucleotides: capillary electrophoresis and mass spectrometry. Adv Chromatogr. 36:127-62.
Cohen, J.S. 1989. Oligodeoxynucleotides: Antisense inhibitors of gene expression. CRC Press, Boca Raton, FL. 255 pp.
Cohen, S.M.N., A.C.Y. Chang, and L. Hsu. 1972. Nonchromosomal antibiotic resistance in bacteria: Genetic transformation of Escherichia coli by R-factor DNA. Proc. Natl. Acad. Sci. USA. 69:2110.
Cooney, M., G. Czernuszewicz, E.H. Postel, S.J. Flint, et al. 1988. Site-specific oligonucleotide binding represses transcription of the human c-myc gene in vitro. Science. 241:456-9.
Cotton, R.G. 1993. Current methods of mutation detection. Mutat Res. 285:125-44.
Cronin, M.T., R.V. Fucini, S.M. Kim, R.S. Masino, et al. 1996. Cystic fibrosis mutation detection by hybridization to light-generated DNA probe arrays. Hum Mutat. 7:244-55.
Cull, M.G., J.F. Miller, and P.J. Schatz. 1992. Screening for receptor ligands using large libraries of peptides linked to the C terminus of the lac repressor. Proc Natl Acad Sci U S A. 89:1865-9.
Cwirla, S.E., E.A. Peters, R.W. Barrett, and W.J. Dower. 1990. Peptides on phage: a vast library of peptides for identifying ligands. Proc Natl Acad Sci USA. 87:6378-82.
de Boer, A.G. 1994. Drug absorption enhancement: Concepts, possibilities, limitations and trends. Harwood Academic Publishers, Langhorne, PA.
de Louvencourt, L., H. Fukuhara, H. Heslot, and M. Wesolowski. 1983. Transformation of Kluyveromyces lactis by killer plasmid DNA. J Bacteriol: 154:737-42.
de Wet, J.R., K.V. Wood, M. DeLuca, D.R. Helinski, et al. 1987. Sturcture and expression in mammalian cells. Mol. Cell Biol. 7:725-737.
Demerec, M., E.A. Adelberg, A.J. Clark, and P.E. Hartman. 1966. A proposal for a uniform nomenclature in bacterial genetics. Genetics. 54:61-76.
Devlin, J.J., L.C. Panganiban, and P.E. Devlin. 1990. Random peptide libraries: a source of specific protein binding molecules. Science. 249:404-6.
DeWitt, S.H., J.S. Kiely, C.J. Stankovic, M.C. Schroeder, et al. 1993. "Diversomers": an approach to nonpeptide, nonoligomeric chemical diversity. Proc Natl.Acad Sci U S A. 90:6909-13.
Diatchenko, L., Y.F. Lau, A.P. Campbell, A. Chenchik, et al. 1996. Suppression subtractive hybridization: a method for generating differentially regulated or tissue-specific cDNA probes and libraries. Proc Natl Acad Sci U S A. 93: 6025-30.
Eichelbaum, M., and B. Evert. 1996. Influence of pharmacogenetics on drug disposition and response. Clin Exp Pharmacol Physiol. 23:983-5.
Ellington, A.D., and J.W. Szostak. 1990. In vitro selection of RNA molecules that bind specific ligands. Nature. 346: 818-22.
Elroy-Stein, O., and B. Moss. 1990. Cytoplasmic expression system based on constitutive synthesis of bacteriophage T7 RNA polymerase in mammalian cells. Proc. Natl. Acad. Sci. USA. 87:6743-6747.
US Patent No. 4,522,811. Serial injection of muramyldipeptides and liposomes enhances the anti-infective activity of muramyldipeptides Serial injection of muramyldipeptides and liposomes enhances the anti-infective activity of muramyldipeptides. 1985.
Eppstein, D.A., Y.V. Marsh, M. van der Pas, P.L. Felgner, et al. 1985. Biological activity of liposome-encapsulated murine interferon gamma is mediated by a cell membrane receptor. Proc Natl Acad Sci U S A. 82:3688-92.
Escudero, J., and B. Hohn. 1997. Transfer and integration of T-DNA without cell injury in the host plant. Plant Cell. 9:2135-2142.
U.S. Patent No. 4,870,009. Method of obtaining gene product through the generation of transgenic animals. 1989.
Fekete, D.M., and C.L. Cepko. 1993. Retroviral infection coupled with tissue transplantation limits gene transfer in the chick embryo. Proc. Natl. Acad. Sci. USA. 90:2350-2354.
Felgner, P.L., T.R. Gadek, M. Holm, R. Roman, et al. 1987. Lipofectin: A highly efficient, lipid-mediated DNA/ transfection procedure. Proc. Natl. Acad. Sci. USA. 84:7413-7417.
Felici, F., L. Castagnoli, A. Musacchio, R. Jappelli, et al. 1991. Selection of antibody ligands from a large library of oligopeptides expressed on a multivalent exposition vector. J Mol Biol. 222:301-10.
Fieck, A., D.L. Wyborski, and J.M. Short. 1992. Modifications of the E.coli Lac repressor for expression in eukaryotic cells: effects of nuclear signal sequences on protein activity and nuclear accumulation. Nucleic Acids Res. 20: 1785-91.
Finer, J.J., K.R. Finer, and T. Ponappa. 1999. Particle bombardment-mediated transformation. Current Topics in microbiology and immunology. 240:59-80.
Finn, P.J., N.J. Gibson, R. Fallon, A. Hamilton, et al. 1996. Synthesis and properties of DNA-PNA chimeric oligomers. Nucleic Acids Res. 24:3357-63.
Fishwild, D.M., S.L. ODonnell, T. Bengoechea, D.V. Hudson, et al. 1996. High-avidity human IgG kappa monoclonal antibodies from a novel strain of minilocus transgenic mice [see comments]. Nat Biotechnol. 14:845-51.
Fleer, R., P. Yeh, N. Amellal, I. Maury, et al. 1991. Stable multicopy vectors for high-level secretion of recombinant human serum albumin by Kluyveromyces yeasts. Biotechnology (N Y). 9:968-75.
Fodor, S.P., R.P. Rava, X.C. Huang, A.C. Pease, et al. 1993. Multiplexed biochemical assays with biological chips. Nature. 364:555-6.
Fromm, M., L.P. Taylor, and V. Walbot. 1985. Expression of genes transferred into monocot and dicot plant cells by electroporation. Proc. Natl. Acad. Sci. USA. 82:5824-5828.
Fujita, T., H. Shubiya, T. Ohashi, K. Yamanishi, et al. 1986. Regulation of human interleukin-2 gene: Functional DNA sequences in the 5' flanking region for the gene expression in activated T lymphocytes. Cell. 46:401-407.
Gabizon, A., R. Shiota, and D. Papahadjopoulos. 1989. Pharmacokinetics and tissue distribution of doxorubicin encapsulated in stable liposomes with long circulation times. J Natl Cancer Inst. 81:1484-8.
Gallagher, S.R. 1992. GUS protocols: Using the GUS gene as a reporter of gene expression. Academic Press, San Diego, CA.
Gallop, M.A., R.W. Barrett, W.J. Dower, S.P. Fodor, et al. 1994. Applications of combinatorial technologies to drug discovery. 1. Background and peptide combinatorial libraries. J Med Chem. 37:1233-51.
Gasparini, P., A. Bonizzato, M. Dognini, and P.F. Pignatti. 1992. Restriction site generating-polymerase chain reaction (RG-PCR) for the probeless detection of hidden genetic variation: application to the study of some common cystic fibrosis mutations. Mol Cell Probes. 6:1-7.
Gautier, C., F. Morvan, B. Rayner, T. Huynh-Dinh, et al. 1987. Alpha-DNA. IV: Alpha-anomeric and beta-anomeric tetrathymidylates covalently linked to intercalating oxazolopyridocarbazole. Synthesis, physicochemical properties and poly (rA) binding. Nucleic Acids Res. 15:6625-41.
Gennaro, A.R. 2000. Remington: The science and practice of pharmacy. Lippincott, Williams & Wilkins, Philadelphia, PA.
Gibbs, R.A., P.N. Nguyen, and C.T. Caskey. 1989. Detection of single DNA base differences by competitive oligonucleotide priming. Nucleic Acids Res. 17:2437-48.
Gietz, R.D., R.A. Woods, P. Manivasakam, and R.H. Schiestl. 1998. Growth and transformation of Saccharomyces cerevisiae. In Cells: A laboratory manual. Vol. I. D. Spector, R. Goldman, and L. Leinwand, editors. Cold Spring Harbor Press, Cold Spring Harbor, NY.

Goding, J.W. 1996. Monoclonal antibodies: Principles and Practice. Academic Press, San Diego. 492 pp.
Gorman, C.M., L.F. Moffat, and B.H. Howard. 1982. Recombinant genomes which express chloramphenicol acetyltransferase in mammalian cells. Mol. Cell. Biol. 2:1044-1051.
Graham, F.L., and A.J. van der Eb. 1973. A new technique for the assay of infectivity of human adenovirus 5 DNA. Virology. 52:456-.
Griffin, H.G., and A.M. Griffin. 1993. DNA sequencing. Recent innovations and future trends. Appl Biochem Biotechnol. 38:147-59.
Grompe, M., D.M. Muzny, and C.T. Caskey. 1989. Scanning detection of mutations in human ornithine transcarbamoylase by chemical mismatch cleavage. Proc Natl Acad Sci U S A. 86:5888-92.
Gruber, M., B.A. Schodin, E.R. Wilson, and D.M. Kranz. 1994. Efficient tumor cell lysis mediated by a bispecific single chain antibody expressed in Escherichia coli. J Immunol. 152:5368-74.
Guatelli, J.C., K.M. Whitfield, D.Y. Kwoh, K.J. Barringer, et al. 1990. Isothermal, in vitro amplification of nucleic acids by a multienzyme reaction modeled after retroviral replication. Proc Natl Acad Sci U S A. 87:1874-8.
Hanahan, D. 1983. Studies on transformation of Escherichia coli with plasmids. J. Mol. Biol. 166:557-580.
Hansen, G., and M.-D. Chilton. 1999. Lessons in gene transfer to plants by a gifted microbe. Curr. Top. Microbiol. Immunol. 240:21-57.
Hansen, G., and M.S. Wright. 1999. Recent advances in the transformation of plants. Trends Plant Sci. 4:226-231.
Harlow, E., and D. Lane. 1988. Antibodies: A laboratory manual. Cold Spring Harbor Laboratory Press, Cold Spring Harbor. 726 pp.
Harlow, E., and D. Lane. 1999. Using antibodies: A laboratory manual. Cold Spring Harbor Laboratory PRess, Cold Spring Harbor, New York.
Haseloff, J., and W.L. Gerlach. 1988. Simple RNA enzymes with new and highly specific endoribonuclease activities. Nature. 334:585-91.
Hayashi, K. 1992. PCR-SSCP: A method for detection of mutations. Genetic and Analytical Techniques Applications. 9:73-79.
Helene, C. 1991. The anti-gene strategy: control of gene expression by triplex-forming- oligonucleotides. Anticancer Drug Des. 6:569-84.
Helene, C., N.T. Thuong, and A. Harel-Bellan. 1992. Control of gene expression by triple helix-forming oligonucleotides. The antigene strategy. Ann NY Acad Sci. 660:27-36.
Hinnen, A., J.B. Hicks, and G.R. Fink. 1978. Transformation of yeast. Proc. Natl. Acad. Sci. USA. 75:1929-1933.
Hoffman, F. 1996. Laser microbeams for the manipulation of plant cells and subcellular structures. Plant Sci. 113: 1-11.
Hogan, B., Beddington, R., Costantini, F., Lacy, E. 1994. Manipulating the Mouse Embryo: A Laboratory Manual. Cold Spring Harbor Laboratory Press. 500 pp.
Holliger, P., T. Prospero, and G. Winter. 1993. "Diabodies": small bivalent and bispecific antibody fragments. Proc Natl Acad Sci U S A. 90:6444-8.
Hoogenboom, H.R., A.D. Griffiths, K.S. Johnson, D.J. Chiswell, et al. 1991. Multi-subunit proteins on the surface of filamentous phage: methodologies for displaying antibody (Fab) heavy and light chains. Nucleic Acids Res. 19: 4133-7.
Houghten, R.A., J.R. Appel, S.E. Blondelle, J.H. Cuervo, et al. 1992. The use of synthetic peptide combinatorial libraries for the identification of bioactive peptides. Biotechniques. 13:412-21.
Hsu, I.C., Q. Yang, M.W. Kahng, and J.F. Xu. 1994. Detection of DNA point mutations with DNA mismatch repair enzymes. Carcinogenesis. 15:1657-62.
Hwang, K.J., K.F. Luk, and P.L. Beaumier. 1980. Hepatic uptake and degradation of unilamellar sphingomyelin/ cholesterol liposomes: a kinetic study. Proc Natl Acad Sci USA. 77:4030-4.
Hyrup, B., and P.E. Nielsen. 1996. Peptide nucleic acids (PNA): synthesis, properties and potential applications. Bioorg Med Chem. 4:5-23.
Inoue, H., Y. Hayase, A. Imura, S. Iwai, et al. 1987a. Synthesis and hybridization studies on two complementary nona(2'-O- methyl)ribonucleotides. Nucleic Acids Res. 15:6131-48.
Inoue, H., Y. Hayase, S. Iwai, and E. Ohtsuka. 1987b. Sequence-dependent hydrolysis of RNA using modified oligonucleotide splints and RNase H. FEBS Lett. 215:327-30.
Ishiura, M., S. Hirose, T. Uchida, Y. Hamada, et al. 1982. Phage particle-mediated gene transfer to cultured mammalian cells. Molecular and Cellular Biology. 2:607-616.
Ito, H., Y. Fukuda, K. Murata, and A. Kimura. 1983. Transformation of intact yeast cells treated with alkali cations. J. Bacteriol. 153:163-168.
Iwabuchi, K., B. Li, P. Bartel, and S. Fields. 1993. Use of the two-hybrid system to identify the domain of p53 involved in oligomerization. Oncogene. 8:1693-6.
Jayasena, S.D. 1999. Aptamers: an emerging class of molecules that rival antibodies in diagnostics. Clin Chem.

45:1628-50.
Jones, P.T., P.H. Dear, J. Foote, M.S. Neuberger, et al. 1986. Replacing the complementarity-determining regions in a human antibody with those from a mouse. Nature. 321:522-5.
Kaufman, R.J. 1990. Vectors used for expression in mammalian cells. Methods Enzymol. 185:487-511.
Kaufman, R.J., P. Murtha, D.E. Ingolia, C.-Y. Yeung, et al. 1986. Selection and amplification of heterologous genes encoding adenosine deaminase in mammalian cells. Proc. Natl. Acad. Sci. USA. 83:3136-3140.
Kawai, S., and M. Nishizawa. 1984. New procedure for DNA transfection with polycation and dimethyl sulfoxide. Mol. Cell. Biol. 4:1172.
Keen, J., D. Lester, C. Inglehearn, A. Curtis, et al. 1991. Rapid detection of single base mismatches as heteroduplexes on Hydrolink gels. Trends Genet. 7:5.
Kelly, J.M., and M.J. Hynes. 1985. Transformation of Aspergillus niger by the amdS gene of Aspergillus nidulans. Embo J. 4:475-9.
Kostelny, S.A., M.S. Cole, and J.Y. Tso. 1992. Formation of a bispecific antibody by the use of leucine zippers. J Immunol. 148:1547-53.
WO94/16101. DNA SEQUENCING BY MASS SPECTROMETRY. 1994.
Kozal, M.J., N. Shah, N. Shen, R. Yang et al. 1996. Extensive polymorphisms observed in HIV-1 clade B protease gene using high-density oligonucleotide arrays. Nat Med. 2:753-9.
Kozbor, D., P. Tripputi, J.C. Roder, and C.M. Croce. 1984. A human hybrid myeloma for production of human monoclonal antibodies. J Immunol. 133:3001-5.
Kriegler, M. 1990. Gene transfer and expression: A laboratory manual. Stockton Press, New York. 242 pp.
WO 91/01140. HOMOLOGOUS RECOMBINATION FOR UNIVERSAL DONOR CELLS AND CHIMERIC MAMMALIAN HOSTS. 1991.
Kwoh, D.Y., G.R. Davis, K.M. Whitfield, H.L. Chappelle, et al. 1989. Transcription-based amplification system and detection of amplified human immunodeficiency virus type 1 with a bead-based sandwich hybridization format. Proc Natl Acad Sci U S A. 86:1173-7.
US Patent No. 5,223,409. Directed evolution of novel binding proteins. 1993.
Lakso, M., B. Sauer, B. Mosinger, E.J. Lee, et al. 1992. Targeted oncogene activation by site-specific recombination in transgenic mice. Proc Natl Acad Sci US A. 89:6232-6.
Lam, K.S. 1997. Application of combinatorial library methods in cancer research and drug discovery. Anticancer Drug Design. 12:145-167.
Lam, K.S., S.E. Salmon, E.M. Hersh, V.J. Hruby, et al. 1991. General method for rapid synthesis of multicomponent peptide mixtures. Nature. 354:82-84.
Landegren, U., R. Kaiser, J. Sanders, and L. Hood. 1988. A ligase-mediated gene detection technique. Science. 241:1077-80.
WO 90/11354. Process for the specific replacement of a copy of a gene present in the receiver genome via the integration of a gene. 1990.
U.S. Patent No. 4,736,866. Transgenic non-human animals. 1988.
Leduc, N., and e. al. 1996. Isolated maize zygotes mimic in vivo embryogenic development and express microinjected genes when cultured in vitro. Dev. Biol. 10:190-203.
Lee, J.S., D.A. Johnson, and A.R. Morgan. 1979. Complexes formed by (pyrimidine)n (purine)n DNAs on lowering the pH are three-stranded. Nucleic Acids Res. 6:3073-91.
Lee, V.H.L. 1990. Peptide and protein drug delivery. Marcel Dekker, New York, NY.
Lemaitre, M., B. Bayard, and B. Lebleu. 1987. Specific antiviral activity of a poly(L-lysine)-conjugated oligodeoxyribonucleotide sequence complementary to vesicular stomatitis virus N protein mRNA initiation site. Proc Natl Acad Sci USA. 84:648-52.
Lemischka, I.R., D.H. Raulet, and R.C. Mulligan. 1986. Developmental potential and dynamic behavior of hematopoietic stem cells. Cell. 45:917-927.
Letsinger, R.L., G.R. Zhang, D.K. Sun, T. Ikeuchi, et al. 1989. Cholesteryl-conjugated oligonucleotides: synthesis, properties, and activity as inhibitors of replication of human immunodeficiency virus in cell culture. Proc Natl Acad Sci U S A. 86:6553-6.
Li, E., T.H. Bestor, and R. Jaenisch. 1992. Targeted mutation of the DNA methyltransferase gene results in embryonic lethality. Cell. 69:915-26.
Linder, M.W., R.A. Prough, and R. Valdes. 1997. Pharmacogenetics: a laboratory tool for optimizing therapeutic efficiency. Clin Chem. 43:254-66.
Littlefield, J.W. 1964. Selection of hybrids from matings of fibroblasts in vitro and their presumed recombinants. Science. 145:709-710.
Lizardi, P.M., C.E. Guerra, H. Lomeli, I. Tussie-Luna, et al. 1988. Exponential amplification of recombinant-RNA hybridization probes. Biotechnology. 6:1197-1202.

Lonberg, N., and D. Huszar. 1995. Human antibodies from transgenic mice. Int Rev Immunol. 13:65-93.
Lonberg, N., L.D. Taylor, F.A. Harding, M. Trounstine, et al. 1994. Antigen-specific human antibodies from mice comprising four distinct genetic modifications [see comments]. Nature. 368:856-9.
Lopata, M.A., D.W. Cleveland, and B. Sollner-Webb. 1984. High-level expression of a chloramphenicol acetyltransferase gene by DEAEdextran-mediated DNA traansfection couled with a dimethylsulfoxide or glycerol shock treatment. Nucleic Acids Research. 12:5707.
Luckow, V.A. 1991. Cloning and expression of heterologous genes in insect cells with baculovirus vectors. In Recombinant DNA technology and applications. A. Prokop, R.K. Bajpai, and C. Ho, editors. McGraw-Hill, New York. 97-152.
Madura, K., R.J. Dohmen, and A. Varshavsky. 1993. N-recognin/Ubc2 interactions in the N-end rule pathway. J Biol Chem. 268:12046-54.
Maher, L.J. 1992. DNA triple-helix formation: an approach to artificial gene repressors? Bioessays. 14:807-15.
Mandel, M., and A. Higa. 1970. Calcium-dependent bacteriophage DNA infection. J. Mol. biol. 53:159-162.
Marasco, W.A., W.A. Haseltine, and S.Y. Chen. 1993. Design, intracellular expression, and activity of a human anti-human immunodeficiency virus type 1 gp120 single-chain antibody. Proc Natl Acad Sci U S A. 90:7889-93.
Marks, J.D., A.D. Griffiths, M. Malmqvist, T.P. Clackson, et al. 1992. By-passing immunization: building high affinity human antibodies by chain shuffling. Biotechnology (N Y). 10:779-83.
Marks, J.D., H.R. Hoogenboom, T.P. Bonnert, J. McCafferty, et al. 1991. By-passing immunization. Human antibodies from V-gene libraries displayed on phage. J Mol Biol. 222:581-97.
Martin, F.J., and D. Papahadjopoulos. 1982. Irreversible coupling of immunoglobulin fragments to preformed vesicles. An improved method for liposome targeting. J Biol Chem. 257:286-8.
Maxam, A.M., and W. Gilbert. 1977. A new method for sequencing DNA. Proc Natl Acad Sci U S A. 74:560-4.
Miller, A.D., and C. Buttimore. 1986. Redesign of retrovirus packaging cell lines to avoid recombination leading to helper virus production. Mol. Cell biol. 6:2895-2902.
Miller, L.K. 1988. Baculoviruses as gene expression vectors. Annu. Rev. Microbiol. 42:177-199.
Milstein, C., and A.C. Cuello. 1983. Hybrid hybridomas and their use in immunohistochemistry. Nature. 305:537-40.
US Patent No. 5,459,039. Methods for mapping genetic mutations. 1995.
Morrison, S.L., L. Wims, S. Wallick, L. Tan, et al. 1987. Genetically engineered antibody molecules and their application. Ann NY Acad Sci. 507:187-98.
US Patent No. 4,683,202. Process for amplifying nucleic acid sequences. 1987.
US Patent No. 4,683,195. Process for amplifying, detecting, and/or cloning nucleic acid sequences. 1987.
Munson, P.J., and D. Rodbard. 1980. Ligand: a versatile computerized approach for characterization of ligand-binding systems. Anal Biochem. 107:220-39.
Myers, R.M., Z. Larin, and T. Maniatis. 1985. Detection of single base substitutions by ribonuclease cleavage at mismatches in RNA:DNA duplexes. Science. 230:1242-6.
US Patent No. 5,328,470. Treatment of diseases by site-specific instillation of cells or site-specific transformation of cells and kits therefor. 1994.
Naeve, C.W., G.A. Buck, R.L. Niece, R.T. Pon, et al. 1995. Accuracy of automated DNA sequencing: a multi-laboratory comparison of sequencing results. Biotechniques. 19:448-53.
Nakai, K., and P. Horton. 1999. PSORT: a program for detecting sorting signals in proteins and predicting their subcellular localization. Trends Biochem Sci. 24:34-6.
Nakazawa, H., D. English, P.L. Randell, K. Nakazawa, et al. 1994. UV and skin cancer: specific p53 gene mutation in normal skin as a biologically relevant exposure measurement. Proc Natl Acad Sci U S A. 91:360-4.
Neumann, E., M. Schaefer-Ridder, Y. Wang, and P.H. Hofschneider. 1982. Gene transfer into mouse lyoma cells by electroporation in high electric fields. EMBO J. 1:841-845:
O'Gorman, S., D.T. Fox, and G.M. Wahl. 1991. Recombinase-mediated gene activation and site-specific integration in mammalian cells. Science. 251:1351-5.
Okano, H., J. Aruga, T. Nakagawa, C. Shiota, et al. 1991. Myelin basic protein gene and the function of antisense RNA in its repression in myelin-deficient mutant mouse. J Neurochem. 56:560-7.
O'Reilly, D.R., L.K. Miller, and V.A. Luckow. 1992. Baculovirus expression vectors. W.H. Freeman and Company, New York.
Orita, M., H. Iwahana, H. Kanazawa, K. Hayashi, et al. 1989. Detection of polymorphisms of human DNA by gel electrophoresis as single-strand conformation polymorphisms. Proc Natl Acad Sci U S A. 86:2766-70.
Ou-Lee, T.M., R. Turgeon, and R. Wu. 1986. Uptake and expression of a foreign gene linked to either a plant virus or Drosophila promoter in protoplasts of rice, wheat and sorghum. Proc. Natl. Acad. Sci. USA. 83:6815-6819.
Palmer, T.D., R.A. Hock, W.R.A. osborne, and A.D. Miller. 1987. Efficient retrovirus-mediated transfer and expression of a human adenosine deaminase gene in diploid skin fibroblasts from an adenosie-deficient human. Proc. Natl. Acad. Sci. USA. 84:1055-1059.

Pear, W., G. Nolan, M. Scott, and D. Baltimore. 1993. Production of high-titer helper-free retroviruses by transient transfection. Proc. Natl. Acad. Sci. USA. 90:8392-8396.
Pennica, D., T.A. Swanson, J.W. Welsh, M.A. Roy, et al. 1998. WISP genes are members of the connective tissue growth factor family that are up-regulated in wnt-1-transformed cells and aberrantly expressed in human colon tumors. Proc Natl Acad Sci U S A. 95:14717-22.
Perry-O'Keefe, H., X.W. Yao, J.M. Coull, M. Fuchs, et al. 1996. Peptide nucleic acid pre-gel hybridization: an alternative to southern hybridization. Proc Natl Acad Sci USA. 93:14670-5..
Petersen, K.H., D.K. Jensen, M. Egholm, O. Buchardt, et al. 1976. A PNA-DNA linker synthesis of N-((4,4'-dimethoxytrityloxy)ehtyl)-N-(thymin-1-ylacetyl)glycine. Biorganic and Medicianl Chemistry Letters. 5:1119-1124.
Potter, H. 1988. Electroporation in biology: Methods, applications,, and instrumentation. Analytical Biochemistry. 174:361-373.
Potter, H., L. Weir, and P. Leder. 1984. Enhancer-dependent expression of human kappa immunoglobulin genes introduced into mouse pre-B lymphocytes by electroporation. Proc. Natl. Acad. Sci. USA. 81:7161-7165.
Presta, L.G. 1992. Antibody engineering. Curr Opin Biotechnol. 3:394-8.
Prosser, J. 1993. Detecting single-base mutations. Trends Biotechnol. 11:238-46.
Rassoulzadegan, M., B. Binetruy, and F. Cuzin. 1982. High frequency of gene transfer after fusion between bacteria and eukaryotic cells. Nature. 295:257.
Reisfeld, R.A., and S. Sell. 1985. Monoclonal antibodies and cancer therapy: Proceedings of the Roche-UCLA symposium held in Park City, Utah, January 26-February 2, 1985. Alan R. Liss, New York. 609 pp.
Rhodes, C.A., D.A. Pierce, I.J. Mettler, D. Mascarenhas, et al. 1988. Genetically transformed maize plants from protoplasts. Science. 240:204-207.
Riechmann, L., M. Clark, H. Waldmann, and G. Winter. 1988. Reshaping human antibodies for therapy. Nature. 332:323-7.
Rose, J.K., L. Buonocore, and M. Whitt. 1991. A new cationic liposome reagent mediating nearly quantitative transfection of animal cells. BioTechniques. 10:520-525.
Rossi, J.J. 1994. Practical ribozymes. Making ribozymes work in cells. Curr Biol. 4:469-71.
Rossiter, B.J., and C.T. Caskey. 1990. Molecular scanning methods of mutation detection. J Biol Chem. 265:12753-6.
US Patent No. 5,283,317. Intermediates for conjugation of polypeptides with high molecular weight polyalkylene glycols. 1994.
Saiki, R.K., T.L. Bugawan, G.T. Horn, K.B. Mullis, et al. 1986. Analysis of enzymatically amplified beta-globin and HLA-DQ alpha DNA with allele-specific oligonucleotide probes. Nature. 324:163-6.
Saiki, R.K., P.S. Walsh, C.H. Levenson, and H.A. Erlich. 1989. Genetic analysis of amplified DNA with immobilized sequence-specific oligonucleotide probes. Proc Natl Acad Sci USA. 86:6230-4.
Saleeba, J.A., and R.G. Cotton. 1993. Chemical cleavage of mismatch to detect mutations. Methods Enzymol. 217: 286-95.
Sambrook, J. 1989. Molecular cloning: a laboratory manual. Cold Spring Harbor Laboratory, Cold Spring Harbor.
Sandri-Goldin, R.M., A.L. Goldin, J.C. Glorioso, and M. Levine. 1981. High-frequency transfer of cloned herpes simjplex virus type I sequences to mammalian cells by protoplast fusion. Mol. Cell. Biol. 1:7453-752.
Sanger, F., S. Nicklen, and A.R. Coulson. 1977. DNA sequencing with chain-terminating inhibitors. Proc Natl Acad Sci U S A. 74:5463-7.
Saunders, J.A., B.F. Matthews, and P.D. Miller. 1989. Plant gene transfer using electrofusion and electroporation. In Electroporation and electrofusion in cell biology. E. Neumann, A.E. Sowers, and C.A. Jordan, editors. Plenum Press, New York. 343-354.
Schade, R., C. Staak, C. Hendriksen, M. Erhard, et al. 1996. The production of avian (egg yold) antibodies: IgY. The report and recommendations of ECVAM workshop. Alternatives to Laboratory Animals (ATLA). 24:925-934.
Schaffner, W. 1980. Direct transfer of cloned genes from bacteria to mammalian cells. Proc. Natl. Acad. Sci. USA. 77:2163.
Schook, L.B. 1987. Monoclonal antibody production techniques and applications. Marcel Dekker, Inc., New York. 336 pp.
Scott, J.K., and G.P. Smith. 1990. Searching for peptide ligands with an epitope library. Science. 249:386-90.
Selden, R.F., K. Burke-Howie, M.E. Rowe, H.M. Goodman, et al. 1986. Human growth hormone as a reporter gene in regulation studies employing transient gene expression. Molecular and Cellular Biololgy. 6:3173-3179.
Shalaby, M.R., H.M. Shepard, L. Presta, M.L. Rodrigues, et al. 1992. Development of humanized bispecific antibodies reactive with cytotoxic lymphocytes and tumor cells overexpressing the HER2 protooncogene. J Exp Med. 175: 217-25.
Shigekawa, K., and W.J. Dower. 1988. Electroporation of eukaryotes and prokaryotes: A general approach to the introduction of macomolecules into cells. BioTechniques. 6:742-751.
Shillito, R. 1999. Methods of genetic transformations: Electroporation and polyethylene glycol treatment. In Molecular improvement of cereal crop. I. Vasil, editor. Kluwer, Dordrecht, The Netherlands. 9-20.
Shilo, B.Z., and R.A. Weinberg. 1981. DNA sequences homologous to vertebrate oncogenes are conserved in Drosophila melanogaster. Proc Natl Acad Sci U S A. 78:6789-92.
Shopes, B. 1992. A genetically engineered human IgG mutant with enhanced cytolytic activity. J Immunol. 148: 2918-22.
Simonsen, C.C., and A.D. Levinson. 1983. Isolation and expression of an altered mouse dihydrofolate reductase cDNA. Proc. Natl. Acad. Sci. USA. 80:2495-2499.
US Patent No. 5,272,057. Method of detecting a predisposition to cancer by the use of restriction fragment length polymorphism of the gene for human poly (ADP-ribose) polymerase. 1993.
Southern, P.J., and P. Berg. 1982. Transformation of mammalian cells to antibiotic resistanced with a bacterial gene under control of the SV40 early region promoter. J. Mol. Appl. Gen. 1:327-341.
Sreekrishna, K., R.H. Potenz, J.A. Cruze, W.R. McCombie, et al. 1988. High level expression of heterologous proteins in methylotrophic yeast Pichia pastoris. J Basic Microbiol. 28:265-78.
Stein, C.A., and J.S. Cohen. 1988. Oligodeoxynucleotides as inhibitors of gene expression: a review. Cancer Res. 48:2659-68.
Stevenson, G.T., A. Pindar, and C.J. Slade. 1989. A chimeric antibody with dual Fc regions (bisFabFc) prepared by manipulations at the IgG hinge. Anticancer Drug Des. 3:219-30.
Suresh, M.R., A.C. Cuello, and C. Milstein. 1986. Bispecific monoclonal antibodies from hybrid hybridomas. Methods Enzymol. 121:210-28.
Thomas, K.R., and M.R. Capecchi. 1987. Site-directed mutagenesis by gene targeting in mouse embryo-derived stem cells. Cell. 51:503-12.
Thompson, J.A., and e. al. 1995. Maize transformation utilizing silicon carbide whiskers: A review. Euphytica. 85: 75-80.
Tilburn, J., C. Scazzocchio, G.G. Taylor, J.H. Zabicky-Zissman, et al. 1983. Transformation by integration in Aspergillus nidulans. Gene. 26:205-21.
Touraev, A., and e. al. 1997. Plant male germ line transformation. Plant J. 12:949-956.
Traunecker, A., F. Oliveri, and K. Karjalainen. 1991. Myeloma based expression system for production of large mammalian proteins. Trends Biotechnol. 9:109-13.
Trick, H.N., and e. al. 1997. Recent advances in soybean transformation. Plant Tissue Cult. Biotechnol. 3:9-26.
Tuerk, C., and L. Gold. 1990. Systematic evolution of ligands by exponential enrichment: RNA ligands to bacteriophage T4 DNA polymerase. Science. 249:505-10.
Turner, D.L., E.Y. Snyder, and C.L. Cepko. 1990. Lineage-independent determinationh of cell type in the embryonic mouse retina. Neuron. 4:833-845.
Tutt, A., G.T. Stevenson, and M.J. Glennie. 1991. Trispecific F(ab')3 derivatives that use cooperative signaling via the TCR/CD3 complex and CD2 to activate and redirect resting cytotoxic T cells. J Immunol. 147:60-9.
van der Krol, A.R., J.N. Mol, and A.R. Stuitje. 1988b. Modulation of eukaryotic gene expression by complementary RNA or DNA sequences. Biotechniques. 6:958-76.
van der Krol, A.R., J.N. Mol, and A.R. Stuitje. 1988a. Modulation of eukaryotic gene expression by complementary RNA or DNA sequences. Biotechniques. 6:958-76.
Verhoeyen, M., C. Milstein, and G. Winter. 1988. Reshaping human antibodies: grafting an antilysozyme activity. Science. 239:1534-6.
Vitetta, E.S., R.J. Fulton, R.D. May, M. Till, et al. 1987. Redesigning nature's poisons to create anti-tumor reagents. Science. 238:1098-104.
US Patent No. 4,873,191. Genetic transformation of zygotes. 1989.
Wells, J.A., M. Vasser, and D.B. Powers. 1985. Cassette mutagenesis: an efficient method for generation of multiple mutations at defined sites. Gene. 34:315-23.
Whitt, M.A., L. Buonocore, J.K. Rose, V. Ciccarone, et al. 1990. TransfectACE reagent promotes transient transfection frequencies greater than 90%. Focus. 13:8-12.
Wigler, M., A. Pellicer, S. Silversttein, and R. Axel. 1978. Biochemical transfer of single-copy eucaryotic genes using total cellular DNA as donor. Cell. 14:725.
Williams, D.A., I.R. Lemischka, D.G. Nathan, and R.C. Mulligan. 1984. Introduction of a new genetic material into pluripotent haematopoietic stem cells of the mouse. Nature. 310:476-480.
Wilmut, I., A.E. Schnieke, J. McWhir, A.J. Kind, et al. 1997. Viable offspring derived from fetal and adult mammalian cells. Nature. 385:810-3.
Wolff, E.A., G.J. Schreiber, W.L. Cosand, and H.V. Raff. 1993. Monoclonal antibody homodimers: enhanced antitumor activity in nude mice. Cancer Res. 53:2560-5.
Wong, G.T., B.J. Gavin, and A.P. McMahon. 1994. Differential transformation of mammary epithelial cells by Wnt genes. Mol Cell Biol. 14:6278-86.

Wong, T.K., and E. Neumann. 1982. Electric field mediated gene transfer. Biochemical and Biophysical Research Communications. 107:584-587.
Wyborski, D.L., L.C. DuCoeur, and J.M. Short. 1996. Parameters affecting the use of the lac repressor system in eukaryotic cells and transgenic animals. Environ Mol Mutagen. 28:447-58.
Wyborski, D.L., and J.M. Short. 1991. Analysis of inducers of the E.coli lac repressor system in mammalian cells and whole animals. Nucleic Acids Res. 19:4647-53.
Yelton, M.M., J.E. Hamer, and W.E. Timberlake. 1984. Transformation of Aspergillus nidulans by using a trpC plasmid. Proc Natl Acad Sci U S A. 81:1470-4.
Zervos, A.S., J. Gyuris, and R. Brent. 1993. Mxi1, a protein that specifically interacts with Max to bind Myc-Max recognition sites. Cell. 72:223-32.
Zhou, G., and e. al. 1983. Introduction of exogenous DNA into cotton embryos. Methods Enzymol. 101:433-481.
Zoller, M.J., and M. Smith. 1987. Oligonucleotide-directed mutagenesis: a simple method using two oligonucleotide primers and a single-stranded DNA template. Methods Enzymol. 154:329-50.
Zon, G. 1988. Oligonucleotide analogues as potential chemotherapeutic agents. Pharm Res. 5:539-49.
Zuckermann, R.N., E.J. Martin, D.C. Spellmeyer, G.B. Stauber, et al. 1994. Discovery of nanomolar ligands for 7-transmembrane G-protein-coupled receptors from a diverse N-(substituted)glycine peptoid library. J Med Chem. 37:2678-85.

## Claims

1. An isolated polypeptide comprising an amino acid sequence having at least 80% sequence identity to the sequence of SEQ ID NOS: 6 or 8, wherein said polypeptide is an active WUP (Wnt1 UPregulated) polypeptide.

2. The polypeptide of Claim 1, wherein said amino acid sequence has at least 90% sequence identity to the sequence of SEQ ID NOS: 6 or 8.

3. The polypeptide of Claim 1, wherein said amino acid sequence has at least 98% sequence identity to the sequence of SEQ ID NOS: 6 or 8.

4. An isolated polypeptide consisting of an amino acid sequence of Table 6 (SEQ ID NO: 39), which corresponds to amino acids 51-126 of SEQ ID NO: 6.

5. An isolated chimeric polypeptide comprising a polypeptide having an amino acid sequence that has at least 80% sequence identity to the sequence of SEQ ID NOS: 6 or 8, fused to a non-WUP polypeptide selected from the group consisting of glutathione-S-transferase, immunoglobulin protein or fragments thereof, human growth hormone, β-glucuronidase, green fluorescent protein, luciferase, chloramphenicol acetyl transferase, β-galactosidase, and secrete alkaline phosphatase.

6. An isolated polynucleotide encoding the polypeptide of any of Claims 1-5, or a complement of said polynucleotide.

7. A polynucleotide comprising a nucleotide sequence having at least 90% sequence identity to the sequence of SEQ ID NO: 5 or at least 80% sequence identity to the sequence of SEQ ID NO: 7, or a complement of said polynucleotide.

8. The polynucleotide of Claim 7, wherein said nucleotide sequence has at least 98% sequence identity to the sequence of SEQ ID NOS: 5 or 7, or a complement of said polynucleotide.

9. An antibody that specifically binds to the polypeptide of any one of Claims 1-5.

10. A method for determining whether a compound up-regulates or down-regulates the transcription of a gene comprising SEQ ID NOs 5 or 7, comprising: contacting said compound with a composition comprising a RNA polymerase and said gene and measuring the amount of polynucleotide corresponding to a mRNA of SEQ ID NOs: 5 or 7 and measuring the amount of gene transcription.

11. A method for determining whether a compound up-regulates or down-regulates the translation of SEQ ID NOs: 5 or 7, comprising: contacting said compound with a composition comprising a ribosome and a polynucleotide corresponding to a mRNA of SEQ ID NOs: 5 or 7 and measuring the amount of gene translation.

12. The method of Claim 10 or 11, wherein said composition is in a cell.

13. A vector, comprising any one of the polynucleotides of Claims 6-8.

14. A cell comprising the vector of Claim 13.

15. A method of screening a tissue sample for tumorigenic potential, comprising: measuring expression of a polynucleotide comprising SEQ ID NOs: 5 or 7 in said tissue sample.

16. The method of Claim 15, wherein said measuring is measuring an amount of mRNA encoding a polypeptide comprising an amino acid sequence of SEQ ID NO: 6 or 8.

17. A transgenic non-human animal, having at least one disrupted WUP gene comprising SEQ ID NOs: 5 or 7.

18. The transgenic non-human animal of Claim 17, wherein the non-human animal is a mouse.

19. A transgenic non-human animal, comprising an exogenous polynucleotide having at least 80% sequence identity to the sequence of SEQ ID NOS: 5 or 7, or a complement of said polynucleotide.

20. The transgenic non-human animal of Claim 19, wherein said exogenous polynucleotide has at least 90% sequence identity to the sequence of SEQ ID NOS: 5 or 7, or a complement of said polynucleotide, preferably at least 98% sequence identity to the sequence of SEQ ID NOS: 5 or 7, or a complement of said polynucleotide.

21. An *in vitro* method of screening a sample for a WUP gene mutation, comprising: detecting expression of a WUP polynucleotide sequence of any of Claims 6-8; and comparing a WUP polynucleotide sequence in the sample with a polynucleotide sequence of SEQ ID NOS: 5 or 7, to identify a WUP gene mutation.

22. A method of determining the clinical stage of tumour comprising comparing expression of a WUP polynucleotide of any of Claims 6-8 in a sample with expression of SEQ ID NOs: 5 or 7 in control samples.

**23.** A method of detecting a Wnt-1 up-regulated polypeptide in a biological sample comprising:

a) detecting expression of the polynucleotide of any of Claims 6-8 in a biological sample; and
b) comparing expression of the polynucleotide to that of a control sample to determine whether expression of the polynucleotide is up-regulated.

**24.** The method of any of Claims 21-23 wherein expression of the polynucleotide of any of Claims 6-8 is detected by a nucleic acid probe that binds to the polynucleotide of any of Claims 6-8 under stringent conditions.

**25.** The method of any of Claims 21-23, wherein expression of the polynucleotide of any of Claims 6-8 is detected by at least one oligonucleotide that can amplify the polynucleotide of any of Claims 6-8.

**26.** The method of any of Claims 22-23, wherein expression of the polynucleotide of any of Claims 6-8 is detected by an antibody of Claim 9.

**27.** The method of Claim 26, wherein the antibody is detectably labelled.

**28.** The method of Claim 27, wherein the label is radioactive moiety or a fluorescent moiety.

**29.** The method of any of Claims 21-28, wherein the sample is from a tumour.

**30.** A kit for detecting up-regulation of a Wnt-1 up-regulated polypeptide comprising a polynucleotide according to any of Claims 6-8 or complement thereof or an antibody according to Claim 9, for detecting expression of the polynucleotide of any of Claims 6-8 in a biological sample.

**31.** The kit of Claim 30, wherein the polynucleotide or complement thereof can serve as a primer to amplify the polynucleotide of any of Claims 6-8.

**32.** The kit of Claim 30, wherein the antibody is detectably labelled.

**33.** The kit of Claim 32, wherein the antibody is labelled with radioactive or fluorescent moiety.

**34.** An isolated polypeptide according to Claim 1, comprising an amino acid sequence of SEQ ID NOS: 6 or 8.

**Patentansprüche**

**1.** Isoliertes Polypeptid, umfassend eine Aminosäuresequenz mit mindestens 80 % Sequenzidentität zur Sequenz SEQ ID Nr. 6 oder 8, wobei das Polypeptid ein aktives WUP- (Wnt1 nach oben reguliertes)-Polypeptid ist.

**2.** Polypeptid nach Anspruch 1, wobei die Aminosäuresequenz mindestens 90 % Sequenzidentität zur Sequenz SEQ ID Nr. 6 oder 8 aufweist.

**3.** Polypeptid nach Anspruch 1, wobei die Aminosäuresequenz mindestens 98 % Sequenzidentität zur Sequenz SEQ ID Nr. 6 oder 8 aufweist.

**4.** Isoliertes Polypeptid, bestehend aus einer Aminosäuresequenz von Tabelle 6 (SEQ ID Nr. 39), welche den Aminosäuren 51 bis 126 von SEQ ID Nr. 6 entspricht.

**5.** Isoliertes chimäres Polypeptid, umfassend ein Polypeptid mit einer Aminosäuresequenz, welches mindestens 80 % Sequenzidentität zur Sequenz SEQ ID Nr. 6 oder 8 aufweist, fusioniert an ein Nicht-WUP-Polypeptid, ausgewählt aus der Gruppe, welche aus Glutathion-S-transferase, Immunglobulinprotein oder Fragmenten davon, humanem Wachstumshormon, β-Glucuronidase, grünem fluoreszenztem Protein, Luziferase, Chloramphenicolacetyltransferase, β-Galactosidase und sekretierter alkalischer Phosphatase besteht.

**6.** Isoliertes Polynukleotid, kodierend das Polypeptid nach einem der Ansprüche 1 bis 5, oder ein Komplement des Polynukleotid.

**7.** Polynukleotid, umfassend eine Nukleotidsequenz mit mindestens 90 % Sequenzidentität zur Sequenz SEQ ID Nr. 5 oder mindestens 80 % Sequenzidentität zur Sequenz SEQ ID Nr. 7, oder ein Komplement des Polynukleotids.

**8.** Polynukleotid nach Anspruch 7, wobei die Nukleotidsequenz mindestens 98 % Sequenzidentität zur Sequenz SEQ ID Nr. 5 oder 7 aufweist, oder ein Komplement des Polynukleotids.

**9.** Antikörper, der spezifisch an das Polypeptid nach einem der Ansprüche 1 bis 5 bindet.

**10.** Verfahren zur Bestimmung, ob eine Verbindung die Transkription eines Gens, welches SEQ ID Nr. 5 oder 7 umfasst, nach oben reguliert oder nach unten reguliert, umfassend: das Inkontaktbringen der Verbindung mit einer Zusammensetzung, welche eine RNA-Polymerase und das Gen umfasst, und das Messen der Menge des Polynukleotids, welches einer mRNA von SEQ ID Nr. 5 oder 7 entspricht, und das Messen des Umfangs der Gentranskription.

**11.** Verfahren zur Bestimmung, ob eine Verbindung die Translation von SEQ ID Nr. 5 oder 7 nach oben reguliert oder nach unten reguliert, umfassend: das Inkontaktbringen der Verbindung mit einer Zusammensetzung, umfassend ein Ribosom und ein Polynukleotid, welches einer mRNA von SEQ ID Nr. 5 oder 7 entspricht, und das Messen des Umfangs der Gentranslation.

**12.** Verfahren nach Anspruch 10 oder 11, wobei die Zusammensetzung in einer Zelle ist.

**13.** Vektor, umfassend eines der Polynukleotide nach den Ansprüchen 6 bis 8.

**14.** Zelle, umfassend den Vektor nach Anspruch 13.

**15.** Verfahren zum Screenen einer Gewebeprobe auf ein die Tumorgenese förderndes Potential, umfassend: das Messen der Expression eines Polynukleotids, umfassend SEQ ID Nr. 5 oder 7, in der Gewebeprobe.

**16.** Verfahren nach Anspruch 15, wobei das Messen das Messen einer Menge von mRNA, welche ein Polypeptid kodiert, das eine Aminosäuresequenz SEQ ID Nr. 6 oder 8 umfasst, ist.

**17.** Transgenes nicht-humanes Tier mit mindestens einem unterbrochenen WUP-Gen, umfassend SEQ ID Nr. 5 oder 7.

**18.** Transgenes nicht-humanes Tier nach Anspruch 17, wobei das nicht-humane Tier eine Maus ist.

**19.** Transgenes nicht-humanes Tier, umfassend ein exogenes Polynukleotid mit mindestens 80 % Sequenzidentität zur Sequenz SEQ ID Nr. 5 oder 7 oder ein Komplement des Polynukleotids.

**20.** Transgenes nicht-humanes Tier nach Anspruch 19, wobei das exogene Polynukleotid mindestens 90 % Sequenzidentität zur Sequenz SEQ ID Nr. 5 oder 7 aufweist, oder ein Komplement des Polynukleotids, bevorzugt mindestens 98 % Sequenzidentität zur Sequenz SEQ ID Nr. 5 oder 7 aufweist, oder ein Komplement des Polynukleotids.

**21.** *In vitro*-Verfahren zum Screenen einer Probe auf eine WUP-Genmutation, umfassend:

das Nachweisen der Expression einer WUP-Polynukleotidsequenz nach einem der Ansprüche 6 bis 8; und das Vergleichen einer WUP-Polynukleotidsequenz in der Probe mit einer Polynukleotidsequenz SEQ ID Nr. 5 oder 7, um eine WUP-Genmutation zu identifizieren.

**22.** Verfahren zum Bestimmen des klinischen Tumorstadiums, umfassend das Vergleichen der Expression eines WUP-Polynukleotids nach einem der Ansprüche 6 bis 8 in einer Probe mit der Expression von SEQ ID Nr. 5 oder 7 in Kontrollproben.

**23.** Verfahren zum Nachweisen eines Wnt-1 nach oben regulierten Polypeptids in einer biologischen Probe, umfassend:

a) das Nachweisen der Expression des Polynukleotids nach einem der Ansprüche 6 bis 8 in einer biologischen Probe; und
b) das Vergleichen der Expression des Polynukleotids mit der einer Kontrollprobe, um zu bestimmen, ob die Expression des Polynukleotids nach oben reguliert ist.

**24.** Verfahren nach einem der Ansprüche 21 bis 23, wobei die Expression des Polynukleotids nach einem der Ansprüche 6 bis 8 durch eine Nukleinsäuresonde, welche an das Polynukleotid nach einem der Ansprüche 6 bis 8 unter stringenten Bedingungen bindet, nachgewiesen wird.

**25.** Verfahren nach einem der Ansprüche 21 bis 23, wobei die Expression des Polynukleotids nach einem der Ansprüche 6 bis 8 durch mindestens ein Oligonukleotid, welches das Polynukleotid nach einem der Ansprüche 6 bis 8 amplifizieren kann, nachgewiesen wird.

**26.** Verfahren nach einem der Ansprüche 22 bis 23, wobei die Expression des Polynukleotids nach einem der Ansprüche 6 bis 8 durch einen Antikörper nach Anspruch 9 nachgewiesen wird.

**27.** Verfahren nach Anspruch 26, wobei der Antikörper nachweisbar markiert ist.

**28.** Verfahren nach Anspruch 27, wobei die Markierung eine radioaktive Einheit oder eine fluoreszente Einheit ist.

**29.** Verfahren nach einem der Ansprüche 21 bis 28, wobei die Probe von einem Tumor stammt.

**30.** Kit zum Nachweisen der Regulation nach oben von einem Wnt-1 nach oben regulierten Polypeptid, umfassend ein Polynukleotid gemäß einem der Ansprüche 6 bis 8 oder ein Komplement davon oder einen Antikörper gemäß Anspruch 9, zum Nachweisen der Expression des Polynukleotids nach einem der Ansprüche 6 bis 8 in einer biologischen Probe.

**31.** Kit nach Anspruch 30, wobei das Polynukleotid oder ein Komplement davon als ein Primer zum Amplifizieren des Polynukleotids nach einem der Ansprüche 6 bis 8 dienen kann.

**32.** Kit nach Anspruch 30, wobei der Antikörper nachweisbar markiert ist.

**33.** Kit nach Anspruch 32, wobei der Antikörper mit einer radioaktiven oder fluoreszenten Einheit markiert ist.

**34.** Isoliertes Polypeptid gemäß Anspruch 1, umfassend eine Aminosäuresequenz SEQ ID Nr. 6 oder 8.

**Revendications**

**1.** Polypeptide isolé comprenant une séquence d'acides aminés présentant une identité de séquence d'au moins 80 % à la séquence de SEQ ID No: 6 ou 8, dans lequel ledit polypeptide est un polypeptide WUP (Wnt1 UPregulated - régulé positivement par Wnt-1) actif.

**2.** Polypeptide selon la revendication 1, dans lequel ladite séquence d'acides aminés présente une identité de séquence d'au moins 90 % à la séquence de SEQ ID No: 6 ou 8.

**3.** Polypeptide selon la revendication 1, dans lequel ladite séquence d'acides aminés présente une identité de séquence d'au moins 98 % à la séquence de SEQ ID No: 6 ou 8.

**4.** Polypeptide isolé consistant en une séquence d'acides aminés du Tableau 6 (SEQ ID No.: 39), qui correspond aux acides aminés 51-126 de SEQ ID No.: 6.

**5.** Polypeptide chimérique isolé comprenant un polypeptide ayant une séquence d'acides aminés qui présente une identité de séquence d' au moins 80 % à la séquence de SEQ ID No: 6 ou 8, fusionné à un polypeptide non-WUP choisi dans le groupe constitué de la glutathione-S-transférase, d'une protéine de type immunoglobuline ou fragments de celle-ci, de l'hormone de croissance humaine, de la β-glucuronidase, de la protéine fluorescente verte, de la luciférase, de la chloramphénicol acétyltransférase, de la β-galactosidase, et de la phosphatase alcaline sécrétée.

**6.** Polynucléotide isolé codant pour le polypeptide selon l'une quelconque des revendications 1 à 5, ou complément dudit polynucléotide.

**7.** Polynucléotide comprenant une séquence de nucléotides présentant une identité de séquence d'au moins 90 % à la séquence de SEQ ID No: 5 ou une identité de séquence d'au moins 80 % à la séquence de SEQ ID No.: 7, ou complément dudit polynucléotide.

**8.** Polynucléotide selon la revendication 7, dans lequel ladite séquence de nucléotides présente une identité de séquence d'au moins 98 % à la séquence de SEQ ID No: 5 ou 7, ou complément dudit polynucléotide.

**9.** Anticorps qui se lie spécifiquement au polypeptide selon l'une quelconque des revendications 1-5.

**10.** Procédé permettant de déterminer si un composé régule positivement ou négativement la transcription d'un gène comprenant SEQ ID No.: 5 ou 7, comprenant : la mise en contact dudit composé avec une composition comprenant une ARN polymérase et ledit gène et la mesure de la quantité de polynucléotide correspondant à un ARNm de SEQ ID No.: 5 ou 7 et la mesure de la quantité de transcription du gène.

**11.** Procédé permettant de déterminer si un composé régule positivement ou négativement la traduction de SEQ ID No.: 5 ou 7, comprenant : la mise en contact dudit composé avec une composition comprenant un ribosome et un polynucléotide correspondant à un ARNm de SEQ ID No.: 5 ou 7 et la mesure de la quantité de traduction du gène.

**12.** Procédé selon la revendication 10 ou 11, dans lequel ladite composition est dans une cellule.

**13.** Vecteur, comprenant l'un quelconque des polynucléotides selon les revendications 6 à 8.

**14.** Cellule comprenant le vecteur selon la revendication 13.

**15.** Procédé de criblage d'un échantillon tissulaire pour déterminer son potentiel tumorigène, comprenant : la mesure de l'expression d'un polynucléotide comprenant SEQ ID No.: 5 ou 7 dans ledit échantillon tissulaire.

**16.** Procédé selon la revendication 15, dans lequel ladite mesure mesure une quantité d'ARNm codant pour un polypeptide comprenant une séquence d'acides aminés de SEQ ID No: 6 ou 8.

**17.** Animal transgénique non-humain, portant au moins un gène WUP rompu comprenant SEQ ID No.: 5 ou 7.

**18.** Animal transgénique non-humain selon la revendication 17, dans lequel l'animal non-humain est une souris.

**19.** Animal transgénique non-humain, comprenant un polynucléotide exogène présentant une identité de séquence d'au moins 80 % à la séquence de SEQ ID No.: 5 ou 7, ou un complément dudit polynucléotide.

**20.** Animal transgénique non-humain selon la revendication 19, dans lequel ledit polynucléotide exogène présente une identité de séquence d'au moins 90 % à la séquence de SEQ ID No.: 5 ou 7, ou complément dudit polynucléotide, de préférence, une identité de séquence d'au moins 98 % à la séquence de SEQ ID No.: 5 ou 7, ou complément dudit polynucléotide.

**21.** Procédé de criblage *in vitro* d'un échantillon pour déterminer la mutation d'un gène WUP, comprenant : la détection de l'expression d'une séquence polynucléotidique WUP selon l'une quelconque des revendications 6 à 8 ; et la comparaison d'une séquence polynucléotidique WUP dans l'échantillon avec une séquence polynucléotidique de SEQ ID No: 5 ou 7, pour identifier une mutation du gène WUP.

**22.** Procédé de détermination du stade clinique d'une tumeur comprenant la comparaison de l'expression d'un polynucléotide WUP selon l'une quelconque des revendications 6 à 8 dans un échantillon avec l'expression de SEQ ID No.: 5 ou 7 dans des échantillons témoins.

**23.** Procédé de détection d'un polypeptide régulé positivement par Wnt-1 dans un échantillon biologique comprenant :

a) la détection de l'expression du polynucléotide selon l'une quelconque des revendications 6 à 8 dans un échantillon biologique ; et
b) la comparaison de l'expression du polynucléotide à celle d'un échantillon témoin pour déterminer si l'expression du polynucléotide est régulée positivement ou non.

**24.** Procédé selon l'une quelconque des revendications 21 à 23, dans lequel l'expression du polynucléotide selon l'une quelconque des revendications 6 à 8 est détectée par une sonde d'acide nucléique qui se lie au polynucléotide selon l'une quelconque des revendications 6 à 8 dans des conditions stringentes.

**25.** Procédé selon l'une quelconque des revendications 21 à 23, dans lequel l'expression du polynucléotide selon l'une quelconque des revendications 6 à 8 est détectée par au moins un oligonucléotide qui peut amplifier le polynucléotide selon l'une quelconque des revendications 6 à 8.

**26.** Procédé selon l'une quelconque des revendications 22-23, dans lequel l'expression du polynucléotide selon l'une quelconque des revendications 6 à 8 est détectée par un anticorps selon la revendication 9.

**27.** Procédé selon la revendication 26, dans lequel l'anticorps est marqué de façon détectable.

**28.** Procédé selon la revendication 27, dans lequel le marqueur est un fragment radioactif ou un fragment fluorescent.

**29.** Procédé selon l'une quelconque des revendications 21 à 28, dans lequel l'échantillon provient d'une tumeur.

**30.** Trousse pour la détection de la régulation positive d'un polypeptide régulé positivement par Wnt-1 comprenant un polynucléotide selon l'une quelconque des revendications 6 à 8 ou son complément ou un anticorps selon la revendication 9, pour détecter l'expression du polynucléotide selon l'une quelconque des revendications 6 à 8 dans un échantillon biologique.

**31.** Trousse selon la revendication 30, dans laquelle le polynucléotide ou son complément peut servir d'amorce pour amplifier le polynucléotide selon l'une quelconque des revendications 6 à 8.

**32.** Trousse selon la revendication 30, dans laquelle l'anticorps est marqué de façon détectable.

**33.** Trousse selon la revendication 32, dans laquelle l'anticorps est marqué avec un fragment radioactif ou fluorescent.

**34.** Polypeptide selon la revendication 1, comprenant une séquence d'acides aminés de SEQ ID No.: 6 ou 8.

# FIGURE 1

# BLOCKS Protein Domain Analysis of 87769892

Probe Size: 76 Amino Acids

Probe File: lrastelliblocks.seq

Target File (s) : blocks.dat

Records Searched: 4034

Scores Done: 4034

Alignments Done: 422518

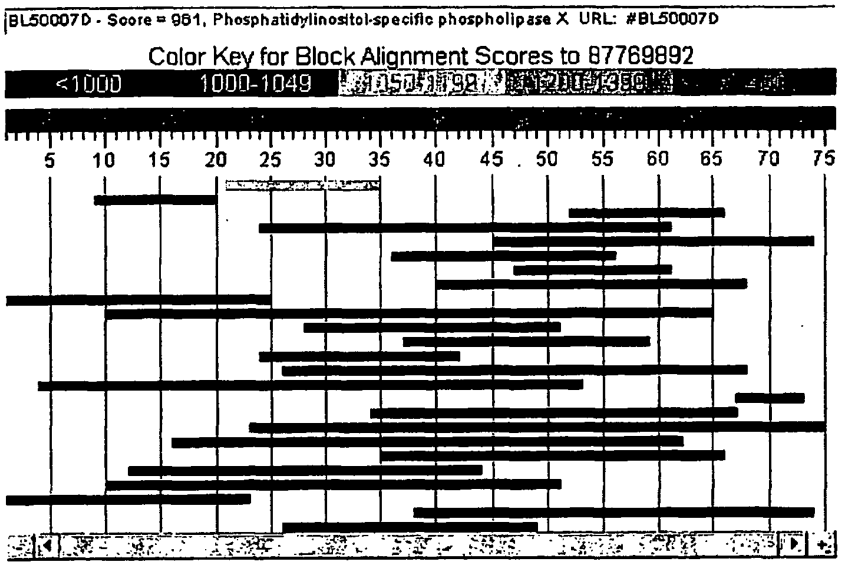


| AC# | | Description | Strength | Score | RF | AA# | |
|---|---|---|---|---|---|---|---|
| BL00134C | 0 | Serine proteases, trypsin family, histidine p | 1245 | 1064 | 0 | 21 | PYIYPLVSPFVSrI |
| BL00122C | 0 | Carboxylesterases type-B serine proteins. | 1354 | 1012 | 0 | 9 | PVLLOIYkkfL |
| BL00301C | 0 | GTP-binding elongation factors proteins. | 1090 | 1000 | 0 | 52 | KTKGADMNGLPTKG |
| BL00687C | 0 | Aldehyde dehydrogenases glutamic acid protein | 1266 | 984 | 0 | 24 | YPLVsPFVSrIwPkkAIQESNDTNkGKVNFkGaDmnG |
| BL00697A | 0 | ATP-dependent DNA ligase AMP-binding site pro | 1256 | 974 | 0 | 45 | DTnKgKVnFKgAdMnglPTKGPtEIcDKK |
| BL00087A | 0 | Copper/Zinc superoxide dismutase proteins. | 1196 | 970 | 0 | 36 | PKKAIqESNDTNKGKVNFKg |
| BL00595B | 0 | Aminotransferases class-V pyridoxal-phosphate | 1086 | 968 | 0 | 47 | KKGKVNFkGADMnG |
| BL01016E | 0 | Glycoprotease family proteins. | 1555 | 967 | 0 | 40 | IqESnDtnkGkVnfKgAdMnGLPtkGpt |
| BL00633B | 0 | Bromodomain proteins. | 1302 | 965 | 0 | 0 | mVciPcIViPVLLwIyKKfLEpYiY |
| BL00367C | 0 | Biopterin-dependent aromatic amino acid hydro | 1568 | 964 | 0 | 10 | vLIwIykkflEpyiypLVspfVsriwPKkAiQEsNDtnKgKVnFKGadmNgLPtk |
| BL00069G | 0 | Glucose-6-phosphate dehydrogenase proteins. | 1442 | 963 | 0 | 28 | SpFVsRIwPKkAIQeSnDTnKgk |
| BL01107C | 0 | Ribosomal protein L27e proteins. | 1684 | 963 | 0 | 37 | KKAiqEsndTnKgKvnFkgadm |
| BL00635B | 0 | Gram-negative pili assembly chaperone protein | 1216 | 961 | 0 | 24 | ypLVSPfVSRIwPKKaIQ |
| BL50007D | 0 | Phosphatidylinositol-specific phospholipase X | 1708 | 961 | 0 | 26 | lvspfvSRIwPKkaiqeSndtKkgkvnfkGadMngLptkgPt |
| BL00110F | 0 | Pyruvate kinase proteins. | 1870 | 960 | 0 | 4 | PCIVipvLLwIykKflcPyiyplvspFvsrIwpkkAIqeSNdTnKGKvn |
| BL00886B | 0 | Dihydroxy-acid and 6-phosphogluconate dehydra | 1148 | 957 | 0 | 67 | teICDK |
| BL00520A | 0 | Ribosomal protein S4e proteins. | 1644 | 956 | 0 | 34 | iwPKKaiqesNdtKkgKVnfKGAdmNglPtkGP |
| BL01292D | 0 | Uncharacterized protein family UPF0026 protei | 1658 | 956 | 0 | 23 | iyplVSpFvSrIwpkKAIqEsndtKKgKVnFkGadMnglptkGpTeicDKk |
| BL00325B | 0 | Actin-depolymerizing proteins. | 1583 | 954 | 0 | 16 | KkfLEpyIypLvSPfVSRIwpKkaiqeSnDTnKgKvNfkGaDmnGl |
| BL00992B | 0 | Serum amyloid A proteins. | 1851 | 953 | 0 | 35 | wpkKAiQEsNdtnkgkvNfkgadmnGLPtKg |
| BL01303E | 0 | BCCT family of transporters proteins. | 1619 | 953 | 0 | 12 | lwIykkflepyiyPlVSpFVsRIwpkkaIqEs |
| BL00359B | 0 | Ribosomal protein L11 proteins. | 1442 | 952 | 0 | 10 | VIlwIykKflepYiypLVSPfVSRIwpKkAiqESnDtKKGK |
| BL00115L | 0 | Eukaryotic RNA polymerase II heptapeptide rep | 1752 | 951 | 0 | -7 | mVciPCIviPvlLWiyKkfLepyIy |
| BL00049C | 0 | Ribosomal protein L14 proteins. | 1563 | 949 | 0 | 38 | kaIQeSnDTKkGKVNfKGaDMnGlptKGPteicDKK |
| BL00290A | 0 | Immunoglobulins and major histocompatibility | 1148 | 945 | 0 | 26 | LVSPFVSRIwPKKAIqESNDTKK |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1354950 A
- WO 02053737 A
- JP 2000402288 A
- WO 0055174 A
- AU 036392
- WO 9013641 A
- WO 9104753 A
- WO 9016448 A
- US 51161992 B, Cech
- US 4987071 A, Cech
- WO 9106629 A
- WO 9010448 A
- WO 8809810 A
- WO 8910134 A
- WO 9733551 A
- US 4166452 A
- US 4816567 A
- US 5545807 A
- US 5545806 A
- US 5569825 A
- US 5633425 A
- US 5661016 A
- US 5625126 A
- WO 9308829 A
- WO 9627011 A
- WO 9100360 A
- WO 9220373 A
- WO 4676980 A
- WO 9411026 A
- US 4485045 A
- US 4544545 A
- US 5013556 A
- US 3773919 A
- WO 9100357 A
- US 4870009 A, Evans
- US 0879693843 B, Hogan
- US 4736866 A, Leder and Stewart
- US 4873191 A, Wagner and Hoppe
- WO 9304169 A, Berns
- WO 9101140 A, Kucherlapati
- WO 9011354 A, Le Mouellic and Brullet
- US 4522811 A, Eppstein
- US 5328470 A, Nabel and Nabel
- US 5223409 A, Ladner
- WO 9410300 A, Brent
- US 5283317 A, Saifer
- US 5272057 A, Smulson
- US 4683202 A, Mullis
- US 4683195 A, Mullis
- WO 9416101 A, Koster
- US 5459039 A, Modrich
- EP 402226 A
- US 5116742 A
- US 0109600 W
- US 60191258 B


### Non-patent literature cited in the description


- **ABRAVAYA, K. ; J.J. CARRINO ; S. MULDOON ; H.H. LEE.** Detection of point mutations with a modified ligase chain reaction (Gap- LCR. *Nucleic Acids Res.,* 1995, vol. 23, 675-82
- **ALAM, J. ; J.L. COOK.** Reporter genes: Application to the study of mammalian gene transcription. *Anal. Biochem.,* 1990, vol. 188, 245-254
- **AUSTIN, C.P. ; C.L. CEPKO.** Cellular migration patterns in the developing mouse cerebral cortex. *Development,* 1990, vol. 110, 713-732
- **AUSUBEL, F.M. ; R. BRENT ; R.E. KINGSTON ; D.D. MOORE et al.** Current protocols in molecular biology. John Wiley & Sons, 1987
- **BARANY, F.** Genetic disease detection and DNA amplification using cloned thermostable ligase. *Proc Natl Acad Sci USA.,* 1991, vol. 88, 189-93
- **BARTEL, D.P. ; J.W. SZOSTAK.** Isolation of new ribozymes from a large pool of random sequences [see comment. *Science,* 1993, vol. 261, 1411-8
- **BARTEL, P. ; C.T. CHIEN ; R. STERNGLANZ ; S. FIELDS.** Elimination of false positives that arise in using the two-hybrid system. *Biotechniques,* 1993, vol. 14, 920-4
- **BEAL, P.A. ; P.B. DERVAN.** Second structural motif for recognition of DNA by oligonucleotide- directed triple-helix formation. *Science,* 1991, vol. 251, 1360-3
- **BECHTOLD, N. ; G. PELLETIER.** In planta Agrobacterium-mediated transformation of adult Arabidopsis thaliana plants by vacuum infiltration. *Methods Mol Biol.,* 1998, vol. 82, 259-66

- **BECKER, D.M. ; L. GUARENTE.** High-efficiency transformation of yeast by electroporation. *Methods Enzymol,* 1991, vol. 194, 182-187
- **BEGGS, J.D.** Transformation of yeast by a replicating hybrid plasmid. *Nature,* 1978, vol. 275, 104-109
- **BERGER, J. ; J. HAUBER ; R. HAUBER ; R. GEIGER et al.** Secreted placental alkaline phosphatase: A powerful new qunatitative indicator of gene expression in eukaryotic cells. *Gene,* 1988, vol. 66, 1-10
- **BODINE, D.M. ; K.T. MCDONAGH ; N.E. SEIDEL ; A.W. NIENHUIS.** Survival and retrovirus infection of murine hematopoietic stem cells in vitro: effects of 5-FU and method of infection. *Exp. Hematol.,* 1991, vol. 19, 206-212
- **BOERNER, P. ; R. LAFOND ; W.Z. LU ; P. BRAMS et al.** Production of antigen-specific human monoclonal antibodies from in vitro-primed human splenocytes. *J Immunol.,* 1991, vol. 147, 86-95
- **BRADLEY.** Teratocarcinomas and Embryonic Stem Cells: A Practical Approach. Oxford University Press, Inc, 1987, 268
- **BRADLEY, A.** Modifying the mammalian genome by gene targeting. *Curr Opin Biotechnol.,* 1991, vol. 2, 823-9
- **BRENNAN, M. ; P.F. DAVISON ; H. PAULUS.** Preparation of bispecific antibodies by chemical recombination of monoclonal immunoglobulin G1 fragments. *Science,* 1985, vol. 229, 81-3
- **BROWN, A.M. ; R.S. WILDIN ; T.J. PRENDERGAST ; H.E. VARMUS.** A retrovirus vector expressing the putative mammary oncogene int-1 causes partial transformation of a mammary epithelial cell line. *Cell,* 1986, vol. 46, 1001-9
- **CAPECCHI, M.R.** High efficiency transformation by direct microinjection of DNA into cultured mammalian cells. *Cell,* 1980, vol. 22, 479
- **CAPECCHI, M.R.** Altering the genome by homologous recombination. *Science,* 1989, vol. 244, 1288-92
- **CARELL, T. ; E.A. WINTNER ; J. REBEK JR.** A novel procedure for the synthesis of libraries containing small organic molecules. *Angewandte Chemie International Edition,* 1994, vol. 33, 2059-2061
- **CARELL, T. ; E.A. WINTNER ; J. REBEK JR.** A solution phase screening procedure for the isolation of active compounds from a molecular library. *Angewandte Chemie International Edition,* 1994, vol. 33, 2061-2064
- **CARON, P.C. ; W. LAIRD ; M.S. CO ; N.M. AVDALOVIC et al.** Engineered humanized dimeric forms of IgG are more effective antibodies. *J Exp Med.,* 1992, vol. 176, 1191-5
- **CARTER, P.** Site-directed mutagenesis. *Biochem J.,* 1986, vol. 237, 1-7
- **CASE, M.E. ; M. SCHWEIZER ; S.R. KUSHNER ; N.H. GILES.** Efficient transformation of Neurospora crassa by utilizing hybrid plasmid DNA. *Proc Natl Acad Sci USA.,* 1979, vol. 76, 5259-63
- **CEPKO, C.L. ; B.E. ROBERTS ; R.E. MULLIGAN.** Construction and applications of a highly transmissible murine retrovirus shuttle vector. *Cell,* 1984, vol. 37, 1053-1062
- **CHALFIE, M. ; Y. TU ; G. EUSKIRCHEN ; W.W. WARD et al.** Green fluorescent protein as a marker for gene expression. *Science,* 1994, vol. 263, 802-805
- **CHANEY, W.G. ; D.R. HOWARD ; J.W. POLLARD ; S. SALLUSTIO et al.** High-frequency transfection of CHO cells using Polybrene. *Somatic Cell Mol. Genet.,* 1986, vol. 12, 237
- **CHEN, C. ; H. OKAYAMA.** Calcium phosphate-mediated gene transfer: A highly efficient system for stably transforming cells with plasmid DNA. *BioTechniques,* 1988, vol. 6, 632-638
- **CHEN, S.H. ; H.D. SHINE ; J.C. GOODMAN ; R.G. GROSSMAN et al.** Gene therapy for brain tumors: regression of experimental gliomas by adenovirus-mediated gene transfer in vivo. *Proc Natl Acad Sci U S A.,* 1994, vol. 91, 3054-7
- **CHO, C.Y. ; E.J. MORAN ; S.R. CHERRY ; J.C. STEPHANS et al.** An unnatural biopolymer. *Science,* 1993, vol. 261, 1303-5
- **COHEN, A.S. ; D.L. SMISEK ; B.H. WANG.** Emerging technologies for sequencing antisense oligonucleotides: capillary electrophoresis and mass spectrometry. *Adv Chromatogr.,* 1996, vol. 36, 127-62
- **COHEN, J.S.** Oligodeoxynucleotides: Antisense inhibitors of gene expression. CRC Press, 1989, 255
- **COHEN, S.M.N. ; A.C.Y. CHANG ; L. HSU.** Nonchromosomal antibiotic resistance in bacteria: Genetic transformation of Escherichia coli by R-factor DNA. *Proc. Natl. Acad. Sci. USA.,* 1972, vol. 69, 2110
- **COONEY, M. ; G. CZERNUSZEWICZ ; E.H. POSTEL ; S.J. FLINT et al.** Site-specific oligonucleotide binding represses transcription of the human c-myc gene in vitro. *Science,* 1988, vol. 241, 456-9
- **COTTON, R.G.** Current methods of mutation detection. *Mutat Res.,* 1993, vol. 285, 125-44
- **CRONIN, M.T. ; R.V. FUCINI ; S.M. KIM ; R.S. MASINO et al.** Cystic fibrosis mutation detection by hybridization to light-generated DNA probe arrays. *Hum Mutat.,* 1996, vol. 7, 244-55
- **CULL, M.G. ; J.F. MILLER ; P.J. SCHATZ.** Screening for receptor ligands using large libraries of peptides linked to the C terminus of the lac repressor. *Proc Natl Acad Sci U S A.,* 1992, vol. 89, 1865-9

- **CWIRLA, S.E. ; E.A. PETERS ; R.W. BARRETT ; W.J. DOWER.** Peptides on phage: a vast library of peptides for identifying ligands. *Proc Natl Acad Sci USA.,* 1990, vol. 87, 6378-82
- **DE BOER, A.G.** Drug absorption enhancement: Concepts, possibilities, limitations and trends. Harwood Academic Publishers, 1994
- **DE LOUVENCOURT, L. ; H. FUKUHARA ; H. HESLOT ; M. WESOLOWSKI.** Transformation of Kluyveromyces lactis by killer plasmid DNA. *J Bacteriol,* 1983, vol. 154, 737-42
- **DE WET, J.R. ; K.V. WOOD ; M. DELUCA ; D.R. HELINSKI et al.** Sturcture and expression in mammalian cells. *Mol. Cell Biol.,* 1987, vol. 7, 725-737
- **DEMEREC, M. ; E.A. ADELBERG ; A.J. CLARK ; P.E. HARTMAN.** A proposal for a uniform nomenclature in bacterial genetics. *Genetics,* 1966, vol. 54, 61-76
- **DEVLIN, J.J. ; L.C. PANGANIBAN ; P.E. DEVLIN.** Random peptide libraries: a source of specific protein binding molecules. *Science,* 1990, vol. 249, 404-6
- **DEWITT, S.H. ; J.S. KIELY ; C.J. STANKOVIC ; M.C. SCHROEDER et al.** Diversomers'': an approach to nonpeptide, nonoligomeric chemical diversity. *Proc Natl.Acad Sci U S A.,* 1993, vol. 90, 6909-13
- **DIATCHENKO, L. ; Y.F. LAU ; A.P. CAMPBELL ; A. CHENCHIK et al.** Suppression subtractive hybridization: a method for generating differentially regulated or tissue-specific cDNA probes and libraries. *Proc Natl Acad Sci U S A.,* 1996, vol. 93, 6025-30
- **EICHELBAUM, M. ; B. EVERT.** Influence of pharmacogenetics on drug disposition and response. *Clin Exp Pharmacol Physiol.,* 1996, vol. 23, 983-5
- **ELLINGTON, A.D. ; J.W. SZOSTAK.** In vitro selection of RNA molecules that bind specific ligands. *Nature,* 1990, vol. 346, 818-22
- **ELROY-STEIN, O. ; B. MOSS.** Cytoplasmic expression system based on constitutive synthesis of bacteriophage T7 RNA polymerase in mammalian cells. *Proc. Natl. Acad. Sci. USA.,* 1990, vol. 87, 6743-6747
- **EPPSTEIN, D.A. ; Y.V. MARSH ; M. VAN DER PAS ; P.L. FELGNER et al.** Biological activity of liposome-encapsulated murine interferon gamma is mediated by a cell membrane receptor. *Proc Natl Acad Sci U S A.,* 1985, vol. 82, 3688-92
- **ESCUDERO, J. ; B. HOHN.** Transfer and integration of T-DNA without cell injury in the host plant. *Plant Cell,* 1997, vol. 9, 2135-2142
- **FEKETE, D.M. ; C.L. CEPKO.** Retroviral infection coupled with tissue transplantation limits gene transfer in the chick embryo. *Proc. Natl. Acad. Sci. USA.,* 1993, vol. 90, 2350-2354
- **FELGNER, P.L. ; T.R. GADEK ; M. HOLM ; R. ROMAN et al.** Lipofectin: A highly efficient, lipid-mediated DNA/transfection procedure. *Proc. Natl. Acad. Sci. USA.,* 1987, vol. 84, 7413-7417
- **FELICI, F. ; L. CASTAGNOLI ; A. MUSACCHIO ; R. JAPPELLI et al.** Selection of antibody ligands from a large library of oligopeptides expressed on a multivalent exposition vector. *J Mol Biol.,* 1991, vol. 222, 301-10
- **FIECK, A. ; D.L. WYBORSKI ; J.M. SHORT.** Modifications of the E.coli Lac repressor for expression in eukaryotic cells: effects of nuclear signal sequences on protein activity and nuclear accumulation. *Nucleic Acids Res.,* 1992, vol. 20, 1785-91
- **FINER, J.J. ; K.R. FINER ; T. PONAPPA.** Particle bombardment-mediated transformation. *Current Topics in microbiology and immunology,* 1999, vol. 240, 59-80
- **FINN, P.J. ; N.J. GIBSON ; R. FALLON ; A. HAMILTON et al.** Synthesis and properties of DNA-PNA chimeric oligomers. *Nucleic Acids Res.,* 1996, vol. 24, 3357-63
- **FISHWILD, D.M. ; S.L. ODONNELL ; T. BENGOECHEA ; D.V. HUDSON et al.** High-avidity human IgG kappa monoclonal antibodies from a novel strain of minilocus transgenic mice [see comments. *Nat Biotechnol.,* 1996, vol. 14, 845-51
- **FLEER, R. ; P. YEH ; N. AMELLAL ; I. MAURY et al.** Stable multicopy vectors for high-level secretion of recombinant human serum albumin by Kluyveromyces yeasts. *Biotechnology (N Y),* 1991, vol. 9, 968-75
- **FODOR, S.P. ; R.P. RAVA ; X.C. HUANG ; A.C. PEASE et al.** Multiplexed biochemical assays with biological chips. *Nature,* 1993, vol. 364, 555-6
- **FROMM, M. ; L.P. TAYLOR ; V. WALBOT.** Expression of genes transferred into monocot and dicot plant cells by electroporation. *Proc. Natl. Acad. Sci. USA.,* 1985, vol. 82, 5824-5828
- **FUJITA, T. ; H. SHUBIYA ; T. OHASHI ; K. YAMANISHI et al.** Regulation of human interleukin-2 gene: Functional DNA sequences in the 5' flanking region for the gene expression in activated T lymphocytes. *Cell,* 1986, vol. 46, 401-407
- **GABIZON, A. ; R. SHIOTA ; D. PAPAHADJOPOULOS.** Pharmacokinetics and tissue distribution of doxorubicin encapsulated in stable liposomes with long circulation times. *J Natl Cancer Inst.,* 1989, vol. 81, 1484-8
- **GALLAGHER, S.R.** GUS protocols: Using the GUS gene as a reporter of gene expression. Academic Press, 1992
- **GALLOP, M.A. ; R.W. BARRETT ; W.J. DOWER ; S.P. FODOR et al.** Applications of combinatorial technologies to drug discovery. 1. Background and peptide combinatorial libraries. *J Med Chem.,* 1994, vol. 37, 1233-51

- **GASPARINI, P. ; A. BONIZZATO ; M. DOGNINI ; P.F. PIGNATTI.** Restriction site generating-polymerase chain reaction (RG-PCR) for the probeless detection of hidden genetic variation: application to the study of some common cystic fibrosis mutations. *Mol Cell Probes.,* 1992, vol. 6, 1-7
- **GAUTIER, C. ; F. MORVAN ; B. RAYNER ; T. HUYNH-DINH et al.** Alpha-DNA. IV: Alpha-anomeric and beta-anomeric tetrathymidylates covalently linked to intercalating oxazolopyridocarbazole. Synthesis, physicochemical properties and poly (rA) binding. *Nucleic Acids Res.,* 1987, vol. 15, 6625-41
- **GENNARO, A.R.** Remington: The science and practice of pharmacy. Lippincott, Williams & Wilkins, 2000
- **GIBBS, R.A. ; P.N. NGUYEN ; C.T. CASKEY.** Detection of single DNA base differences by competitive oligonucleotide priming. *Nucleic Acids Res.,* 1989, vol. 17, 2437-48
- Growth and transformation of Saccharomyces cerevisiae. **GIETZ, R.D. ; R.A. WOODS ; P. MANIVASAKAM ; R.H. SCHIESTL.** Cells: A laboratory manual. Cold Spring Harbor Press, 1998, vol. I
- **GODING, J.W.** Monoclonal antibodies: Principles and Practice. Academic Press, 1996, 492
- **GORMAN, C.M. ; L.F. MOFFAT ; B.H. HOWARD.** Recombinant genomes which express chloramphenicol acetyltransferase in mammalian cells. *Mol. Cell. Biol.,* 1982, vol. 2, 1044-1051
- **GRAHAM, F.L. ; A.J. VAN DER EB.** A new technique for the assay of infectivity of human adenovirus 5 DNA. *Virology,* 1973, vol. 52, 456
- **GRIFFIN, H.G. ; A.M. GRIFFIN.** DNA sequencing. Recent innovations and future trends. *Appl Biochem Biotechnol.,* 1993, vol. 38, 147-59
- **GROMPE, M. ; D.M. MUZNY ; C.T. CASKEY.** Scanning detection of mutations in human ornithine transcarbamoylase by chemical mismatch cleavage. *Proc Natl Acad Sci U S A.,* 1989, vol. 86, 5888-92
- **GRUBER, M. ; B.A. SCHODIN ; E.R. WILSON ; D.M. KRANZ.** Efficient tumor cell lysis mediated by a bispecific single chain antibody expressed in Escherichia coli. *J Immunol.,* 1994, vol. 152, 5368-74
- **GUATELLI, J.C. ; K.M. WHITFIELD ; D.Y. KWOH ; K.J. BARRINGER et al.** Isothermal, in vitro amplification of nucleic acids by a multienzyme reaction modeled after retroviral replication. *Proc Natl Acad Sci U S A.,* 1990, vol. 87, 1874-8
- **HANAHAN, D.** Studies on transformation of Escherichia coli with plasmids. *J. Mol. Biol,* 1983, vol. 166, 557-580
- **HANSEN, G. ; M.-D. CHILTON.** Lessons in gene transfer to plants by a gifted microbe. *Curr. Top. Microbiol. Immunol.,* 1999, vol. 240, 21-57
- **HANSEN, G. ; M.S. WRIGHT.** Recent advances in the transformation of plants. *Trends Plant Sci.,* 1999, vol. 4, 226-231
- **HARLOW, E. ; D. LANE.** Antibodies: A laboratory manual. Cold Spring Harbor Laboratory Press, 1988, 726
- **HARLOW, E. ; D. LANE.** Using antibodies: A laboratory manual. Cold Spring Harbor Laboratory PRess, 1999
- **HASELOFF, J. ; W.L. GERLACH.** Simple RNA enzymes with new and highly specific endoribonuclease activities. *Nature,* 1988, vol. 334, 585-91
- **HAYASHI, K.** PCR-SSCP: A method for detection of mutations. *Genetic and Analytical Techniques Applications.,* 1992, vol. 9, 73-79
- **HELENE, C.** The anti-gene strategy: control of gene expression by triplex-forming- oligonucleotides. *Anticancer Drug Des.,* 1991, vol. 6, 569-84
- **HELENE, C. ; N.T. THUONG ; A. HAREL-BELLAN.** Control of gene expression by triple helix-forming oligonucleotides. The antigene strategy. *Ann NY Acad Sci.,* 1992, vol. 660, 27-36
- **HINNEN, A. ; J.B. HICKS ; G.R. FINK.** Transformation of yeast. *Proc. Natl. Acad. Sci. USA.,* 1978, vol. 75, 1929-1933
- **HOFFMAN, F.** Laser microbeams for the manipulation of plant cells and subcellular structures. *Plant Sci.,* 1996, vol. 113, 1-11
- **HOGAN, B. ; BEDDINGTON, R. ; COSTANTINI, F. ; LACY, E.** Manipulating the Mouse Embryo: A Laboratory Manual. Cold Spring Harbor Laboratory Press, 1994, 500
- **HOLLIGER, P. ; T. PROSPERO ; G. WINTER.** Diabodies'': small bivalent and bispecific antibody fragments. *Proc Natl Acad Sci U S A,* 1993, vol. 90, 6444-8
- **HOOGENBOOM, H.R. ; A.D. GRIFFITHS ; K.S. JOHNSON ; D.J. CHISWELL et al.** Multi-subunit proteins on the surface of filamentous phage: methodologies for displaying antibody (Fab) heavy and light chains. *Nucleic Acids Res.,* 1991, vol. 19, 4133-7
- **HOUGHTEN, R.A. ; J.R. APPEL ; S.E. BLONDELLE ; J.H. CUERVO et al.** The use of synthetic peptide combinatorial libraries for the identification of bioactive peptides. *Biotechniques,* 1992, vol. 13, 412-21
- **HSU, I.C. ; Q. YANG ; M.W. KAHNG ; J.F. XU.** Detection of DNA point mutations with DNA mismatch repair enzymes. *Carcinogenesis,* 1994, vol. 15, 1657-62
- **HWANG, K.J. ; K.F. LUK ; P.L. BEAUMIER.** Hepatic uptake and degradation of unilamellar sphingomyelin/cholesterol liposomes: a kinetic study. *Proc Natl Acad Sci USA.,* 1980, vol. 77, 4030-4
- **HYRUP, B. ; P.E. NIELSEN.** Peptide nucleic acids (PNA): synthesis, properties and potential applications. *Bioorg Med Chem.,* 1996, vol. 4, 5-23

- **INOUE, H. ; Y. HAYASE ; A. IMURA ; S. IWAI et al.** Synthesis and hybridization studies on two complementary nona(2'-O- methyl)ribonucleotides. *Nucleic Acids Res.,* 1987, vol. 15, 6131-48
- **INOUE, H. ; Y. HAYASE ; S. IWAI ; E. OHTSUKA.** Sequence-dependent hydrolysis of RNA using modified oligonucleotide splints and RNase H. *FEBS Lett.,* 1987, vol. 215, 327-30
- **ISHIURA, M. ; S. HIROSE ; T. UCHIDA ; Y. HAMADA et al.** Phage particle-mediated gene transfer to cultured mammalian cells. *Molecular and Cellular Biology,* 1982, vol. 2, 607-616
- **ITO, H. ; Y. FUKUDA ; K. MURATA ; A. KIMURA.** Transformation of intact yeast cells treated with alkali cations. *J. Bacteriol.,* 1983, vol. 153, 163-168
- **IWABUCHI, K. ; B. LI ; P. BARTEL ; S. FIELDS.** Use of the two-hybrid system to identify the domain of p53 involved in oligomerization. *Oncogene,* 1993, vol. 8, 1693-6
- **JAYASENA, S.D.** Aptamers: an emerging class of molecules that rival antibodies in diagnostics. *Clin Chem.,* 1999, vol. 45, 1628-50
- **JONES, P.T. ; P.H. DEAR ; J. FOOTE ; M.S. NEUBERGER et al.** Replacing the complementarity-determining regions in a human antibody with those from a mouse. *Nature,* 1986, vol. 321, 522-5
- **KAUFMAN, R.J.** Vectors used for expression in mammalian cells. *Methods Enzymol.,* 1990, vol. 185, 487-511
- **KAUFMAN, R.J. ; P. MURTHA ; D.E. INGOLIA ; C.-Y. YEUNG et al.** Selection and amplification of heterologous genes encoding adenosine deaminase in mammalian cells. *Proc. Natl. Acad. Sci. USA.,* 1986, vol. 83, 3136-3140
- **KAWAI, S. ; M. NISHIZAWA.** New procedure for DNA transfection with polycation and dimethyl sulfoxide. *Mol. Cell. Biol.,* 1984, vol. 4, 1172
- **KEEN, J. ; D. LESTER ; C. INGLEHEARN ; A. CURTIS et al.** Rapid detection of single base mismatches as heteroduplexes on Hydrolink gels. *Trends Genet.,* 1991, vol. 7, 5
- **KELLY, J.M. ; M.J. HYNES.** Transformation of Aspergillus niger by the amdS gene of Aspergillus nidulans. *Embo J.,* 1985, vol. 4, 475-9
- **KOSTELNY, S.A. ; M.S. COLE ; J.Y. TSO.** Formation of a bispecific antibody by the use of leucine zippers. *J Immunol.,* 1992, vol. 148, 1547-53
- **KOZAL, M.J. ; N. SHAH ; N. SHEN ; R. YANG et al.** Extensive polymorphisms observed in HIV-1 clade B protease gene using high-density oligonucleotide arrays. *Nat Med.,* 1996, vol. 2, 753-9
- **KOZBOR, D. ; P. TRIPPUTI ; J.C. RODER ; C.M. CROCE.** A human hybrid myeloma for production of human monoclonal antibodies. *J Immunol.,* 1984, vol. 133, 3001-5
- **KRIEGLER, M.** Gene transfer and expression: A laboratory manual. Stockton Press, 1990, 242
- **KWOH, D.Y. ; G.R. DAVIS ; K.M. WHITFIELD ; H.L. CHAPPELLE et al.** Transcription-based amplification system and detection of amplified human immunodeficiency virus type 1 with a bead-based sandwich hybridization format. *Proc Natl Acad Sci U S A.,* 1989, vol. 86, 1173-7
- **LAKSO, M. ; B. SAUER ; B. MOSINGER ; E.J. LEE et al.** Targeted oncogene activation by site-specific recombination in transgenic mice. *Proc Natl Acad Sci US A.,* 1992, vol. 89, 6232-6
- **LAM, K.S.** Application of combinatorial library methods in cancer research and drug discovery. *Anticancer Drug Design.,* 1997, vol. 12, 145-167
- **LAM, K.S. ; S.E. SALMON ; E.M. HERSH ; V.J. HRUBY et al.** General method for rapid synthesis of multicomponent peptide mixtures. *Nature,* 1991, vol. 354, 82-84
- **LANDEGREN, U. ; R. KAISER ; J. SANDERS ; L. HOOD.** A ligase-mediated gene detection technique. *Science,* 1988, vol. 241, 1077-80
- **LEDUC, N.** Isolated maize zygotes mimic in vivo embryogenic development and express microinjected genes when cultured in vitro. *Dev. Biol.,* 1996, vol. 10, 190-203
- **LEE, J.S. ; D.A. JOHNSON ; A.R. MORGAN.** Complexes formed by (pyrimidine)n (purine)n DNAs on lowering the pH are three-stranded. *Nucleic Acids Res.,* 1979, vol. 6, 3073-91
- **LEE, V.H.L.** Peptide and protein drug delivery. Marcel Dekker, 1990
- **LEMAITRE, M. ; B. BAYARD ; B. LEBLEU.** Specific antiviral activity of a poly(L-lysine)-conjugated oligodeoxyribonucleotide sequence complementary to vesicular stomatitis virus N protein mRNA initiation site. *Proc Natl Acad Sci USA.,* 1987, vol. 84, 648-52
- **LEMISCHKA, I.R. ; D.H. RAULET ; R.C. MULLIGAN.** Developmental potential and dynamic behavior of hematopoietic stem cells. *Cell,* 1986, vol. 45, 917-927
- **LETSINGER, R.L. ; G.R. ZHANG ; D.K. SUN ; T. IKEUCHI et al.** Cholesteryl-conjugated oligonucleotides: synthesis, properties, and activity as inhibitors of replication of human immunodeficiency virus in cell culture. *Proc Natl Acad Sci U S A.,* 1989, vol. 86, 6553-6
- **LI, E. ; T.H. BESTOR ; R. JAENISCH.** Targeted mutation of the DNA methyltransferase gene results in embryonic lethality. *Cell,* 1992, vol. 69, 915-26
- **LINDER, M.W. ; R.A. PROUGH ; R. VALDES.** Pharmacogenetics: a laboratory tool for optimizing therapeutic efficiency. *Clin Chem.,* 1997, vol. 43, 254-66
- **LITTLEFIELD, J.W.** Selection of hybrids from matings of fibroblasts in vitro and their presumed recombinants. *Science,* 1964, vol. 145, 709-710

- **LIZARDI, P.M. ; C.E. GUERRA ; H. LOMELI ; I. TUSSIE-LUNA et al.** Exponential amplification of recombinant-RNA hybridization probes. *Biotechnology,* 1988, vol. 6, 1197-1202
- **LONBERG, N. ; D. HUSZAR.** Human antibodies from transgenic mice. *Int Rev Immunol.,* 1995, vol. 13, 65-93
- **LONBERG, N. ; L.D. TAYLOR ; F.A. HARDING ; M. TROUNSTINE et al.** Antigen-specific human antibodies from mice comprising four distinct genetic modifications [see comments. *Nature,* 1994, vol. 368, 856-9
- **LOPATA, M.A. ; D.W. CLEVELAND ; B. SOLLNER-WEBB.** High-level expression of a chloramphenicol acetyltransferase gene by DEAEdextran-mediated DNA traansfection couled with a dimethylsulfoxide or glycerol shock treatment. *Nucleic Acids Research,* 1984, vol. 12, 5707
- Cloning and expression of heterologous genes in insect cells with baculovirus vectors. **LUCKOW, V.A.** In Recombinant DNA technology and applications. McGraw-Hill, 1991, 97-152
- **MADURA, K. ; R.J. DOHMEN ; A. VARSHAVSKY.** N-recognin/Ubc2 interactions in the N-end rule pathway. *J Biol Chem.,* 1993, vol. 268, 12046-54
- **MAHER, L.J.** DNA triple-helix formation: an approach to artificial gene repressors?. *Bioessays,* 1992, vol. 14, 807-15
- **MANDEL, M. ; A. HIGA.** Calcium-dependent bacteriophage DNA infection. *J. Mol. biol.,* 1970, vol. 53, 159-162
- **MARASCO, W.A. ; W.A. HASELTINE ; S.Y. CHEN.** Design, intracellular expression, and activity of a human anti-human immunodeficiency virus type 1 gp120 single-chain antibody. *Proc Natl Acad Sci U S A.,* 1993, vol. 90, 7889-93
- **MARKS, J.D. ; A.D. GRIFFITHS ; M. MALMQVIST ; T.P. CLACKSON et al.** By-passing immunization: building high affinity human antibodies by chain shuffling. *Biotechnology (N Y),* 1992, vol. 10, 779-83
- **MARKS, J.D. ; H.R. HOOGENBOOM ; T.P. BONNERT ; J. MCCAFFERTY et al.** By-passing immunization. Human antibodies from V-gene libraries displayed on phage. *J Mol Biol.,* 1991, vol. 222, 581-97
- **MARTIN, F.J. ; D. PAPAHADJOPOULOS.** Irreversible coupling of immunoglobulin fragments to preformed vesicles. An improved method for liposome targeting. *J Biol Chem.,* 1982, vol. 257, 286-8
- **MAXAM, A.M. ; W. GILBERT.** A new method for sequencing DNA. *Proc Natl Acad Sci U S A,* 1977, vol. 74, 560-4
- **MILLER, A.D. ; C. BUTTIMORE.** Redesign of retrovirus packaging cell lines to avoid recombination leading to helper virus production. *Mol. Cell biol.,* 1986, vol. 6, 2895-2902
- **MILLER, L.K.** Baculoviruses as gene expression vectors. *Annu. Rev. Microbiol.,* 1988, vol. 42, 177-199
- **MILSTEIN, C. ; A.C. CUELLO.** Hybrid hybridomas and their use in immunohistochemistry. *Nature,* 1983, vol. 305, 537-40
- **MORRISON, S.L. ; L. WIMS ; S. WALLICK ; L. TAN et al.** Genetically engineered antibody molecules and their application. *Ann NY Acad Sci.,* 1987, vol. 507, 187-98
- **MUNSON, P.J. ; D. RODBARD.** Ligand: a versatile computerized approach for characterization of ligand-binding systems. *Anal Biochem.,* 1980, vol. 107, 220-39
- **MYERS, R.M. ; Z. LARIN ; T. MANIATIS.** Detection of single base substitutions by ribonuclease cleavage at mismatches in RNA:DNA duplexes. *Science,* 1985, vol. 230, 1242-6
- **NAEVE, C.W. ; G.A. BUCK ; R.L. NIECE ; R.T. PON et al.** Accuracy of automated DNA sequencing: a multi-laboratory comparison of sequencing results. *Biotechniques,* 1995, vol. 19, 448-53
- **NAKAI, K. ; P. HORTON.** PSORT: a program for detecting sorting signals in proteins and predicting their subcellular localization. *Trends Biochem Sci.,* 1999, vol. 24, 34-6
- **NAKAZAWA, H. ; D. ENGLISH ; P.L. RANDELL ; K. NAKAZAWA et al.** UV and skin cancer: specific p53 gene mutation in normal skin as a biologically relevant exposure measurement. *Proc Natl Acad Sci U S A.,* 1994, vol. 91, 360-4
- **NEUMANN, E. ; M. SCHAEFER-RIDDER ; Y. WANG ; P.H. HOFSCHNEIDER.** Gene transfer into mouse lyoma cells by electroporation in high electric fields. *EMBO J.,* 1982, vol. 1, 841-845
- **O'GORMAN, S. ; D.T. FOX ; G.M. WAHL.** Recombinase-mediated gene activation and site-specific integration in mammalian cells. *Science,* 1991, vol. 251, 1351-5
- **OKANO, H. ; J. ARUGA ; T. NAKAGAWA ; C. SHIOTA et al.** Myelin basic protein gene and the function of antisense RNA in its repression in myelin-deficient mutant mouse. *J Neurochem.,* 1991, vol. 56, 560-7
- **O'REILLY, D.R. ; L.K. MILLER ; V.A. LUCKOW.** Baculovirus expression vectors. W.H. Freeman and Company, 1992
- **ORITA, M. ; H. IWAHANA ; H. KANAZAWA ; K. HAYASHI et al.** Detection of polymorphisms of human DNA by gel electrophoresis as single-strand conformation polymorphisms. *Proc Natl Acad Sci U S A.,* 1989, vol. 86, 2766-70
- **OU-LEE, T.M. ; R. TURGEON ; R. WU.** Uptake and expression of a foreign gene linked to either a plant virus or Drosophila promoter in protoplasts of rice, wheat and sorghum. *Proc. Natl. Acad. Sci. USA.,* 1986, vol. 83, 6815-6819

- **PALMER, T.D. ; R.A. HOCK ; W.R.A. OSBORNE ; A.D. MILLER.** Efficient retrovirus-mediated transfer and expression of a human adenosine deaminase gene in diploid skin fibroblasts from an adenosie-deficient human. *Proc. Natl. Acad. Sci. USA.,* 1987, vol. 84, 1055-1059
- **PEAR, W. ; G. NOLAN ; M. SCOTT ; D. BALTIMORE.** Production of high-titer helper-free retroviruses by transient transfection. *Proc. Natl. Acad. Sci. USA.,* 1993, vol. 90, 8392-8396
- **PENNICA, D. ; T.A. SWANSON ; J.W. WELSH ; M.A. ROY et al.** WISP genes are members of the connective tissue growth factor family that are up-regulated in wnt-1-transformed cells and aberrantly expressed in human colon tumors. *Proc Natl Acad Sci U S A.,* 1998, vol. 95, 14717-22
- **PERRY-O'KEEFE, H. ; X.W. YAO ; J.M. COULL ; M. FUCHS et al.** Peptide nucleic acid pre-gel hybridization: an alternative to southern hybridization. *Proc Natl Acad Sci USA.,* 1996, vol. 93, 14670-5
- **PETERSEN, K.H. ; D.K. JENSEN ; M. EGHOLM ; O. BUCHARDT et al.** A PNA-DNA linker synthesis of N-((4,4'-dimethoxytrityloxy)ehtyl)-N-(thymin-1-ylacetyl)glycine. *Biorganic and Medicianl Chemistry Letters,* 1976, vol. 5, 1119-1124
- **POTTER, H.** Electroporation in biology: Methods, applications,, and instrumentation. *Analytical Biochemistry,* 1988, vol. 174, 361-373
- **POTTER, H. ; L. WEIR ; P. LEDER.** Enhancer-dependent expression of human kappa immunoglobulin genes introduced into mouse pre-B lymphocytes by electroporation. *Proc. Natl. Acad. Sci. USA.,* 1984, vol. 81, 7161-7165
- **PRESTA, L.G.** Antibody engineering. *Curr Opin Biotechnol.,* 1992, vol. 3, 394-8
- **PROSSER, J.** Detecting single-base mutations. *Trends Biotechnol.,* 1993, vol. 11, 238-46
- **RASSOULZADEGAN, M. ; B. BINETRUY ; F. CUZIN.** High frequency of gene transfer after fusion between bacteria and eukaryotic cells. *Nature,* 1982, vol. 295, 257
- **REISFELD, R.A. ; S. SELL.** Monoclonal antibodies and cancer therapy: Proceedings of the Roche-UCLA symposium held in Park City. Alan R. Liss, 1985, 609
- **RHODES, C.A. ; D.A. PIERCE ; I.J. METTLER ; D. MASCARENHAS et al.** Genetically transformed maize plants from protoplasts. *Science,* 1988, vol. 240, 204-207
- **RIECHMANN, L. ; M. CLARK ; H. WALDMANN ; G. WINTER.** Reshaping human antibodies for therapy. *Nature,* 1988, vol. 332, 323-7
- **ROSE, J.K. ; L. BUONOCORE ; M. WHITT.** A new cationic liposome reagent mediating nearly quantitative transfection of animal cells. *BioTechniques,* 1991, vol. 10, 520-525, BioTechniques
- **ROSSI, J.J.** Practical ribozymes. Making ribozymes work in cells. *Curr Biol.,* 1994, vol. 4, 469-71, Curr Biol.
- **ROSSITER, B.J. ; C.T. CASKEY.** Molecular scanning methods of mutation detection. *J Biol Chem.,* 1990, vol. 265, 12753-6
- **SAIKI, R.K. ; T.L. BUGAWAN ; G.T. HORN ; K.B. MULLIS et al.** Analysis of enzymatically amplified beta-globin and HLA-DQ alpha DNA with allele-specific oligonucleotide probes. *Nature,* 1986, vol. 324, 163-6
- **SAIKI, R.K. ; P.S. WALSH ; C.H. LEVENSON ; H.A. ERLICH.** Genetic analysis of amplified DNA with immobilized sequence-specific oligonucleotide probes. *Proc Natl Acad Sci USA.,* 1989, vol. 86, 6230-4
- **SALEEBA, J.A. ; R.G. COTTON.** Chemical cleavage of mismatch to detect mutations. *Methods Enzymol.,* 1993, vol. 217, 286-95
- **SAMBROOK, J.** Molecular cloning: a laboratory manual. Cold Spring Harbor Laboratory, 1989
- **SANDRI-GOLDIN, R.M. ; A.L. GOLDIN ; J.C. GLORIOSO ; M. LEVINE.** High-frequency transfer of cloned herpes simjplex virus type I sequences to mammalian cells by protoplast fusion. *Mol. Cell. Biol.,* 1981, vol. 1, 7453-752
- **SANGER, F. ; S. NICKLEN ; A.R. COULSON.** DNA sequencing with chain-terminating inhibitors. *Proc Natl Acad Sci U S A.,* 1977, vol. 74, 5463-7
- Plant gene transfer using electrofusion and electroporation. **SAUNDERS, J.A. ; B.F. MATTHEWS ; P.D. MILLER.** In Electroporation and electrofusion in cell biology. Plenum Press, 1989, 343-354
- **SCHADE, R. ; C. STAAK ; C. HENDRIKSEN ; M. ERHARD et al.** The production of avian (egg yold) antibodies: IgY. The report and recommendations of ECVAM workshop. *Alternatives to Laboratory Animals (ATLA),* 1996, vol. 24, 925-934
- **SCHAFFNER, W.** Direct transfer of cloned genes from bacteria to mammalian cells. *Proc. Natl. Acad. Sci. USA.,* 1980, vol. 77, 2163
- **SCHOOK, L.B.** Monoclonal antibody production techniques and applications. Marcel Dekker, Inc, 1987, 336
- **SCOTT, J.K. ; G.P. SMITH.** Searching for peptide ligands with an epitope library. *Science,* 1990, vol. 249, 386-90
- **SELDEN, R.F. ; K. BURKE-HOWIE ; M.E. ROWE ; H.M. GOODMAN et al.** Human growth hormone as a reporter gene in regulation studies employing transient gene expression. *Molecular and Cellular Biololgy.,* 1986, vol. 6, 3173-3179
- **SHALABY, M.R. ; H.M. SHEPARD ; L. PRESTA ; M.L. RODRIGUES et al.** Development of humanized bispecific antibodies reactive with cytotoxic lymphocytes and tumor cells overexpressing the HER2 protooncogene. *J Exp Med.,* 1992, vol. 175, 217-25

- **SHIGEKAWA, K. ; W.J. DOWER.** Electroporation of eukaryotes and prokaryotes: A general approach to the introduction of macomolecules into cells. *BioTechniques,* 1988, vol. 6, 742-751
- **SHILLITO, R.** Methods of genetic transformations: Electroporation and polyethylene glycol treatment. In Molecular improvement of cereal crop. I. Kluwer, 1999, 9-20
- **SHILO, B.Z. ; R.A. WEINBERG.** DNA sequences homologous to vertebrate oncogenes are conserved in Drosophila melanogaster. *Proc Natl Acad Sci U S A.,* 1981, vol. 78, 6789-92
- **SHOPES, B.** A genetically engineered human IgG mutant with enhanced cytolytic activity. *J Immunol.,* 1992, vol. 148, 2918-22
- **SIMONSEN, C.C. ; A.D. LEVINSON.** Isolation and expression of an altered mouse dihydrofolate reductase cDNA. *Proc. Natl. Acad. Sci. USA.,* 1983, vol. 80, 2495-2499
- **SOUTHERN, P.J. ; P. BERG.** Transformation of mammalian cells to antibiotic resistanced with a bacterial gene under control of the SV40 early region promoter. *J. Mol. Appl. Gen.,* 1982, vol. 1, 327-341
- **SREEKRISHNA, K. ; R.H. POTENZ ; J.A. CRUZE ; W.R. MCCOMBIE et al.** High level expression of heterologous proteins in methylotrophic yeast Pichia pastoris. *J Basic Microbiol.,* 1988, vol. 28, 265-78
- **STEIN, C.A. ; J.S. COHEN.** Oligodeoxynucleotides as inhibitors of gene expression: a review. *Cancer Res.,* 1988, vol. 48, 2659-68
- **STEVENSON, G.T. ; A. PINDAR ; C.J. SLADE.** A chimeric antibody with dual Fc regions (bisFabFc) prepared by manipulations at the IgG hinge. *Anticancer Drug Des.,* 1989, vol. 3, 219-30
- **SURESH, M.R. ; A.C. CUELLO ; C. MILSTEIN.** Bispecific monoclonal antibodies from hybrid hybridomas. *Methods Enzymol.,* 1986, vol. 121, 210-28
- **THOMAS, K.R. ; M.R. CAPECCHI.** Site-directed mutagenesis by gene targeting in mouse embryo-derived stem cells. *Cell,* 1987, vol. 51, 503-12
- **THOMPSON, J.A.** Maize transformation utilizing silicon carbide whiskers: A review. *Euphytica,* 1995, vol. 85, 75-80
- **TILBURN, J. ; C. SCAZZOCCHIO ; G.G. TAYLOR ; J.H. ZABICKY-ZISSMAN et al.** Transformation by integration in Aspergillus nidulans. *Gene,* 1983, vol. 26, 205-21
- **TOURAEV, A.** Plant male germ line transformation. *Plant J.,* 1997, vol. 12, 949-956
- **TRAUNECKER, A. ; F. OLIVERI ; K. KARJALAINEN.** Myeloma based expression system for production of large mammalian proteins. *Trends Biotechnol.,* 1991, vol. 9, 109-13
- **TRICK, H.N.** Recent advances in soybean transformation. *Plant Tissue Cult. Biotechnol.,* 1997, vol. 3, 9-26
- **TUERK, C. ; L. GOLD.** Systematic evolution of ligands by exponential enrichment: RNA ligands to bacteriophage T4 DNA polymerase. *Science,* 1990, vol. 249, 505-10
- **TURNER, D.L. ; E.Y. SNYDER ; C.L. CEPKO.** Lineage-independent determinationh of cell type in the embryonic mouse retina. *Neuron,* 1990, vol. 4, 833-845
- **TUTT, A. ; G.T. STEVENSON ; M.J. GLENNIE.** Trispecific F(ab')3 derivatives that use cooperative signaling via the TCR/CD3 complex and CD2 to activate and redirect resting cytotoxic T cells. *J Immunol.,* 1991, vol. 147, 60-9
- **VAN DER KROL, A.R. ; J.N. MOL ; A.R. STUITJE.** Modulation of eukaryotic gene expression by complementary RNA or DNA sequences. *Biotechniques,* 1988, vol. 6, 958-76
- **VERHOEYEN, M. ; C. MILSTEIN ; G. WINTER.** Reshaping human antibodies: grafting an antilysozyme activity. *Science,* 1988, vol. 239, 1534-6
- **VITETTA, E.S. ; R.J. FULTON ; R.D. MAY ; M. TILL et al.** Redesigning nature's poisons to create anti-tumor reagents. *Science,* 1987, vol. 238, 1098-104
- **WELLS, J.A. ; M. VASSER ; D.B. POWERS.** Cassette mutagenesis: an efficient method for generation of multiple mutations at defined sites. *Gene,* 1985, vol. 34, 315-23
- **WHITT, M.A. ; L. BUONOCORE ; J.K. ROSE ; V. CICCARONE et al.** TransfectACE reagent promotes transient transfection frequencies greater than 90%. *Focus,* 1990, vol. 13, 8-12
- **WIGLER, M. ; A. PELLICER ; S. SILVERSTTEIN ; R. AXEL.** Biochemical transfer of single-copy eucaryotic genes using total cellular DNA as donor. *Cell,* 1978, vol. 14, 725
- **WILLIAMS, D.A. ; I.R. LEMISCHKA ; D.G. NATHAN ; R.C. MULLIGAN.** Introduction of a new genetic material into pluripotent haematopoietic stem cells of the mouse. *Nature,* 1984, vol. 310, 476-480
- **WILMUT, I. ; A.E. SCHNIEKE ; J. MCWHIR ; A.J. KIND et al.** Viable offspring derived from fetal and adult mammalian cells. *Nature,* 1997, vol. 385, 810-3
- **WOLFF, E.A. ; G.J. SCHREIBER ; W.L. COSAND ; H.V. RAFF.** Monoclonal antibody homodimers: enhanced antitumor activity in nude mice. *Cancer Res.,* 1993, vol. 53, 2560-5
- **WONG, G.T. ; B.J. GAVIN ; A.P. MCMAHON.** Differential transformation of mammary epithelial cells by Wnt genes. *Mol Cell Biol.,* 1994, vol. 14, 6278-86

- **WONG, T.K. ; E. NEUMANN.** Electric field mediated gene transfer. *Biochemical and Biophysical Research Communications,* 1982, vol. 107, 584-587
- **WYBORSKI, D.L. ; L.C. DUCOEUR ; J.M. SHORT.** Parameters affecting the use of the lac repressor system in eukaryotic cells and transgenic animals. *Environ Mol Mutagen.,* 1996, vol. 28, 447-58
- **WYBORSKI, D.L. ; J.M. SHORT.** Analysis of inducers of the E.coli lac repressor system in mammalian cells and whole animals. *Nucleic Acids Res.,* 1991, vol. 19, 4647-53
- **YELTON, M.M. ; J.E. HAMER ; W.E. TIMBERLAKE.** Transformation of Aspergillus nidulans by using a trpC plasmid. *Proc Natl Acad Sci U S A.,* 1984, vol. 81, 1470-4
- **ZERVOS, A.S. ; J. GYURIS ; R. BRENT.** Mxi1, a protein that specifically interacts with Max to bind Myc-Max recognition sites. *Cell,* 1993, vol. 72, 223-32
- **ZHOU, G.** ntroduction of exogenous DNA into cotton embryos. *Methods Enzymol.,* 1983, vol. 101, 433-481
- **ZOLLER, M.J. ; M. SMITH.** Oligonucleotide-directed mutagenesis: a simple method using two oligonucleotide primers and a single-stranded DNA template. *Methods Enzymol.,* 1987, vol. 154, 329-50
- **ZON, G.** Oligonucleotide analogues as potential chemotherapeutic agents. *Pharm Res.,* 1988, vol. 5, 539-49
- **ZUCKERMANN, R.N. ; E.J. MARTIN ; D.C. SPELLMEYER ; G.B. STAUBER et al.** Discovery of nanomolar ligands for 7-transmembrane G-protein-coupled receptors from a diverse N-(substituted)glycine peptoid library. *J Med Chem.,* 1994, vol. 37, 2678-85

SEQUENCE LISTING

<110> Genentech, Inc.
CuraGen Corporation

<120> NOVEL POLYPEPTIDES, AND NUCLEIC ACIDS ENCODING THE SAME

<130> 11669.210EPWO

<140> PCT/US01/09600
<141> 2001-03-22

<150> US 60/191,258
<151> 2000-03-22

<160> 39

<170> Patent In version 3.3

<210> 1
<211> 1066
<212> DNA
<213> Murine sp.

<400> 1

```
cccaggcgtc ttggtggtgg tgagtgaggt ttagggagct ggggctcgcg cagcggtgtc     60
tgccagcgga ctgttcggcg gcttgacgtc cccagacgct gtgcttgagc cggtgcaccc    120
caggaattag gtagcctgct tgccttgcat ttctgcaccg ctctccgtcc gtggacctcg    180
gtgtcccctc cttgtttctc tcgcggcttt cctccctttg gaccggcacg tgtcggagct    240
ccaacctggg acaatggtgt gcattccttg cattgtcatt ccagtcctgc tctggatctt    300
caaaaagttc ctggagccat acatataccc tgtggtcagt cgcatatggc ctaaaaaagc    360
cgtccagcaa tccggcgata agaatatgag caaggtagac tgcaagggtg caggtactaa    420
tggattaccc acaaaaggac caacagaagt ctcggataaa aagaaagact agtgtgggtc    480
tcctgaaggc ccttggctgt ttgcaaatgg acctaatgat atgaagcctt ctttgtctct    540
gaccttttt ctctgagacc aggaatctag ataatagttt agcttctgcc tgatactgat     600
ccgggagcac atgatattta tatttaaaat tccagtagtt atatttaaga tctcacccct    660
gagtttcttt ttcattaaag tagctttcat ttctattatt ccaatttact gatatgaaca    720
aatagaaggt ccgtgtgagc agacgctcag aacagagccc ttggcccttc gagttctttc    780
ttacgagttt gccgttctca cttctgtggg ctcctatacc ttgagtggga tgagtcttag    840
tgggaaacag tgccgtccga ggtgggatgc gatgagaaga tgtgatcact gcaggcgcag    900
cggcgagtgg acagctggcc gagaccagct ccaaggcagc tggagaagga aggacgggag    960
cttccttgaa aaatgtaacc tggacatcgt tgtcaatccc acaacccctg actctctgtg   1020
cttctagtcc tgacggtgta ttaaacgtcc atttaacttg tgaaaa                  1066
```

<210> 2
<211> 113
<212> PRT
<213> Murine sp.

<400> 2

```
Val Ala Cys Leu Pro Cys Ile Ser Ala Pro Leu Ser Val Arg Gly Pro
1               5                   10                  15

Arg Cys Pro Leu Leu Val Ser Leu Ala Ala Phe Leu Pro Leu Asp Arg
            20                  25                  30

His Val Ser Glu Leu Gln Pro Gly Thr Met Val Cys Ile Pro Cys Ile
        35                  40                  45

Val Ile Pro Val Leu Leu Trp Ile Phe Lys Lys Phe Leu Glu Pro Tyr
    50                  55                  60

Ile Tyr Pro Val Val Ser Arg Ile Trp Pro Lys Lys Ala Val Gln Gln
65                  70                  75                  80

Ser Gly Asp Lys Asn Met Ser Lys Val Asp Cys Lys Gly Ala Gly Thr
                85                  90                  95

Asn Gly Leu Pro Thr Lys Gly Pro Thr Glu Val Ser Asp Lys Lys Lys
            100                 105                 110

Asp
```

<210> 3
<211> 955
<212> DNA
<213> Murine sp.

<220>
<221> misc feature
<222> (1)..(3)
<223> n is a, c, g, or t

<220>
<221> misc_feature
<222> (7)..(7)
<223> n is a, c, g, or t

<220>
<221> misc_feature
<222> (48)..(48)
<223> n is a, c, g, or t

<220>
<221> misc_feature
<222> (128)..(128)
<223> n is a, c, g, or t

<220>
<221> misc_feature

<222> (268) .. (268)
<223> n is a, c, g, or t

<220>
<221> misc feature
<222> (801)..(801)
<223> n is a, c, g, or t

<400> 3

```
nnngtgngtg aggtttaggg agctggggct cgcgcagcgg gtgtctgnca gcggagctgt      60
tcggcggctt gacgtcccca gacgctgtgc gttgagccgg tgcaccccag gaattagtgt     120
cggagctnca acctgggaca atggtgtgca ttccttgcat tgtcattcca gtcctgctct     180
ggatcttcaa aaagttcctg gagccataca tataccctgt ggtcagtcgc atatggccta     240
aaaaagccgt ccagcaatcc ggcgatanga atatgagcaa ggtagactgc aagggtgcag     300
gtactaatgg attacccaca aaaggaccaa cagaagtctc ggataaaaag aaagactagt     360
gtgggtctcc tgaaggccct tggctgtttg caaatggacc taatgatatg aagccttctt     420
tgtctctgac ctttttctc tgagaccagg aatctagata atagtttagc ttctgcctga      480
tactgatccg ggagcacatg atatttatat ttaaaattcc agtagttata tttaatgatc     540
tcacccctga gtttcttttt cattaaagta gctttcattt ctattattcc aatttactga     600
tatgaacaaa tagaaggtcc gtgtgagcag acgctcagaa cagagccctt ggcccttcga     660
gttctttctt acgagtttgc cgttctcact tctgtgggct cctatacctt gagtgggatg     720
agtcttagtg ggaaacagtg ccgtccgagg tgggatgcga tgagaagatg tgatcactgc     780
aggcgcagcg gcgagtggac ngctggccga gaccagctcc aaggcagctg gagaaggaag     840
gacgggagct tccttgaaaa atgtaacctg gacatcgttg tcaatcccac aacccctgac     900
tctctgtgct tctagtcctg acggtgtatt aaacgtccat ttaacttgtg gaaaa          955
```

<210> 4
<211> 87
<212> PRT
<213> Murine sp.

<220>
<221> misc_feature
<222> (58)..(58)
<223> Xaa can be any naturally occurring amino acid

<400> 4

```
Ala Gly Ala Pro Gln Glu Leu Val Ser Glu Leu Gln Pro Gly Thr Met
1               5                   10                  15

Val Cys Ile Pro Cys Ile Val Ile Pro Val Leu Leu Trp Ile Phe Lys
            20                  25                  30

Lys Phe Leu Glu Pro Tyr Ile Tyr Pro Val Val Ser Arg Ile Trp Pro
        35                  40                  45

Lys Lys Ala Val Gln Gln Ser Gly Asp Xaa Asn Met Ser Lys Val Asp
    50                  55                  60

Cys Lys Gly Ala Gly Thr Asn Gly Leu Pro Thr Lys Gly Pro Thr Glu
65                  70                  75                  80

Val Ser Asp Lys Lys Lys Asp
                85
```

<210> 5
<211> 1099
<212> DNA
<213> Homo sapiens

<220>
<221> misc_feature
<222> (64) ..(64)
<223> n is a, c, g, or t

<400> 5

```
ggctttgtag ctgctccgca gcccagcccg ggcgcgctcg cagagtccta ggcggtgcgc      60
ggcntcctgc ctcctccctc ctcggcggtc gcggcccgcg cctccgcggt gcctgccttc     120
gctctcaggt tgaggagctc aagcttggga aaatggtgtg cattccttgt atcgtcattc     180
cagttctgct ctggatctac aaaaaattcc tggagccata tatataccct ctggtttccc     240
ccttcgttag tcgtatatgg cctaagaaag caatacaaga atccaatgat acaaacaaag     300
gcaaagtaaa ctttaagggt gcagacatga atggattacc aacaaaagga ccaacagaaa     360
tctgtgataa aaagaaagac taaagaaatt ttcctaaagg accccatcat ttaaaaaatg     420
gacctgataa tatgaagcat cttccttgta attgtctctg accttttttat ctgagaccgg     480
aattcaggat aggagtctag atatttacct gatactaatc aggaaatata tgatatccgt     540
atttaaaatg tagttagtta tatttaatga cctcattcct aagttccttt ttcgttaatg     600
tagctttcat ttctgttatt gctgtttgaa taatatgatt aaatagaagg tttgtgccag     660
tagacattat gttactaaat cagcacttta aaatctttgg ttctctaatt catatgaatt     720

tgctgtttgc tctaatttct ttgggctctt ctaatttgag tggagtacaa ttttgttgtg     780
aaacagtcca gtgaaactgt gcagggaaat gaaggtagaa ttttgggagg taataatgat     840
gtgaaacata aagatttaat aattactgtc caacacagtg gagcagcttg tccacaaata     900
tagtaattac tatttattgc tctaaggaag attaaaaaaa gatagggaaa agggggaaac     960
ttctttgaaa aatgaaacat ctgttacatt aatgtctaat tataaaattt taatccttac    1020
tgcatttctt ctgttcctac aaatgtatta aacattcagt ttaactggta aaaaaaaaaa    1080
aaaaaaaccc ggggggggg                                                 1099
```

<210> 6
<211> 126
<212> PRT
<213> Homo sapiens

<220>
<221> misc_feature
<222> (21) .. (21)
<223> Xaa can be any naturally occurring amino acid

<400> 6

```
Leu Cys Ser Cys Ser Ala Ala Gln Pro Gly Arg Ala Arg Arg Val Leu
1               5                   10                  15

Gly Gly Ala Arg Xaa Pro Ala Ser Ser Leu Leu Gly Gly Arg Gly Pro
            20                  25                  30

Arg Leu Arg Gly Ala Cys Leu Arg Ser Gln Val Glu Glu Leu Lys Leu
        35                  40                  45

Gly Lys Met Val Cys Ile Pro Cys Ile Val Ile Pro Val Leu Leu Trp
    50                  55                  60

Ile Tyr Lys Lys Phe Leu Glu Pro Tyr Ile Tyr Pro Leu Val Ser Pro
65                  70                  75                  80

Phe Val Ser Arg Ile Trp Pro Lys Lys Ala Ile Gln Glu Ser Asn Asp
                85                  90                  95

Thr Asn Lys Gly Lys Val Asn Phe Lys Gly Ala Asp Met Asn Gly Leu
            100                 105                 110

Pro Thr Lys Gly Pro Thr Glu Ile Cys Asp Lys Lys Lys Asp
        115                 120                 125
```

<210> 7
<211> 1113
<212> DNA
<213> Homo sapiens

<400> 7

gtgagtgtgc ccgggctagc ggcctgggtt gggctttgta gctgctccgc ggcccagccc 60

gggcgcgctc gcagagtcct aggcggtgcg cggcctcctg cctcctccct cctcggcggt 120

cgcggcccgc cggcctccgc ggtgcctgcc ttcgctctca ggttgaggag ctcaagcttg 180

ggaaaatggt gtgcattcct tgtatcgtca ttccagttct gctctggatc tacaaaaaat 240

tcctggagcc atatatatac cctctggttt ccccttcgt tagtcgtata tggcctaaga 300

aagcaataca agaatccaat gatacaaaca aaggcaaagt aaactttaag ggtgcagaca 360

tgaatggatt accaacaaaa ggaccaacag aaatctgtga taaaaagaaa gactaaagaa 420

attttcctaa aggaccccat catttaaaaa atggacctga taatatgaag catcttcctt 480

gtaattgtct ctgacctttt tatctgagac cggaattcag gataggagtc tagatattta 540

cctgatacta atcaggaaat atatgatatc cgtatttaaa atgtagttag ttatatttaa 600

tgacctcatt cctaagttcc tttttcgtta atgtagcttt catttctgtt attgctgttt 660

gaataatatg attaaataga aggtttgtgc cagtagacat tatgttacta aatcagcact 720

ttaaaatctt tggttctcta attcatatga atttgctgtt tgctctaatt tctttgggct 780

cttctaattt gagtggagta caattttgtt gtgaaacagt ccagtgaaac tgtgcaggga 840

aatgaaggta gaattttggg aggtaataat gatgtgaaac ataaagattt aataattact 900

gtccaacaca gtggagcagc ttgtccacaa atatagtaat tactatttat tgctctaagg 960

aagattaaaa aaagataggg aaaaggggga aacttctttg aaaaatgaaa catctgttac 1020

attaatgtct aattataaaa ttttaatcct tactgcattt cttctgttcc tacaaatgta 1080

ttaaacattc agtttaaaaa aaaaaaaaaa aaa 1113

<210> 8
<211> 124
<212> PRT
<213> Homo sapiens

<400> 8

Leu Leu Arg Gly Pro Ala Arg Ala Arg Ser Gln Ser Pro Arg Arg Cys
1 5 10 15

Ala Ala Ser Cys Leu Leu Pro Pro Arg Arg Ser Arg Pro Ala Gly Leu
20 25 30

Arg Gly Ala Cys Leu Arg Ser Gln Val Glu Glu Leu Lys Leu Gly Lys
35 40 45

Met Val Cys Ile Pro Cys Ile Val Ile Pro Val Leu Leu Trp Ile Tyr
50 55 60

Lys Lys Phe Leu Glu Pro Tyr Ile Tyr Pro Leu Val Ser Pro Phe Val
65 70 75 80

Ser Arg Ile Trp Pro Lys Lys Ala Ile Gln Glu Ser Asn Asp Thr Asn
85 90 95

Lys Gly Lys Val Asn Phe Lys Gly Ala Asp Met Asn Gly Leu Pro Thr
100 105 110

Lys Gly Pro Thr Glu Ile Cys Asp Lys Lys Lys Asp
115 120

<210> 9
<211> 113
<212> PRT
<213> Rattus sp.

<400> 9

Ala Trp Ala Ser Trp Trp Trp Arg Val Thr Leu Ala Ala Val Val Ser
1 5 10 15

Gly Leu Thr Ser Pro Asp Ala Val Arg Cys Ala Gly Ile Pro Gln Glu
20 25 30

Leu Val Ser Glu Ile Gln Arg Gly Thr Met Val Cys Ile Pro Cys Ile
35 40 45

Val Ile Pro Val Leu Leu Trp Ile Phe Lys Lys Phe Leu Glu Pro Tyr
50 55 60

Ile Tyr Pro Val Val Ser Arg Ile Trp Pro Arg Lys Ala Val Gln Gln
65 70 75 80

Leu Asp Asn Arg Asn Thr Gly Lys Val Asp Cys Lys Gly Ala Asp Thr
85 90 95

Asn Gly Phe Ser Thr Lys Gly Pro Thr Glu Val Ser Asp Lys Lys Lys
100 105 110

Asp

<210> 10
<211> 80
<212 > PRT
<213> Oryctolagus sp.

<220>
<221> misc_feature
<222> (28).. (28)
<223> Xaa can be any naturally occurring amino acid

<220>
<221> misc_feature
<222> (38)..(38)
<223> Xaa can be any naturally occurring amino acid

<400> 10

```
Glu Leu Lys Ala Gly Thr Met Val Cys Ile Pro Cys Ile Val Ile Pro
1               5                   10                  15

Ile Leu Leu Trp Ile Tyr Lys Lys Phe Leu Glu Xaa Tyr Ile Tyr Pro
            20                  25                  30

Leu Ile Ser Pro Phe Xaa Ser Arg Ile Trp Pro Arg Lys Ala Val Gln
        35                  40                  45

Glu Ser Ser Asp Asn Gly Arg Val Asp Cys Lys Gly Thr Asp Thr Asn
    50                  55                  60

Gly Leu Pro Thr Lys Gly Pro Thr Glu Ile Pro Asp Lys Lys Lys Asp
65                  70                  75                  80
```

<210> 11
<211> 70
<212> PRT
<213> Osteichthyes sp.

<400> 11

Phe Gln Pro Thr Arg Pro Val Gly Pro Lys Lys Phe Trp Ile Val Cys
1               5                   10                  15

Ser Ile Pro Val Thr Thr Met Val Cys Ile Pro Cys Ile Val Ile Pro
            20                  25                  30

Phe Val Leu Trp Phe Tyr His Lys Tyr Leu Gln Pro Ile Leu Tyr Pro
        35                  40                  45

Ile Ile Ser Lys Phe Trp Thr Pro Lys Ala Val Glu Ala Asn Gly Thr
    50                  55                  60

Ser Lys Phe Phe Phe Phe
65                  70

<210> 12
<211> 99
<212> PRT
<213> Drosophila sp.

<400> 12

Met Val Cys Val Pro Cys Ile Ile Ile Pro Leu Leu Leu Tyr Ile Trp
1               5                   10                  15

His Lys Phe Val Gln Pro Ile Leu Leu Arg Tyr Trp Asn Pro Trp Glu
            20                  25                  30

Lys Lys Asp Asp Asp Gly Asn Val Ile Lys Lys Gly Pro Asp Phe Pro
        35                  40                  45

Phe Glu Cys Lys Gly Gly Val Cys Pro Phe Val Pro Gly Gly Lys Lys
    50                  55                  60

Thr Glu Asn Val Ser Asp Asp Asp Ala Glu Glu Ser Glu Asn Pro Pro
65                  70                  75                  80

Leu Asn Ala Thr Ala Met Ala Ala Glu Thr Glu Val Asp Glu Ser Lys
                85                  90                  95

Lys Glu Ile

<210> 13
<211> 87
<212> PRT
<213> Unknown

<220>
<223> AA966965

<400> 13

Ala Gly Ala Pro Gln Glu Leu Val Ser Glu Leu Gln Pro Gly Thr Met
1 5 10 15

Val Cys Ile Pro Cys Ile Val Ile Pro Val Leu Leu Trp Ile Phe Lys
20 25 30

Lys Phe Leu Glu Pro Tyr Ile Tyr Pro Val Val Ser Arg Ile Trp Pro
35 40 45

Lys Lys Ala Val Gln Gln Ser Gly Asp Lys Asn Met Ser Lys Val Asp
50 55 60

Cys Lys Gly Ala Gly Thr Asn Gly Leu Pro Thr Lys Gly Pro Thr Glu
65 70 75 80

Val Ser Asp Lys Lys Lys Asp
85

<210> 14
<211> 14
<212> PRT
<213> Artificial Sequence

<220>
<223> Illustrative peptide

<400> 14

Pro Tyr Ile Tyr Pro Leu Val Ser Pro Phe Val Ser Arg Ile
1 5 10

<210> 15
<211> 11
<212> > PRT
<213> Artificial Sequence

<220>
<223> Illustrative peptide

<400> 15

Pro Val Leu Leu Trp Ile Tyr Lys Lys Phe Leu
1 5 10

<210> 16
<211> 14
<212> PRT
<213> Artificial Sequence

<220>
<223> Illustrative peptide

<400> 16

Asn Phe Lys Gly Ala Asp Met Asn Gly Leu Pro Thr Lys Gly
1 5 10

<210> 17
<211> 37
<212> PRT
<213> Artificial Sequence

<220>
<223> Illustrative peptide

<400> 17

Tyr Pro Leu Val Ser Pro Phe Val Ser Arg Ile Trp Pro Lys Lys Ala
1 5 10 15

Ile Gln Glu Ser Asn Asp Thr Asn Lys Gly Lys Val Asn Phe Lys Gly
20 25 30

Ala Asp Met Asn Gly
35

<210> 18
<211> 29
<212> PRT
<213> Artificial Sequence

<220>
<223> Illustrative peptide

<400> 18

Asp Thr Asn Lys Gly Lys Val Asn Phe Lys Gly Ala Asp Met Asn Gly
1 5 10 15

Leu Pro Thr Lys Gly Pro Thr Glu Ile Cys Asp Lys Lys
20 25

<210> 19
<211> 20
<212> PRT
<213> Artificial Sequence

<220>
<223> Illustrative peptide

<400> 19

Pro Lys Lys Ala Ile Gly Glu Ser Asn Asp Thr Asn Lys Gly Lys Val
1 5 10 15

Asn Phe Lys Gly
20

<210> 20
<211> 14
<212> PRT
<213> Artificial Sequence

<220>
<223> Illustrative peptide

<400> 20

Asn Lys Gly Lys Val Asn Phe Lys Gly Ala Asp Met Asn Gly
1 5 10

<210> 21
<211> 28
<212> PRT
<213> Artificial Sequence

<220>
<223> Illustrative peptide

<400> 21

Ile Gly Glu Ser Asn Asp Thr Asn Lys Gly Lys Val Asn Phe Lys Gly
1 5 10 15

Ala Asp Met Asn Gly Leu Pro Thr Lys Gly Pro Thr
20 25

<210> 22
<211> 25
<212> > PRT
<213> Artificial Sequence

<220>
<223> Illustrative peptide

<400> 22

Met Val Cys Ile Pro Cys Ile Val Ile Pro Val Leu Leu Trp Ile Tyr
1 5 10 15

Lys Lys Phe Leu Glu Pro Tyr Ile Tyr
20 25

<210> 23
<211> 55
<212> PRT
<213> Artificial Sequence

<220>
<223> Illustrative peptide

<400> 23

Val Leu Leu Trp Ile Tyr Lys Lys Phe Leu Glu Pro Tyr Ile Tyr Pro
1 5 10 15

Leu Val Ser Pro Phe Val Ser Arg Ile Trp Pro Lys Lys Ala Ile Gln
20 25 30

Glu Ser Asn Asp Thr Asn Lys Gly Lys Val Asn Phe Lys Gly Ala Asp
35 40 45

Met Asn Gly Leu Pro Thr Lys
50 55

<210> 24
<211> 23
<212> PRT

<213> Artificial Sequence

<220>
<223> Illustrative peptide

<400> 24

```
Ser Pro Phe Val Ser Arg Ile Trp Pro Lys Lys Ala Ile Gln Glu Ser
1               5                   10                  15

Asn Asp Thr Asn Lys Gly Lys
            20
```

<210> 25
<211> 22
<212> PRT
<213> Artificial Sequence

<220>
<223> Illustrative peptide

<400> 25

```
Lys Lys Ala Ile Gly Glu Ser Asn Asp Thr Asn Lys Gly Lys Val Asn
1               5                   10                  15

Phe Lys Gly Ala Asp Met
            20
```

<210> 26
<211> 18
<212> PRT
<213> Artificial Sequence

<220>
<223> Illustrative peptide

<400> 26

```
Tyr Pro Leu Val Ser Pro Phe Val Ser Arg Ile Trp Pro Lys Lys Ala
1               5                   10                  15

Ile Gln
```

<210> 27
<211> 42
<212> PRT
<213> Artificial Sequence

<220>
<223> Illustrative peptide

<400> 27

Leu Val Ser Pro Phe Val Ser Arg Ile Trp Pro Lys Lys Ala Ile Gly
1 5 10 15

Glu Ser Asn Asp Thr Asn Lys Gly Lys Val Asn Phe Lys Gly Ala Asp
20 25 30

Met Asn Gly Leu Pro Thr Lys Gly Pro Thr
35 40

<210> 28
<211> 49
<212> PRT
<213> Artificial Sequence

<220>
<223> Illustrative peptide

<400> 28

Pro Cys Ile Val Ile Pro Val Leu Leu Trp Ile Tyr Lys Lys Phe Leu
1 5 10 15

Glu Pro Tyr Ile Tyr Pro Leu Val Ser Pro Phe Val Ser Arg Ile Trp
20 25 30

Pro Lys Lys Ala Ile Gly Glu Ser Asn Asp Thr Asn Lys Gly Lys Val
35 40 45

Asn

<210> 29
<211> 6
<212> PRT
<213> Artificial Sequence

<220>
<223> Illustrative peptide

<400> 29

Thr Glu Ile Cys Asp Lys
1 5

<210> 30

<211> 33
<212> PRT
<213> Artificial Sequence

<220>
<223> Illustrative peptide

<400> 30

```
Ile Trp Pro Lys Lys Ala Ile Gly Glu Ser Asn Asp Thr Asn Lys Gly
1               5                   10                  15

Lys Val Asn Phe Lys Gly Ala Asp Met Asn Gly Leu Pro Thr Lys Gly
            20                  25                  30

Pro
```

<210> 31
<211> 52
<212> PRT
<213> Artificial Sequence

<220>
<223> Illustrative peptide

<400> 31

```
Ile Tyr Pro Leu Val Ser Pro Phe Val Ser Arg Ile Trp Pro Lys Lys
1               5                   10                  15

Ala Ile Gly Glu Ser Asn Asp Thr Asn Lys Gly Lys Val Asn Phe Lys
            20                  25                  30

Gly Ala Asp Met Asn Gly Leu Pro Thr Lys Gly Pro Thr Glu Ile Cys
        35                  40                  45

Asp Lys Lys Lys
    50
```

<210> 32
<211> 46
<212> PRT
<213> Artificial Sequence

<220>
<223> Illustrative peptide

<400> 32

Lys Lys Phe Leu Glu Pro Tyr Ile Tyr Pro Leu Val Ser Pro Phe Val
1 5 10 15

Ser Arg Ile Trp Pro Lys Lys Ala Ile Gly Glu Ser Asn Asp Thr Asn
20 25 30

Lys Gly Lys Val Asn Phe Lys Gly Ala Asp Met Asn Gly Leu
35 40 45

<210> 33
<211> 31
<212> PRT
<213> Artificial Sequence

<220>
<223> Illustrative peptide

<400> 33

Trp Pro Lys Lys Ala Ile Gln Glu Ser Asn Asp Thr Asn Lys Gly Lys
1 5 10 15

Val Asn Phe Lys Gly Ala Asp Met Asn Gly Leu Pro Thr Lys Gly
20 25 30

<210> 34
<211> 32
<212> PRT
<213> Artificial Sequence

<220>
<223> Illustrative peptide

<400> 34

Leu Trp Ile Tyr Lys Lys Phe Leu Glu Pro Tyr Ile Tyr Pro Ile Val
1 5 10 15

Ser Pro Phe Val Ser Arg Ile Trp Pro Lys Lys Ala Ile Gly Glu Ser
20 25 30

<210> 35
<211> 41
<212> PRT
<213> Artificial Sequence

<220>

<223> Illustrative peptide

<400> 35

```
Val Leu Leu Trp Ile Tyr Lys Lys Phe Leu Glu Pro Tyr Ile Tyr Pro
1               5                   10                  15

Leu Val Ser Pro Phe Val Ser Arg Ile Trp Pro Lys Lys Ala Ile Gly
            20                  25                  30

Glu Ser Asn Asp Thr Asn Lys Gly Lys
        35                  40
```

<210> 36
<211> 25
<212> PRT
<213> Artificial Sequence

<220>
<223> Illustrative peptide

<400> 36

```
Met Val Cys Ile Pro Cys Ile Val Ile Pro Val Leu Leu Trp Ile Tyr
1               5                   10                  15

Lys Lys Phe Leu Glu Pro Tyr Ile Tyr
            20                  25
```

<210> 37
<211> 36
<212> PRT
<213> Artificial Sequence

<220>
<223> Illustrative peptide

<400> 37

```
Lys Ala Ile Gln Glu Ser Asn Asp Thr Asn Lys Gly Lys Val Asn Phe
1               5                   10                  15

Lys Gly Ala Asp Met Asn Gly Leu Pro Thr Lys Gly Pro Thr Glu Ile
            20                  25                  30

Cys Asp Lys Lys
        35
```

<210> 38
<211> 23
<212> PRT
<213> Artificial Sequence

<220>
<223> Illustrative peptide

<400> 38

```
Leu Val Ser Pro Phe Val Ser Arg Ile Trp Pro Lys Lys Ala Ile Gly
1               5                   10                  15

Glu Ser Asn Asp Thr Asn Lys
            20
```

<210> 39
<211> 76
<212> PRT
<213> Homo sapiens

<400> 39

```
Met Val Cys Ile Pro Cys Ile Val Ile Pro Val Leu Leu Trp Ile Tyr
1               5                   10                  15

Lys Lys Phe Leu Glu Pro Tyr Ile Tyr Pro Leu Val Ser Pro Phe Val
            20                  25                  30

Ser Arg Ile Trp Pro Lys Lys Ala Ile Gln Glu Ser Asn Asp Thr Asn
        35                  40                  45

Lys Gly Lys Val Asn Phe Lys Gly Ala Asp Met Asn Gly Leu Pro Thr
    50                  55                  60

Lys Gly Pro Thr Glu Ile Cys Asp Lys Lys Lys Asp
65                  70                  75
```